# EUROPÄISCHE PATENTSCHRIFT

(11) **EP 3 140 651 B1**
(45) Veröffentlichungstag und Bekanntmachung des Hinweises auf die Patenterteilung: **26.10.2022**
(21) Anmeldenummer: 15727889.6
(22) Anmeldetag: 11.05.2015
(51) Int. Cl.: G01N 33/50, G01N 33/564, G01N 33/68

(54) **MARKERSEQUENZEN ZUR DIAGNOSE UND STRATIFIZIERUNG VON SYSTEMISCHE SKLEROSE PATIENTEN**
MARKER SEQUENCES FOR DIAGNOSING AND STRATIFYING SYSTEMIC SCLEROSIS PATIENTS
SÉQUENCES DE MARQUEURS POUR LE DIAGNOSTIC ET LA STRATIFICATION DES PATIENTS ATTEINTS DE SCLÉROSE SYSTÉMIQUE

(30) Priorität: 09.05.2014 EP 14167807
(43) Veröffentlichungstag der Anmeldung: 15.03.2017
(73) Patentinhaber: Oncimmune Germany GmbH, 44227 Dortmund (DE)
(72) Erfinder: BUDDE, Petra, Dortmund (DE); ZUCHT, Dieter, Hannover (DE); LÜKING, Angelika, Berlin (DE); SCHNEIDER, Matthias, Düsseldorf (DE)
(74) Vertreter: Boult Wade Tennant LLP
(86) Internationale Anmeldenummer: PCT/EP2015/060396
(87) Internationale Veröffentlichungsnummer: WO 2015/169973

(56) Entgegenhaltungen:
- WO-A-2012/129423
- HUDSON MARIE ET AL: "Diagnostic criteria of systemic sclerosis", JOURNAL OF AUTOIMMUNITY, Bd. 48, 22. Januar 2014 (2014-01-22), Seiten 38-41, XP028636651, ISSN: 0896-8411, DOI: 10.1016/J.JAUT.2013.11.004
- MARLENE KRÄMER ET AL: "Inhibition of H3K27 histone trimethylation activates fibroblasts and induces fibrosis", ANNALS OF THE RHEUMATIC DISEASES, Bd. 72, Nr. 4, 25. April 2013 (2013-04-25) , Seiten 614-620, XP055461459, GB ISSN: 0003-4967, DOI: 10.1136/annrheumdis-2012-201615
- ALBON S ET AL: "Performance of a multiplex assay compared to enzyme and precipitation methods for anti-ENA testing in systemic lupus and systemic sclerosis", JOURNAL OF IMMUNOLOGICAL METHODS, ELSEVIER SCIENCE PUBLISHERS B.V.,AMSTERDAM, NL, Bd. 365, Nr. 1, 14. Dezember 2010 (2010-12-14), Seiten 126-131, XP028141194, ISSN: 0022-1759, DOI: 10.1016/J.JIM.2010.12.010 [gefunden am 2010-12-22]
- KATRIJN OP DE BEÉCK ET AL: "Antinuclear antibody detection by automated multiplex immunoassay in untreated patients at the time of diagnosis", AUTOIMMUNITY REVIEWS, Bd. 12, Nr. 2, 1. Dezember 2012 (2012-12-01), Seiten 137-143, XP055145218, ISSN: 1568-9972, DOI: 10.1016/j.autrev.2012.02.013
- RUDOLF MIERAU ET AL: "Frequency of disease-associated and other nuclear autoantibodies in patients of the German network for systemic scleroderma: correlation with characteristic clinical features", ARTHRITIS RESEARCH AND THERAPY, BIOMED CENTRAL, LONDON, GB, Bd. 13, Nr. 5, 21. Oktober 2011 (2011-10-21), Seite R172, XP021119708, ISSN: 1478-6354, DOI: 10.1186/AR3495 in der Anmeldung erwähnt
- WATTS ET AL: "Autoantibodies in the autoimmune rheumatic diseases", MEDICINE - U K EDITION, THE MEDICINE PUBLISHING COMPANY, GB, Bd. 34, Nr. 11, 1. November 2006 (2006-11-01), Seiten 441-444, XP028019676, ISSN: 1357-3039, DOI: 10.1053/J.MPMED.2006.08.002 [gefunden am 2006-11-01] in der Anmeldung erwähnt
- SONAL MEHRA ET AL: "Autoantibodies in systemic sclerosis", AUTOIMMUNITY REVIEWS, Bd. 12, Nr. 3, 1. Januar 2013 (2013-01-01) , Seiten 340-354, XP055145172, ISSN: 1568-9972, DOI: 10.1016/j.autrev.2012.05.011 in der Anmeldung erwähnt
- A Buliard ET AL: "Apport de la technologie Luminex pour la recherche simultanée de neuf autoanticorps associés aux connectivites. Résultats d'une étude multicentrique", Ann Biol Clin, 1. Januar 2005 (2005-01-01), Seiten 51-58, XP055145410, Gefunden im Internet: URL:http://www.jle.com/download/-abc-26444 3-apport_de_la_technologie_luminex_pour_la _recherche_simultanee_de_neuf_autoanticorp s_associes_aux_connectivites._resultats_du ne-VDZg3H8AAQEAAEN3BeEAAAAJ.pdf [gefunden am 2014-10-09]
- A. CARLSSON ET AL: "Serum Protein Profiling of Systemic Lupus Erythematosus and Systemic Sclerosis Using Recombinant Antibody Microarrays", MOLECULAR & CELLULAR PROTEOMICS, Bd. 10, Nr. 5, 1. Mai 2011 (2011-05-01), XP055015523, ISSN: 1535-9476, DOI: 10.1074/mcp.M110.005033
- HUDSON MARIE ET AL: "Diagnostic criteria of systemic sclerosis", JOURNAL OF AUTOIMMUNITY, vol. 48, 22 January 2014 (2014-01-22), pages 38-41, XP028636651, ISSN: 0896-8411, DOI: 10.1016/J.JAUT.2013.11.004
- MARLENE KRÄMER ET AL: "Inhibition of H3K27 histone trimethylation activates fibroblasts and induces fibrosis", ANNALS OF THE RHEUMATIC DISEASES, vol. 72, no. 4, 25 April 2013 (2013-04-25) , pages 614-620, XP055461459, GB ISSN: 0003-4967, DOI: 10.1136/annrheumdis-2012-201615

## Beschreibung

Offenbahrt sind Verfahren zur Identifizierung von Markern für Systemische Sklerose (SSc; auch Sklerodermie oder synonym Progressive Systemische Sklerose (PSS)) und die mit Hilfe dieses Verfahrens identifizierten Marker, die zwischen SSc und anderen Autoimmunerkrankungen einerseits und zwischen verschiedenen SSc-Subgruppen andererseits differenzieren können. Die vorliegende Erfindung betrifft die Verwendung von den Markern CENPB (SEQ ID No. 793 oder kodiert von SEQ ID NO: 155 oder 474), TOP1 (SEQ ID No. 952 oder kodiert von SEQ ID NO: 314 oder 633), KDM6B (SEQ ID No. 639 oder kodiert von SEQ ID NO: 1 oder 320,) und BICD2 (SEQ ID No. 640 oder kodiert von SEQ ID NO: 2 oder 321) bei der in vitro/ex vivo diagnose von SSc; sowie einen festen Träger, auf dem diese Marker fixiert sind.

Die SSc ist eine chronische, entzündliche, rheumatische Erkrankung, die zu den klassischen immunologischen Bindegewebserkrankungen (Kollagenosen) zählt.

Die SSc ist eine heterogene Erkrankung mit übermäßiger Fibrose der Haut. Es können auch weitere Organsysteme wie z.B. Lunge, Magen-Darm, Niere, Herz und Blutgefäße betroffen sein. Zusätzlich treten Gelenksymptome (Arthritis) auf.

SSc ist eine sehr seltene Erkrankung. Die Inzidenz beträgt ca. 0,5-1,5/100.000 Einwohner/Jahr. Sie tritt meist zwischen dem 30.-50. Lebensjahr auf. Frauen sind 10-15 mal häufiger betroffen als Männer (LeRoy et al. 1988).

Klinisch werden nach LeRoy et al. (1988) zwischen der limitierten und der diffusen SSc unterschieden. In frühen Phasen der Erkrankung ist es oftmals schwierig Patienten eindeutig zu klassifizieren, welches als undifferenzierte SSc bezeichnet wird. Treten zusätzlich zur Sklerodermie auch wesentliche Symptome anderer rheumatischer Krankheiten auf, spricht man von einem Sklerodermie-Überlappungssyndrom oder Overlap-Syndrome.

Die limitierte Form der SSc tritt mit einer Frequenz von bis zu 60% auf. Diese ist charakterisiert durch eine Fibrose der Hände und Füße, die sich bis unterhalb der Ellbogen und Kniegelenke ausbreitet. Vor Auftreten der Hautfibrose besteht häufig bereits über mehrere Jahre das Raynaud-Phänomen. Häufig treten auch gastrointestinale Veränderungen (Schluckbeschwerden) und pulmonale arterielle Hypertonie (PAH) auf. Zur limitierten Form gehört auch das CREST-Syndrom: Calcinosis cutis, Raynaud-Phänomen, Ösophagusmotilitätsstörung, Sklerodaktylie und Teleangiektasie.

Bei der diffusen Form handelt es sich um die schneller und schwerwiegender verlaufende Form der SSc. Die Fibrose breitet sich dabei über die Ellbogen über den Körper und Gesicht aus. Im Gegensatz zur limitierten Form treten bereits 1-2 Jahre nach dem Auftreten des Raynaud-Phänomens Hautfibrosen auf.

Beim Sklerodermie-Überlappungssyndrom treten neben den Hauptsymptomen der Sklerodermie auch Symptome weiterer nichtorganspezifischer Autoimmunerkrankungen, wie z.B. der Myositis, Lupus Erythematodes/SLE, Sjögren-Syndrom und rheumatoide Arthritis/RA auf.

Patienten mit undifferenzierter SSc weisen das Raynaud-Syndrom auf und haben für SSc typische geschwollene Finger und pulmonale arterielle Hypertonie. Nur ein Teil der Patienten entwickelt später tatsächlich eine diffuse oder limitierte SSc.

Die Diagnose der SSc kann aufgrund des klinischen Bildes mit den typischen Hautveränderungen gestellt werden. Dies kann jedoch in Frühstadien der Erkrankung zum Teil schwierig sein. Weiterhin wird der Nachweis von antinukleären Antikörpern (ANA) herangezogen. In etwa 90% der SSc Patienten sind ANA nachweisbar. Jedoch ist der ANA-Test nicht spezifisch für SSc, da auch andere Kollagenosen und bis zu 20% der Gesunden positiv getestet werden. Die drei wichtigsten Autoantikörper bei SSc sind anti-Topoisomerase I (Scl-70), anti-Centromere (CENP) und anti-RNA-Polymerase III (anti-RNAP III). Diese Autoantikörper weisen eine hohe Spezifität für SSc auf und sind häufig mit einer Subform der SSc assoziiert. Jedoch eignen sich diese 3 Autoantikörper nur eingeschränkt zur Subtypisierung der SSc, da diese nicht ausschließlich in einem Subtyp vorkommen und deren Frequenz offenbar in verschiedenen Ethnizitäten abweichen kann. Anti-Topoisomerase-Antikörper weisen eine hohe Spezifität für SSc auf und sind in etwa 30% der Patienten mit diffuser SSc nachweisbar. Anti-Centromere Antikörper sind dagegen in etwa 50-60% der Patienten mit limitierter SSc und in 10% der Patienten mit diffuser SSc nachweisbar. Beide Autoantikörper schließen sich aus und sind nur in sehr seltenen Fällen gemeinsam in Patienten nachweisbar. Anti-RNAP III Antikörper sind häufiger in der diffusen Form nachweisbar und stellen einen Risikofaktor für die eine renale Krise dar. Insgesamt können mit den Autoantikörpern gegen anti-Topoisomerase, anti-Centromere und anti-RNAP nur etwa 70% der SSc Patienten diagnostisch identifiziert werden (Mierau et al. 2011; Mehra et al. 2013).

Seltener treten auch Antikörper gegen U1-RNP und PM-Scl Antikörper auf. Diese weisen jedoch nur eine geringe Spezifität für SSc auf: Anti-PM-Scl Antikörper werden häufig in Patienten mit Polymyositis/SSc Overlap Syndrom nachgewiesen. Antikörper gegen U1-RNP sind sowohl bei SSc als auch Mixed Connective Tissue Diseases (MCTD) und SLE nachweisbar. Bei etwa einem Drittel der Patienten werden auch Antikörper gegen typische Kollagenosen-Antigene, wie Rho52/SS-A, Ro60/SS-B, sowie citrullinated Peptide (ACPA) und Rheumafaktoren nachgewiesen.

In der klinischen Praxis ist die Diagnosestellung einer frühen Form der SSc und deren Klassifizierung in die Subgruppen diffus, limitiert oder Overlap Syndrom oftmals schwierig, da noch nicht alle Symptome vorliegen bzw. etwa 10-30% der Patienten Symptome einer anderen Kollagenose (Bindegewebserkrankung, Connective Tissue Disease) tragen. Da die verschiedenen Unterformen eine sehr unterschiedliche Prognose haben, besteht ein substantieller Bedarf an Biomarkern für eine verbesserte Diagnosestellung der SSc und für eine Einteilung in SSc-Subgruppen. Weiterhin besteht ein hoher Bedarf an prognostischen und prädiktiven Biomarkern.

Ein weiteres Problem der derzeit angewendeten diagnostischen Verfahren ist, dass die Tauglichkeit der bisher untersuchten Autoantigene zur Diagnose von Organbeteiligungen und Komplikationen umstritten sind und zum Teil widersprüchliche Daten veröffentlicht wurden.

Hudson M. et al. "Diagnostic criteria of systemic sclerosis", Journal of Autoimmunity 48, 38-41 offenbahrt Bemühungen, Kriterien für die Diagnose von SSc aufzustellen.

Daher besteht weiterhin Bedarf, an neuen Markern für SSc sowie die Sensitivität und Spezifität der bisher am häufigsten diagnostisch eingesetzten Autoantigene durch den Einsatz neuer Autoantigene oder Marker zu verbessern.

Offenbahrt ist, dass ein differentielles, eine Vielzahl von Schritten umfassendes Verfahren entwickelt wurde, bei dem Serumproben einer Vielzahl von Gesunden und Patienten mit verschiedenen Autoimmunerkrankungen vergleichend im Hinblick auf ihre Reaktivität mit einer Vielzahl von potentiellen Antigenen untersucht und diese Ergebnisse statistisch ausgewertet wurden. Die Auswahl der Serumproben und die Abfolge der Schritte ermöglichte überraschend die Identifizierung hochspezifischer Marker für SSc, die auch geeignet sind, SSc-Subgruppen zu identifizieren und

Komplikationen zu diagnostizieren sowie eine Differentialdiagnose im Hinblick auf andere Autoimmunerkrankungen wie rheumatoide Arthritis (RA), insbesondere Frühstadien von RA ("frühe RA") und Spondylitis ankylosans bzw. Morbus Bechterew (SPA).

Offenbahrt ist ein mehrstufiges Verfahren zur Identifizierung von spezifischen Markern für SSc sowie die mit Hilfe des Verfahrens identifizierten Marker für SSc, die Verwendung und / oder spezifische therapeutische Anwendung dieser Marker zur Diagnose und / oder differentiellen Diagnose der Systemischen Sklerose und / oder zur Unterscheidung von klinischen Subgruppen von SSc.

Offenbahrt ist daherein Verfahren zur Identifizierung von Markern für Systemische Sklerose (SSc) umfassend die Schritte:
a) Serumproben von mindestens 50, vorzugsweise 100 SSc Patienten werden mit mehr als 5000 an Beads, beispielsweise Luminex-Beads gekoppelten Antigenen in Kontakt gebracht, die Bindung der einzelnen Antigene an Proteine in den Serumproben der SSc Patienten durch Immunfluoreszenzassay gemessen und die mittlere Fluoreszenzintensität (MFI = median fluorescence intensity) für jedes einzelne Antigen bestimmt;
b) Serumproben von mindestens 50, vorzugsweise 100 Patienten mit Lupus Erythematodes (SLE) werden mit denselben an Beads, beispielsweise Luminex-Beads gekoppelten Antigenen in Kontakt gebracht, die Bindung der einzelnen Antigene an Proteine in den Serumproben der SLE Patienten durch Immunfluoreszenzassay gemessen und daraus die mittlere Fluoreszenzintensität (MFI) für jedes einzelne Antigen bestimmt;
c) Serumproben von mindestens 50, vorzugsweise 537 Patienten mit früher rheumatoider Arthritis (RA) werden mit denselben an Beads, beispielsweise Luminex-Beads gekoppelten Antigenen in Kontakt gebracht, die Bindung der einzelnen Antigene an Proteine in den Serumproben der RA Patienten durch Immunfluoreszenzassay gemessen und daraus die mittlere Fluoreszenzintensität (MFI) für jedes einzelne Antigen bestimmt;
d) Serumproben von mindestens 50, vorzugsweise 82 Patienten mit ankylosierender Spondylitis (SPA) werden mit denselben an Beads, beispielsweise Luminex-Beads gekoppelten Antigenen in Kontakt gebracht, die Bindung der einzelnen Antigene an Proteine in den Serumproben der SPA Patienten durch Immunfluoreszenzassay gemessen und daraus die mittlere Fluoreszenzintensität (MFI) für jedes einzelne Antigen bestimmt;
e) Serumproben von mindestens 50, vorzugsweise 343 Gesunden werden mit denselben an Beads, beispielsweise Luminex-Beads gekoppelten Antigenen in Kontakt gebracht, die Bindung der einzelnen Antigene an Proteine in den Serumproben der Gesunden durch Immunfluoreszenzassay gemessen und daraus die mittlere Fluoreszenzintensität (MFI) für jedes einzelne Antigen bestimmt;
f) die MFI Daten jedes einzelnen Antigens aus a), b), c), d) und e) werden statistisch mittels univariater Analyse ausgewertet und dadurch Marker identifiziert, mit denen SSc Patienten von Patienten mit SLE, Patienten mit früher RA, Patienten mit SPA und von Gesunden differenziert werden können;
g) und wobei die Marker ausgewählt werden aus den Sequenzen SEQ ID No. 1 bis 955, Homologen der Sequenzen SEQ ID No. 1 bis 955 mit mindestens 95 % Homologie und Teilsequenzen von SEQ ID No. 1 bis 955 und Teilsequenzen der Homologen von SEQ ID No. 1 bis 955 mit mindestens 95 % Homologie und Sequenzen kodiert durch SEQ ID No. 1 bis 319.

Die Beads, die in dem offenbahrten Verfahren in den Schritten a) bis e) verwendet werden, sind vorzugsweise fluoreszenzmarkiert.

Die Begriffe Systemische Sklerose (SSc), RA bzw. frühe RA, SLE und SPA sind beispielsweise in Pschyrembel, Klinisches Wörterbuch, de Gruyter, 261. Auflage (2011) definiert.

Zum Beispiel werden die Marker nach univariater statistischer Auswertung dadurch ausgewählt, dass sie einen Schwellenwert von p kleiner 0,05 und eine 1,5-fach veränderte Reaktivität in der SSc-Gruppe gegenüber der Kontrollgruppe haben. Die Kontrollgruppe umfasst oder besteht aus Patienten mit SLE und / oder Patienten mit früher RA und / oder Patienten mit SPA und / oder Gesunden. Gesunde sind Personen, bei denen keine SSc, keine SSc-Subform, keine frühe RA und keine SPA nachgewiesen wurde oder nachweisbar ist.

Offenbahrt ist auch ein Marker für SSc oder eine oder mehrere SSc-Subformen erhältlich durch das erfindungsgemäße Verfahren. Die SSc-Subgruppen sind beispielsweise diffuse SSc (kurz: dSSc), limitierte SSc (kurz: lSSc) und / oder SSc Overlap-Syndrom (kurz: SSc-OS).

Aufgrund der hohen klinischen und serologischen Heterogenität der SSC Erkrankung ist es schwierig, mit nur einem Biomarker SSC eindeutig zu diagnostizieren. Daher ist es oft erforderlich, möglichst unkorrelierte Autoantigene zu sogenannten Paneln von Markern ("Biomarker-Panel für SSc") zu kombinieren. Beispielsweise im Rahmen der individualisierten Medizin können entsprechende Panel von Markern für SSc für einzelne Patienten oder Patientengruppen individuell für den betreffenden SSc-Subtyp (Subgruppe) zusammengestellt werden. Deshalb ist es auch notwendig, eine Vielzahl von potentiellen Markern für SSc zur Verfügung zu haben, um entsprechende Subgruppen bzw. Subtypen spezifische Marker für SSc für den jeweils individuellen Fall auszuwählen. Ein entsprechendes Panel kann beispielsweise in Form einer Anordnung, eines Arrays oder auch eines oder mehrerer Beads ausgestaltet sein.

Gegenstand der Erfindung ist somit ein fester Träger, auf dem die Marker CENPB (SEQ ID No. 793 oder kodiert von SEQ ID NO: 155 oder 474), TOP1 (SEQ ID No. 952 oder kodiert von SEQ ID NO: 314 oder 633), KDM6B (SEQ ID No. 639 oder kodiert von SEQ ID NO: 1 oder 320) und BICD2 (SEQ ID No. 640 oder kodiert von SEQ ID NO: 2 oder 321) fixiert sind.

Gegenstand der Erfindung ist auch die Verwendung von den Markern CENPB (SEQ ID No. 793 oder kodiert von SEQ ID NO: 155 oder 474), TOP1 (SEQ ID No. 952 oder kodiert von SEQ ID NO: 314 oder 633), KDM6B (SEQ ID No. 639 oder kodiert von SEQ ID NO: 1 oder 320,) und BICD2 (SEQ ID No. 640 oder kodiert von SEQ ID NO: 2 oder 321) bei der in vitro/ex vivo diagnose von Systemische Sklerose (SSc), wobei CENPB, TOP1, KDM6B und BICD2 verwendet werden um eine Wechselwirkung mit Antikörpern aus einer Körperflüssigkeit oder eines Gewebeauszug eines Patienten mit SSc aufzuweisen.

Vorzugsweise werden dabei zusätzlich NSUN5 (SEQ ID No. 641 oder kodiert von SEQ ID NO: 3 oder 322) und/oder PPP1R2 (SEQ ID No. 933 oder kodiert von SEQ ID NO: 295 oder 614) verwended um eine Wechselwirkung mit Antikörpern aus einer Körperflüssigkeit oder eines Gewebeauszug eines Patienten mit SSc aufzuweisen.

Vorzugsweise werden dabei zusätzlich mindestens ein Marker ausgewählt aus der Gruppe RTEL1 (SEQ ID No. 642 oder kodiert von SEQ ID NO: 4 oder 323), MYST2 (SEQ ID No. 643 oder kodiert von SEQ ID NO: 5 oder 324), PSAT1 (SEQ ID No. 644 oder kodiert von SEQ ID NO: 6 oder 325), TEX264 (SEQ ID No. 645 oder kodiert von SEQ ID NO: 7 oder 326), TRIM21 (SEQ ID No. 646 oder kodiert von SEQ ID NO: 8 oder 327), ABCB8 (SEQ ID No. 647 oder kodiert von SEQ ID NO: 9 oder 328), AVEN (SEQ ID No. 648 oder kodiert von SEQ ID NO: 10 oder 329), CENPA (SEQ ID No. 651 oder kodiert von SEQ ID NO: 13 oder 332), CENPC1 (SEQ ID No. 652 oder kodiert von SEQ ID NO: 14 oder 333), GYS1 (SEQ ID No. 656 oder kodiert von SEQ ID NO: 18 oder 337), MARVELD2 (SEQ ID No. 660 oder kodiert von SEQ ID NO: 22 oder 341), PMF1 (SEQ ID No. 665 oder kodiert von SEQ ID NO: 27 oder 346), TTLL3 (SEQ ID No. 668 oder kodiert von SEQ ID NO: 30 oder 349), ATP13A2 (SEQ ID No. 682 oder kodiert von SEQ ID NO: 44 oder 363), LTF (SEQ ID No. 713 oder kodiert von SEQ ID NO: 75 oder 394), STMN4 (SEQ ID No. 730 oder kodiert von SEQ ID NO: 92 oder 411), ACBD6 (SEQ ID No. 746 oder kodiert von SEQ ID NO: 108 oder 427), GTF2F1 (SEQ ID No. 772 oder kodiert von SEQ ID NO: 134 oder 453), KDR (SEQ ID No. 776 oder kodiert von SEQ ID NO: 138 oder 457), DIP2C (SEQ ID No. 851 oder kodiert von SEQ ID NO: 213 oder 532), FXC1 (SEQ ID No. 862 oder kodiert von SEQ ID NO: 224 oder 543), HNRNPA1 (SEQ ID No. 865 oder kodiert von SEQ ID NO: 227 oder 546), HSBP1L1 (SEQ ID No. 867 oder kodiert von SEQ ID NO: 229 oder 548), KARS (SEQ ID No. 870 oder kodiert von SEQ ID NO: 232 oder 551), PBXIP1 (SEQ ID No. 895 oder kodiert von SEQ ID NO: 257 oder 576), ZNF431 (SEQ ID No. 928 oder kodiert von SEQ ID NO: 290 oder 609), STMN1 (SEQ ID No. 931 oder kodiert von SEQ ID NO: 293 oder 612), SSRP1 (SEQ ID No. 935 oder kodiert von SEQ ID NO: 297 oder 616) verwended um eine Wechselwirkung mit Antikörpern aus einer Körperflüssigkeit oder eines Gewebeauszug eines Patienten mit SSc aufzuweisen.

Vorzugsweise werden dabei zusätzlich die Marker NSUN5 (SEQ ID No. 641 oder kodiert von SEQ ID NO: 3 oder 322) PPP1R2 (SEQ ID No. 933 oder kodiert von SEQ ID NO: 295 oder 614) RTEL1 (SEQ ID No. 642 oder kodiert von SEQ ID NO: 4 oder 323), TRIM21 (SEQ ID No. 646 oder kodiert von SEQ ID NO: 8 oder 327), ABCB8 (SEQ ID No. 647 oder kodiert von SEQ ID NO: 9 oder 328), AVEN (SEQ ID No. 648 oder kodiert von SEQ ID NO: 10 oder 329), CENPA (SEQ ID No. 651 oder kodiert von SEQ ID NO: 13 oder 332), CENPC1 (SEQ ID No. 652 oder kodiert von SEQ ID NO: 14 oder 333), GYS1 (SEQ ID No. 656 oder kodiert von SEQ ID NO: 18 oder 337), MARVELD2 (SEQ ID No. 660 oder kodiert von SEQ ID NO: 22 oder 341), PMF1 (SEQ ID No. 665 oder kodiert von SEQ ID NO: 27 oder 346), TTLL3 (SEQ ID No. 668 oder kodiert von SEQ ID NO: 30 oder 349), ATP13A2 (SEQ ID No. 682 oder kodiert von SEQ ID NO: 44 oder 363), LTF (SEQ ID No. 713 oder kodiert von SEQ ID NO: 75 oder 394), STMN4 (SEQ ID No. 730 oder kodiert von SEQ ID NO: 92 oder 411), ACBD6 (SEQ ID No. 746 oder kodiert von SEQ ID NO: 108 oder 427), GTF2F1 (SEQ ID No. 772 oder kodiert von SEQ ID NO: 134 oder 453), KDR (SEQ ID No. 776 oder kodiert von SEQ ID NO: 138 oder 457), DIP2C (SEQ ID No. 851 oder kodiert von SEQ ID NO: 213 oder 532), FXC1 (SEQ ID No. 862 oder kodiert von SEQ ID NO: 224 oder 543), HNRNPA1 (SEQ ID No. 865 oder kodiert von SEQ ID NO: 227 oder 546), HSBP1L1 (SEQ ID No. 867 oder kodiert von SEQ ID NO: 229 oder 548), KARS (SEQ ID No. 870 oder kodiert von SEQ ID NO: 232 oder 551), PBXIP1 (SEQ ID No. 895 oder kodiert von SEQ ID NO: 257 oder 576), ZNF431 (SEQ ID No. 928 oder kodiert von SEQ ID NO: 290 oder 609), STMN1 (SEQ ID No. 931 oder kodiert von SEQ ID NO: 293 oder 612), und SSRP1 (SEQ ID No. 935 oder kodiert von SEQ ID NO: 297 oder 616) verwended um eine Wechselwirkung mit Antikörpern aus einer Körperflüssigkeit oder eines Gewebeauszug eines Patienten mit SSc aufzuweisen.

Vorzugsweise werden dabei zusätzlich die Marker NSUN5 (SEQ ID No. 641 oder kodiert von SEQ ID NO: 3 oder 322), RTEL1 (SEQ ID No. 642 oder kodiert von SEQ ID NO: 4 oder 323), TRIM21 (SEQ ID No. 646 oder kodiert von SEQ ID NO: 8 oder 327), ABCB8 (SEQ ID No. 647 oder kodiert von SEQ ID NO: 9 oder 328), AVEN (SEQ ID No. 648 oder kodiert von SEQ ID NO: 10 oder 329), CENPA (SEQ ID No. 651 oder kodiert von SEQ ID NO: 13 oder 332), CENPC1 (SEQ ID No. 652 oder kodiert von SEQ ID NO: 14 oder 333), CENPT (SEQ ID No. 653 oder kodiert von SEQ ID NO: 15 oder 334), GYS1 (SEQ ID No. 656 oder kodiert von SEQ ID NO: 18 oder 337), C19or2 (SEQ ID No. 686 oder kodiert von SEQ ID NO: 48 oder 367), STMN4 (SEQ ID No. 730 oder kodiert von SEQ ID NO: 92 oder 411), ACBD6 (SEQ ID No. 746 oder kodiert von SEQ ID NO: 108 oder 427), CBX3 (SEQ ID No. 753 oder kodiert von SEQ ID NO: 115 oder 434), CENPH (SEQ ID No. 756 oder kodiert von SEQ ID NO: 118 oder 437), EIF4E (SEQ ID No. 761 oder kodiert von SEQ ID NO: 123 oder 442), GTF2F1 (SEQ ID No. 772 oder kodiert von SEQ ID NO: 134 oder 453), MAPT (SEQ ID No. 778 oder kodiert von SEQ ID NO: 140 oder 459), DIP2C (SEQ ID No. 851 oder kodiert von SEQ ID NO: 213 oder 532), PBXIP1 (SEQ ID No. 895 oder kodiert von SEQ ID NO: 257 oder 576), NEFL (SEQ ID No. 930 oder kodiert von SEQ ID NO: 292 oder 611), STMN1 (SEQ ID No. 931 oder kodiert von SEQ ID NO: 293 oder 612), und SSRP1 (SEQ ID No. 935 oder kodiert von SEQ ID NO: 297 oder 616) verwended um eine Wechselwirkung mit Antikörpern aus einer Körperflüssigkeit oder eines Gewebeauszug eines Patienten mit SSc aufzuweisen.

Die große klinische Heterogenität von SSc stellt derzeit sowohl für die Diagnostik als auch für die Wirkstoffentwicklung ein großes Problem dar. Die Identifizierung von spezifischen Antikörpersignaturen in SSC-Patienten-Subgruppen stellt daher einen wichtigen Schritt für die bessere Definition von Patientengruppen in klinischen Studien dar. So könnten beispielsweise spezifische Autoantikörper für dSSc, lSSc oder SSc-OS dazu verwendet werden, diese Subgruppe für Medikamentenstudien zu rekrutieren.

Die Erfindung umfasst einem festen Träger,auf dem die Marker CENPB (SEQ ID No. 793 oder kodiert von SEQ ID NO: 155 oder 474), TOP1 (SEQ ID No. 952 oder kodiert von SEQ ID NO: 314 oder 633), KDM6B (SEQ ID No. 639 oder kodiert von SEQ ID NO: 1 oder 320) und BICD2 (SEQ ID No. 640 oder kodiert von SEQ ID NO: 2 oder 321) fixiert sind.

Der feste Träger ist beispielsweise ein Filter, eine Membran, ein kleines Plättchen oder Kügelchen, beispielsweise ein magnetisches oder Fluorophor-markiertes Kügelchen, ein Silizium-Wafer, ein Bead, ein Chip, ein massenspektrometrometrischer Target oder eine Matrix oder ähnliches. Als Träger sind unterschiedliche Materialien geeignet, die dem Fachmann bekannt sind, beispielsweise Glas, Metall, Kunststoff, Filter, PVDF, Nitrocellulose oder Nylon (z.B. Immobilon P Millipore, Protran Whatman, Hybond N+ Amersham).

Der Träger kann beispielsweise einem Gitter entsprechen, dass die Größenordnung einer Mikrotiterplatte (8-12 Wells Streifen, 96 Wells, 384 Wells oder mehr), eines Silizium-Wafers, eines Chips, eines massenspektrometrischen Targets oder einer Matrix besitzt.

In einer Ausführungsform der Erfindung liegen die Marker für SSc als Klonsequenzen bzw. Klon(e) vor.

Die erfindungsgemäßen Marker CENPB, TOP1, KDM6B und BICD2 können mit bekannten Biomarkern für SSc oder Biomarkern für andere Erkrankungen kombiniert, ergänzt oder erweitert werden.

Die Verwendung der SSc Marker erfolgt außerhalb des menschlichen oder tierischen Körpers, also erfolgt die Diagnose ex vivo / in vitro.

"Diagnose" im Sinne dieser Erfindung bedeutet die positive Feststellung von SSc mit Hilfe der erfindungsgemäßen Marker und die Zuordnung der Patienten bzw. deren Symptomen zu der Erkrankung SSc dar. Der Begriff der Diagnose umfasst die medizinische Diagnostik und diesbezügliche Untersuchungen, insbesondere die in-vitro Diagnostik und Labordiagnostik,

ebenfalls Proteomics und Nukleinsäureblots. Weitere Untersuchungen können zur Absicherung und zum Ausschluss anderer Krankheiten vonnöten sein. Daher umfasst der Begriff Diagnose insbesondere die Differentialdiagnose von SSc mittels der erfindungsgemäßen Marker.

Im Rahmen dieser Erfindung wird unter "Patient" bzw. "Patientin" ein beliebiger Proband, ein beliebiges Individuum - Mensch oder Säugetier - verstanden, mit der Maßgabe, dass der Proband bzw. das Individuum auf SSc untersucht wird.

Der Begriff "Marker für SSc" im Sinne dieser Erfindung bedeutet, dass die kodierte Aminosäuresequenz oder das Polypeptid oder Protein signifikant (spezifisch) für SSc und / oder die mit SSc einhergehenden Autoantikörperprofile sind. "Erfindungsgemäße Marker" sind CENPB, TOP1, KDM6B und BICD2 in Kombination, möglicherweise zusätzlich mit den zusätzlichen Markern, die in Ansprüchen 2-6 erwähnt sind. Offenbahrt sind Nukleinsäuresequenzen und / oder Aminosäuresequenzen gemäß der Definition im anliegenden Sequenzprotokoll (SEQ ID No. 1 bis SEQ ID No. 955), deren Homologe und Teilsequenzen und wobei auch modifizierte Nukleinsäure- und Aminosäuresequenzen offenbahrt sind. Dabei bedeutet, Marker für SSc beispielsweise, dass das Polypeptid oder Protein eine Wechselwirkung mit Substanzen aus der Körperflüssigkeit oder Gewebeauszug eines Patienten mit SSc aufweisen (z.B. Antigen (Epitop) / Antikörper (Paratop) Wechselwirkung). In einer besonders bevorzugten Ausführungsform der Erfindung ist der Marker für SSc ein (Auto-)Antigen oder ein Teil eines Antigens.

Die Substanzen aus der Körperflüssigkeit oder Gewebeauszug treten entweder nur oder zumindest verstärkt bei SSc auf beziehungsweise werden exprimiert, wohingegen diese Substanzen bei Patienten ohne SSc oder Gesunden nicht oder zumindest in geringerem Maße (geringere Menge, geringerer Konzentration) vorhanden sind. Marker für SSc können sich andererseits auch dadurch auszeichnen, dass sie eine Wechselwirkung mit Substanzen aus der Körperflüssigkeit oder Gewebeauszug von Patienten mit SSc eingehen, weil diese Substanzen nicht mehr oder zumindest in deutlich geringerer Menge/Konzentration bei SSC auftreten beziehungsweise exprimiert werden, wohingegen diese Substanzen bei Patienten ohne SSC vorhanden oder zumindest in deutlich höherem Maße vorhanden sind. Marker für SSC können auch in gesunden Probanden vorhanden sein, jedoch verändert sich ihre Menge (Konzentration) beispielsweise bei der Entstehung, Etablierung und Therapie von SSC. Ein oder mehrere Marker können auf diese Weise ein Profil von Substanzen aus Körperflüssigkeit und Gewebeauszug abbilden, beispielsweise ein SSc assoziiertes Autoantikörperprofil des betreffenden Patienten. Erfindungsgemäße Marker sind Biomarker für SSc.

Autoantikörperprofile umfassen die Menge an einem oder mehreren Autoantikörpern deren Vorkommen/Expression mit der Entstehung und/oder Etablierung von SSc einhergehen. Autoantikörperprofile umfassen somit einerseits die Zusammensetzung, d.h. es werden beispielsweise ein oder mehrere Autoantikörper nur bei SSc exprimiert, und anderseits die Menge/Konzentration einzelner Autoantikörper, d.h. die Menge/Konzentration einzelner Autoantikörper verändert sich bei der Entstehung und Etablierung von SSc. Diese Veränderungen können mit Hilfe der erfindungsgemäßen Marker(sequenzen) nachgewiesen werden.

In einer besonders bevorzugten Ausführungsform erkennt/bindet der SSc Marker an Autoantikörper, die im Verlauf der Entstehung, Etablierung und Therapie von SSc (verstärkt) vorhanden sind oder in geringerem Maße (oder nicht mehr) vorhanden sind. Autoantikörper werden vom Körper gegen körpereigene Antigene, die beispielsweise bei SSc entstehen, gebildet. Autoantikörper werden von Körper gegen unterschiedliche Substanzen und Pathogene gebildet. Im Rahmen der vorliegenden Erfindung werden insbesondere die Autoantikörper detektiert, die beim Auftreten und im Laufe der Entstehung von SSc gebildet werden und/oder in ihrer Expression hoch- beziehungsweise herunterreguliert werden. Diese Autoantikörper können mit Hilfe der erfindungsgemäßen Verfahren und Marker nachgewiesen werden und ihr Nachweis und ihre Überwachung (z.B. der Menge) kann zur Früherkennung, Diagnose und/oder Therapieüberwachung/Therapiesteuerung sowie zur Prognose und Vorhersage des Risikos des Wiederauftretens von SSc im Rahmen der Nachsorge verwendet werden.

Die Autoantikörperprofile können bereits bei Verwendung der SSc Marker CENPB, TOP1, KDM6B und BICD2 ausreichend charakterisiert werden.

In einer Ausführungsform der Erfindung können Autoantikörper mit SSc Markern nachgewiesen werden, die sich von einem anderen Individuum ableiten und die beispielsweise aus einer kommerziellen cDNA-Bank stammen, wobei mindestens CENPB, TOP1, KDM6B und BICD2 verwendet werden.

In einer anderen Ausführungsform der Erfindung können diese Autoantikörper mit SSc Markern nachgewiesen werden, die sich von dem gleichen Individuum ableiten und die beispielsweise aus einer eigens für den Patienten oder eine Gruppe von Patienten beispielsweise im Rahmen der individualisierten Medizin hergestellten cDNA-Bank stammen. Mindestens werden CENPB, TOP1, KDM6B und BICD2 verwendet.

Autoantikörper können bereits viele Jahre vor Auftreten der ersten Krankheitssymptome vom Patienten gebildet werden. Damit sind eine Früherkennung, Diagnose und auch Prognose und vorbeugende Behandlung bzw. Umstellung der Lebensweise und andere Möglichkeiten der Prävention bereits Jahre vor dem sichtbaren Krankheitsausbruch möglich. Die erfindungsgemäßen Verwendungen oder feste Träger ermöglichen somit ein im Vergleich zu bekannten Verfahren sehr frühes Eingreifen, was die Prävention, Behandlungsmöglichkeiten und Folgen von SSc deutlich verbessert.

Da sich die SSc-assoziierten Autoantikörperprofile während der Etablierung und Behandlung/Therapie von SSc verändern, ermöglicht die Erfindung auch den Nachweis und die Überwachung von SSc in jedem Stadium der Entstehung und Behandlung sowie die Überwachung im Rahmen der SSC Nachsorge. Die erfindungsgemäßen Verwendungen oder feste Träger erlauben auch eine einfache Handhabung zu Hause durch den Patienten und die kostengünstige routinemäßige Vorsorge zur Früherkennung.

Verschiedene Patienten können unterschiedliche SSc assoziierte Autoantikörperprofile aufweisen, beispielsweise unterscheiden sich verschiedene Kohorten oder Bevölkerungsgruppen. Jeder Patient kann dabei ein oder mehrere verschiedene SSc assoziierte Autoantikörper im Laufe der Entstehung von SSc und des Fortschreitens der SSc Erkrankung, d.h. ebenfalls verschiedene Autoantikörperprofile, bilden. Außerdem kann sich die Zusammensetzung und/oder die Menge der gebildeten Autoantikörper im Laufe der SSc Entstehung und des Fortschreitens der Krankheit verändern, so dass eine quantitative Auswertung notwendig wird. Auch die Therapie/Behandlung von SSc führt zu Veränderungen in der Zusammensetzung und/oder der Menge an SSc assoziierten Autoantikörpern. Die große Auswahl an SSc Markern, die im Rahmen dieser Erfindung zusammen mit CENPB, TOP1, KDM6B und BICD2 verwendet werden können, ermöglicht die individuelle Zusammenstellung von SSc Markern in einer Anordnung, einem Panel für einzelne Patienten, Gruppen von Patienten, bestimmte Kohorten, Bevölkerungsgruppen und dergleichen.

Der Nachweis von SSc assoziierten Autoantikörpern beispielsweise im Serum oder Plasma von Patienten hat gegenüber anderen Biomarkern den Vorteil einer hohen Stabilität und Lagerfähigkeit und einer guten Nachweisbarkeit. Auch unterliegt die Anwesenheit von Autoantikörpern keinem circardianen Rhythmus, so dass die Probennahme unabhängig von Tageszeit, Nahrungsaufnahme und dergleichen ist.

Daneben können die SSc assoziierten Autoantikörper mit Hilfe der korrespondierenden Antigene/Autoantigene in bekannten Assays wie z.B. ELISA oder Western-Blot nachgewiesen werden und auf diese Weise die Ergebnisse überprüft werden.

Im Sinne der Erfindung wird eine Wechselwirkung zwischen dem SSc Marker und dem betreffenden Serum, beispielsweise einem Autoantikörper des Patienten, nachgewiesen. Eine solche Wechselwirkung ist z.B. eine Bindung, insbesondere eine bindende Substanz an mindestens einen Marker für SSc. Die Wechselwirkung zwischen der Körperflüssigkeit oder dem Gewebeauszuges eines Patienten und den Markern für SSc ist eine Protein-Protein Wechselwirkung.

Solche Substanzen, beispielsweise Antigene, Autoantigene, SSc assoziierte Autoantikörper, sind erfindungsgemäß Bestandteil einer Körperflüssigkeit, insbesondere Blut, Vollblut, Blutplasma, Blutserum, Patientenserum, Urin, Cerebrospinalflüssigkeit, Synovialflüssigkeit oder eines Gewebeauszuges des Patienten. Die Erfindung betrifft insbesondere die Verwendung dieser Körperflüssigkeiten und Gewebeauszüge für Früherkennung, Diagnose, Prognose, Therapiesteuerung und Nachsorge.

Die SSc spezifischen Marker verfügen in einer weiteren Ausführungsform der Erfindung über ein Erkennungssignal, welches an die zu bindende Substanz adressiert ist (z.B. Antikörper). Erfindungsgemäß bevorzugt ist für ein Protein das Erkennungssignal Epitop und / oder Paratop und / oder Hapten.

Die SSc spezifischen Marker können Modifikationen der Aminosäuresequenz aufweisen, beispielsweise Citrullinierung, Acetylierung, Phosphorylierung, Glykosilierung, Methylierung, polyA-Strang-Verlängerung und andere dem Fachmann einschlägig bekannte Modifikationen.

In einer weiteren Ausführungsform kann der jeweilige SSc Marker in unterschiedlichen Mengen in einem oder mehreren Bereichen auf dem Träger repräsentiert sein. Dies erlaubt eine Variation der Sensitivität. Die Bereiche können jeweils eine Gesamtheit von SSc Markern aufweisen, d.h. eine genügende Zahl an verschiedenen SSc Markern, wobei mindestens CENPB, TOP1, KDM6B und BICD2 auf dem Träger repräsentiert sind. Beispielsweise können 20 bis 50 (numerisch) oder mehr, bevorzugt mehr als 100, besonders bevorzugt 150 oder mehr, beispielsweise 25.000 oder 5.000 oder 10.000 verschiedene oder gleiche SSc Markersequenzen und gegebenenfalls weitere Nukleinsäuren und/oder Proteine, insbesondere andere Biomarker auf dem Träger oder in dem Panel repräsentiert sein.

Es bedeutet "Anordnung" synonym "Array" und synonym "Panel. Sofern dieser "Array" zur Identifizierung von Substanzen an SSc Markern verwendet wird, ist hierunter vorzugsweise ein "Assay" oder ein Bead oder eine diagnostische Vorrichtung oder ein Screening Assay zu verstehen. Im Rahmen dieser Erfindung ist die Anordnung derart gestaltet, dass die auf der Anordnung repräsentierten Marker, welche CENPB, TOP1, KDM6B und BICD2 umfassen, in Form eines Gitters auf einem Träger vorliegen. Ferner sind solche Anordnungen bevorzugt, die eine hochdichte (high-density) Anordnung von SSC Markern erlauben. Vorzugsweise werden die Marker gespottet. Solche hochdichte gespotteten Anordnungen sind beispielsweise in der WO 99/57311 und WO 99/57312 offenbart und können vorteilhaft in einem robotergestützten automatisierten High-Throughput Verfahren zur Anwendung kommen.

Im Rahmen dieser Erfindung kann die beanspruchte Verwendung Ausführungsformen wie ELISA, Bead-based Assay, Line Assay, Western Blot, immunchromatographische Verfahren (z.B. so genannte Lateral Flow Immunoassays) oder ähnliche immunologische Single- oder Multiplex-Nachweisverfahren beinhalten.

Ein "Proteinarray" im Sinne dieser Erfindung ist die systematische Anordnung von SSc Markern umfassend CENPB, TOP1, KDM6B und BICD2 auf einem festen Träger und wobei der Träger jede beliebige Form und/oder Größe haben kann und wobei der Träger ein fester Träger ist.

Die SSc Marker der Anordnung sind auf dem Träger fixiert, vorzugsweise gespottet oder immobilisiert, aufgedruckt oder ähnliches, insbesondere reproduzierbar aufgebracht. Ein oder mehrere SSc Marker können mehrfach in der Gesamtheit aller SSc Marker präsent sein und in unterschiedlichen Mengen bezogen auf einen Spot vorliegen. Ferner können die SSc Marker auf dem Träger standardisiert sein (z.B. mittels serieller Verdünnungsreihen von z.B. Humanglobulinen als interne Kalibratoren zur Datennormalisierung und quantitativen Auswertung). Gegebenenfalls kann auch ein Standard (z.B. ein Gold Standard) auf dem Träger aufgebracht sein.

Expressionsbibliotheken sind dem Fachmann bekannt, diese können nach Standardwerken, wie Sambrook et al, "Molecular Cloning, A laboratory handbook, 2nd edition (1989), CSH press, Cold Spring Harbor, New York hergestellt werden. Weiterhin bevorzugt sind solche Expressionsbibliotheken, die gewebespezifisch sind (z.B. humanes Gewebe, insbesondere humane Organe.

Weiterhin bevorzugt sind Proteinarrays oder entsprechende Expressionsbibliotheken, die keine Redundanz aufweisen (so genannte: Uniclone^{®}-Bibliothek) und nach den Lehren der WO 99/57311 und WO 99/57312 beispielsweise hergestellt werden können. Diese bevorzugten Uniclone- Bibliotheken weisen einen hohen Anteil an nicht-fehlerhaften vollständig exprimierten Proteinen einer cDNA-Expressionsbibliothek auf.

Zusätzlich können die SSc Marker in der jeweiligen Form als Fusionsprotein vorliegen, welches beispielsweise mindestens ein Affinitätsepitop oder "Tag" enthält, wobei das Tag beispielsweise ausgewählt wird aus c-myc, His-Tag, Arg-tag, FLAG, alkalische Phosphatase, V5-Tag, T7-Tag oder Strep-Tag, HAT-tag, NusA, S-tag, SBP-tag, Thioredoxin, DsbA oder das Fusionsprotein beispielsweise ein oder mehrere zusätzliche Domänen aufweist, beispielsweise eine Cellulose-bindende Domäne, grünfluoreszierendes Protein, Maltose bindendes Protein, Calmodulin-bindendes Protein, Glutathione S-transferase oder lacZ.

Weiterhin offenbahrt ist einen Assay, beispielsweise einen multiplex Assay, einen Bead basierten Assay oder Proteinarray zum Identifizieren und Charakterisieren einer Substanz, beispielsweise eines Hits, einer Leitsubstanz, eines Wirkstoffs für SSc. Dabei wird eine zu untersuchende Substanz eingesetzt. Diese kann ein beliebiges natives oder nichtnatives Biomolekül, ein (synthetisches) chemisches Molekül, ein Naturstoff, eine Mischung oder eine Substanzbibliothek sein. Nachdem die zu untersuchende Substanz einen SSC Marker kontaktiert hat, erfolgt die Auswertung des Bindungserfolges, beispielsweise unter Verwendung mit handelsüblicher Image-Analyse Software (GenePix Pro (Axon Laboratories), Aida (Raytest), ScanArray (Packard Bioscience).

Die Visualisierung erfindungsgemäßer Bindungen, Bindungserfolge, Wechselwirkungen wie Protein-Protein-Wechselwirkungen (z.B. Protein an SSc Marker wie Antigen/Antikörper) oder entsprechende "Mittel zum Nachweis des Bindungserfolges" kann beispielsweise mittels Fluoresenzmarkierung, Biotiniylierung, Radio-Isotopen-Markierung oder kolloidale Gold- oder Latex-Partikel-Markierung in üblicher Weise erfolgen. Ein Nachweis von gebundenen Antikörpern erfolgt vorzugsweise mit Hilfe von sekundären Antikörpern, die mit handelsüblichen Reportermolekülen markiert sind (z.B. Cy-, Alexa-, Dyomics, FITC- oder ähnliche Fluoreszenzfarbstoffe, kolloidale Gold- oder Latex-Partikel), oder mit Reporter-Enzymen wie alkalischer Phosphatase, Meerrettichperoxidase, usw. und den entsprechenden colorimetrischen, fluoreszenten oder chemolumineszenten Substraten. Eine Auslesung erfolgt z.B. mittels eines Microarray-Laserscanners, einer CCD-Kamera oder visuell.

In einer weiteren Ausführungsform betrifft die Erfindung ein Arzneimittel oder einen Wirkstoff oder eine Prodrug für SSc entwickelt und erhältlich durch den Einsatz eines erfindungsgemäßen SSc Markers.

Offenbahrt ist auch die Verwendung eines SSc Markers ausgewählt aus Sequenzen SEQ ID No. 1 bis 955 und Teilsequenzen von SEQ ID No. 1 bis 955 mit mindestens 90 %, vorzugsweise mindestens 95 % der Länge von SEQ ID No. 1 bis 955 und Homologen von SEQ ID No. 1 bis 955 und deren Teilsequenzen mit einer Identität von mindestens 95 %, vorzugsweise mindestens 98 % oder mehr zu den entsprechenden Sequenzen und Proteinen/Peptiden kodiert durch die Sequenzen SEQ ID No. 1 bis 638, kodiert durch deren Teilsequenzen und Homologen als Affinitätsmaterial zur Durchführung einer Apherese oder Blutwäsche für Patienten mit SSc. Offenbahrt ist somit die Verwendung der offenbahrten Marker, vorzugsweise in Form einer Anordnung, als Affinitätsmaterial zur Durchführung einer Blutwäsche im weiteren Sinne, wobei Substanzen aus Körperflüssigkeiten eines Patienten mit SSc, wie Blut oder Plasma, an die Marker binden und folglich der Körperflüssigkeit selektiv entzogen werden können.

Die nachfolgenden Beispiele dienen der Erläuterung der Erfindung ohne die Erfindung jedoch auf die Beispiele einzuschränken. In den nachfolgen Figuren wird die Systemische Sklerose mit PPS (Progressive Systemische Sklerose) bezeichnet.
Figur 1: Vulcano Plot der relativen Antigenreaktivitäten der SSc Patienten im Vergleich zu Gesundkontrollen
Figur 2: Vulcano Plot der Antigenreaktivitäten von SSc Patienten gegen eine vereinigte Gruppe von Patienten mit verschiedenen Autoimmunerkrankungen wie SSC SPA, frühere Rheumatoide Arthritis und SPA.
Figur 3: Autoantikörperreaktivität in SSc Patientenseren im Vergleich zu Gesundkontrollen und SSC Patienten.
Figur 4: Frequenz der Autoantikörperreaktivitäten ausgewählter Antigen in SSc Patienten und Gesundproben. Es wurde ein Schwellenwert von 3 SD Abweichungen über dem Mittelwert der Gesundprobe angesetzt.
Figur 5: Vulcano Plot der Autoantikörperreaktivitäten von SSc Patienten mit diffuser Subform gegen Gesundkontrollen
Figur 6: Frequenz der Autoantikörperreaktivitäten ausgewählter Antigen in der limitierten und diffusen SSc-Subform. Es wurde ein Schwellenwert von 3 Standard Abweichungen über dem Mittelwert der Gesundprobe angesetzt.
Figur 7: Vulcano Plot der Autoantikörperreaktivitäten von SSc Patienten mit limitierter Subform gegen Gesundkontrollen.
Figur 8: Vulcano Plot der Autoantikörperreaktivitäten von Patienten mit Overlap Syndrome gegen Gesundkontrollen
Figur 9: Frequenz der Autoantikörper in anti-CENP und anti-Sc170 negativen Patienten.
Figur 10: Receiver Operating Characteristic-Kurven(ROC)für die Diagnose von SSc im Vergleich zu Gesundproben; A) ROC Kurve Panel I, B) ROC Kurve Panel II.
Figur 11: Boxplot basierend auf den anti-KDM6B und anti-BICD2 ELISA Messungen für die Diagnose von SSc im Vergleich zu Gesundkontrollen.
Figur 12: Receiver Operating Characteristic-Kurven(ROC)für die ELISA Bestimmung anti-KDM6B und anti-BICD2 Antikörpern:
   a) Anti-KDM6B ELISA: SSc im Vergleich zu Gesundkontrollen
   b) Anti-BICD2 ELISA: SSc im Vergleich zu Gesundkontrollen Beispiele:
      Beispiel 1: Auswahl der SSc Patienten- und Kontrollproben Patienten und Probanden

Auswahl der zu testenden Patientengruppen: Es wurden Blutproben von 100 SSc Patienten, 100 Patienten mit SLS, 537 Patienten mit früher Rheumatoide Arthritis ("RA"; Erkrankungsdauer unter 6 Monaten) und 82 Patienten mit ankylosierende Spondylitis (SPA) bzw. Morbus Bechterew analysiert. Als Kontrollgruppe wurden 343 Blutproben vom Bayrischen Roten Kreuz (BRK) bezogen. Von allen Probanden wurde eine Einverständniserklärung (Informed Consent) der Ethikkommission der klinischen Partner und der Biobank des BRK eingeholt.

**Tabelle 1: Patientenproben und klinische Daten**

| | SLE | SSc | SSc | SSc | SSc | frühe RA (<6 Monate) | SPA | Gesund |
|---|---|---|---|---|---|---|---|---|
| | | Subgruppe lSSc | Subgruppe dSSc | Subgruppe SSc-OV | ohne Angabe der Subgruppe | | | |
| Anzahl der Patienten bzw. Proben | 100 | 50 | 32 | 9 | 9 | 537 | 82 | 343 |
| Durchschnittliches Alter (Jahre) | 39.8 +/-11.9 | 61.53 +/-16.97 | 53.88 +/-14.75 | 51.78 +/-9.18 | 47.56 +/-16.97 | 56.8 +/-14.3 | 43.7 +/-10.1 | 47.7 +/-11.7 |
| Anzahl der weibl. Patien-ten bzw. Proben | 83 | 50 | 32 | 8 | 9 | 62.2 | 15.9 | 58.3 |
| ANA positive | 100 | 47 | 32 | 9 | 7 | N.D. | N.D. | N.D. |
| Anti-CENP | N.D. | 30 | 4 | 2 | 2 | N.D. | N.D. | N.D. |
| Anti-Sc170 | N.D. | 9 | 20 | 1 | 2 | N.D. | N.D. | N.D. |

### Beispiel 2: Antigen Produktion

Fünf cDNA Bibliotheken, die aus verschiedenen humanen Geweben (fötales Gehirn, Darm, Lunge, Leber und T-Zellen) erzeugt worden waren, wurden für die Produktion der rekombinanten Antigene verwendet. Alle cDNAs wurden in E.coli unter der transkriptionellen Kontrolle des Lactose-induzierbaren Promotors exprimiert. Die resultierenden Proteine tragen an ihrem Aminoterminus eine zusätzliche Sequenz für einen Hexahistidin-Aufreinigungstag (His6-Tag). Targetantigene, die nicht in der cDNA Biobliothek vorhanden waren, wurden durch chemische Synthese (Life Technologies) erzeugt und in den Expressionsvektor pQE30-NST, der bereits einen Aminoterminalen His6-Tag kodiert, kloniert.

Nach rekombinanter Expression der Proteine wurden diese unter denaturierenden Bedingungen isoliert und mittels Metallaffinitätschromatographie (IMAC) aufgereinigt. Bis zur weiteren Verwendung wurden die Proteine lyophilisiert und bei -20°C gelagert.

### Beispiel 3: Herstellung von Bead Based Arrays (BBAs)

Die Herstellung von BBAs wurde auf ein Mikrotiterplatten-Format adaptiert, so dass 384 Kopplungsreaktionen parallel mit Pipettierautomaten (Starlet, Hamilton Robotics, Evo Freedom 150, Tecan) angesetzt werden konnten. Für die Verwendung von Pipettierautomaten wurden die einzelnen Beadregionen in Kopplungsplatten (96 Well Greiner) und die Antigene in 2D Barcode Gefäße (Thermo Scientific) überführt. Für jede Kopplungsreaktion wurden 0.6 bis 2.5 Millionen Beads und abhängig von Antigen 1 bis 100 µg Protein eingesetzt.

Alle Wasch- und Pipettierschritte der Kopplungsreaktion wurden in Kopplungsplatten, die auf Magneten fixiert wurden, durchgeführt. Die Beads wurden zweimal mit 100 µl LxAP-Puffer (100 mM NaH₂PO₄, pH 6.2) gewaschen und anschließend in 120 µl LxAP-Puffer aufgenommen. Für die Aktivierung wurden 15 µl 1-Ethyl-3-(3-dimethylaminopropyl)carbodiimid (EDC; 50mg/ml) und 15 µl N-Hydroxysulfosuccinimide (Sulfo-NHS; 50mg/ml) zur Bead-Suspension pipettiert und diese wurde anschließend 20 Minuten auf dem Schüttler (RT, 900 rpm, lichtgeschützt) inkubiert. Die Beads wurden 3x mit 150 µl LxKPT-Puffer gewaschen und anschließend die Proteinlösung zugegeben. Nach zweistündiger Inkubation auf dem Schüttler (RT, 900 rpm, lichtgeschützt) wurden die Beads dreimal mit 150 µl LxWPT-Puffer gewaschen. Zur Blockierung von freien Bindungsstellen wurden 100 µl LxCBSP-Puffer (PBS, 1% BSA, 0.05% ProClin300) zugegeben und 20 min auf dem Schüttler inkubiert (RT, 900 rpm, lichtgeschützt). Anschließend folgte eine Inkubation bei 4-8°C über Nacht. Das BBA wurde durch die Vereinigung von mit Antigen gekoppelten Beads hergestellt und bis zur Anwendung bei 4-8°C lichtgeschützt gelagert.

### Beispiel 4: Anwendung von BBAs

Für die Anwendung wurden BBAs mit Seren inkubiert und alle an Antigene gebunden IgG-basierte Autoantikörper wurden mit Hilfe eines sekundären Antikörper detektiert. Um einen hohen Durchsatz an Messungen zu ermöglichen, wurde die Anwendung von BBAs auf ein Mikrotiterplatten-Format adaptiert, so dass entweder ein 8-Kanal (Starlet, Hamilton Robotics) oder ein 96-Kanal (Evo Freedom 150, Tecan) Pipettierautomat verwendet werden konnte. Die zu untersuchenden Seren wurden in 2D Barcode Gefäße überführt und anschließend 1:100 mit Assaypuffer (PBS, 0.5% BSA, 10% E.coli Lysate, 50% Low-Cross Buffer (Candor Technologies)) verdünnt. Um humane Antikörper, die gegen E.coli Proteine gerichtet sind, zu neutralisieren, erfolgte eine Präinkubation der Serenverdünnungen für 20 min. In dieser Zeit wurden 500 Beads pro Beadregion in die Assayplatte verteilt. In die Kopplungsplatte wurden 50 µl verdünntes Serum zu den Beads gegeben und die Reaktionsansätze 18-22 h auf dem Schüttler inkubiert (4-8 °C, 900 rpm, lichtgeschützt). Nach drei Waschschritte mit jeweils 100 µl LxWPT-Puffer wurden 5 pg/ml des Detektionsantikörpers Ziegeanti-human IgG-PE (Dianova) zu den Reaktionsansätzen gegeben und für 1 h auf dem Schüttler inkubiert (RT, 900 rpm). Anschließenden wurden die Beads dreimal mit 100 µl L×WPT gewaschen und in 100 µl Trägerflüssigkeit (Luminex) aufgenommen. Das Fluoreszenzsignal der Beads wurde mit Hilfe des FlexMAP3D Instruments detektiert. Dabei wurden zum einen der Beadcount und zum anderen der MFI-Wert (Median der Fluoreszenzintensitität)

### Beispiel 5: Biostatistische Analyse

Die biostatistische Analyse umfasste univariate und multivariate Methoden um die statistischen Eigenschaften einzelner Antigene sowie von Gruppen von Antigenen zu beschreiben. Für das Auffinden von interessanten Kandidaten für Panels war die entscheidende Eigenschaft eine gute Trennung zwischen den Gruppen von Proben auf Basis der MFI-Werte. Um Antigen-Kandidaten für eine Panel-Generierung zu finden, wurden als Methoden univariates Testen, Receiver Operating Characteristic (ROC) Analysen, Korrelationsprofile, Powered Partial Least Squares Diskriminanzanalyse (PPLS-DA) und Zufallswälder angewendet. Biostatistische Analysen wurden einer Expertenbewertung unterzogen um finale Antigen-Panels zu definieren.

Vor der statistischen Analyse wurden die MFI-Werte log2-transformiert Antigene, bei denen mehr als 20% der Werte fehlten, wurden von der Analyse ausgeschlossen und fehlende Werte durch Median-Imputation ersetzt. Eine Quantil-Normalisierung unter Berücksichtigung der Referenzseren wurde durchgeführt, um pro BBA-Set alle gemessenen Proben auf individuellen Platten zu normalisieren.

Neben deskriptiven Standardstatistiken für MFI-Werte wurden mit Hilfe des zweiseitigen Mann-Whitney-U-Tests nichtparametrische Tests durchgeführt um Unterschiede in den Medianwerten der Gruppen zu entdecken. Korrektur des Testniveaus für multiples Testen erfolgte nach der Bonferroni-Holm Prozedur. Außerdem wurde die Benjamini-Hochberg Prozedur inklusive der Bestimmung der False Discovery Rate (FDR, q-Wert) angewendet. Zusätzlich wurden Fold-Change und Effektgröße bestimmt. Um die Klassifikationsgüte zu bewerten wurde eine ROC-Analyse durchgeführt, in deren Rahmen Sensitivität, Spezifität und die Fläche unter der ROC-Kurve (AUC) berechnet wurden, jeweils inklusive der 95% Konfidenzintervalle auf Basis des Bootstrap-Verfahrens. Zur grafischen Darstellung wurden Boxplots und Volcanoplots genutzt. Auf Basis der univariaten Ergebnisse wurde ein Scoring-System implementiert.

Durch die Anwendung einer PPLS-DA wurde die Korrelation zwischen den Komponenten und der Response-Matrix maximiert. Für die finale Klassifizierung wurde eine lineare Diskriminanzanalyse mit den latenten Komponenten als Prädiktoren genutzt. Ein Zufallswald wurde angewendet, in dem binäre Entscheidungsbäume kombiniert werden. Die Entscheidungsbäume wurden gebildet auf Basis mehrerer Bootstrap-Stichproben einer Lernstichprobe und durch zufälliges Auswählen einer Subgruppe von erklärenden Variablen an jedem Knoten. Die Anzahl der Eingangsvariablen, die an jedem Teilungsschritt zufällig gewählt wurde, wurde als die Quadratwurzel der Gesamtzahl der Variablen festgelegt, und die Anzahl der Bäume im Zufallswald wurde auf 1000 gesetzt. Für beide multivariaten Ansätze wurde eine Kreuzvalidierung mit 500-fachem Durchlauf implementiert.

Bespiel 6: Autoantikörper/ Antigenreaktivitäten differenzieren SSc von Gesundkontrollen, SLE, Rheumatoider Arthritis und anderen Autoimmunerkrankungen (AID)

In einem Screening wurden die Antigenreaktivitäten von 100 SSc Patienten, 537 Patienten mit Early RA, 82 Patienten mit SPA und 343 nach Alter und Geschlecht zugeordnete Gesundkontrollen wurden differentiell getestet. Dazu wurden die Autoantikörperreaktivitäten dieser Blutproben an 5857 an Luminex-Beads gekoppelten Antigenen getestet.

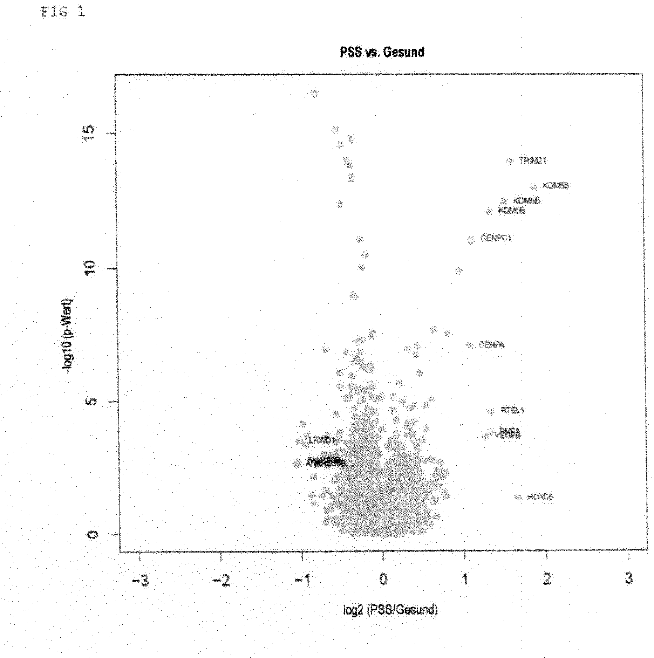

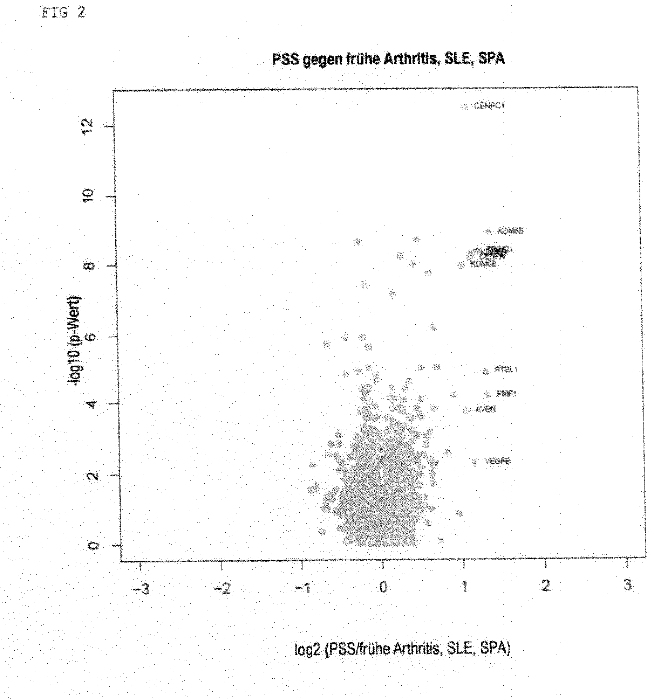

Um Antigene zu identifizieren, mit denen die Gruppe aller SSc Patienten von verschiedenen Kontrollgruppen bestehend aus Gesundproben und Patienten mit verschiedenen rheumatischen Erkrankungen unterschieden werden kann, wurden univariate statistische Tests durchgeführt. Das Ergebnis der statistischen Tests ist als Vulcano Plot für alle 5857 Antigene dargestellt. Im Vulcano Plot wird auf der x-Achse die relative Änderung der Antigenreaktivtät in SSc Patienten im Vergleich zu Gesundkontrollen (Figur 1) und AID Patienten (Figur 2) dargestellt. Die Y-Achse gibt den p-Wert des statistischen Tests wieder. Figuren 1 und 2 zeigen, dass spezifische Autoantikörperreaktivitäten gefunden wurden, die in der Gruppe aller SSc erhöht sind und diese sowohl von gesunden Spendern als auch von Patienten mit rheumatischen Erkrankungen unterschieden unterscheiden können.

Tabelle 2 umfasst alle in SSc Patienten identifizierte Autoantigene (Tabelle 2).

### Tabelle 2: Zusammenfassung der in SSc identifizierten (Auto-)Antigene (auch "Marker" oder "Biomarker" genannt)

Angegeben ist die fortlaufende Sequenz ID, die Gene ID, Gene Symbol und der Gene Name. Die Gruppe bezeichnet die Verwendung des Biomarkers zur Identifizierung aller SSc Patienten oder bestimmter SSc-Subgruppen basierend auf einem statistischen Schwellenwert von p<0.05 und relativ höheren Reaktivität (Fold-Change) gegenüber der Kontrollgruppe von größer 1.5.
Gruppe 1: Marker zur Identifizierung aller SSc Patienten unabhängig von der klinischen Subform;
Gruppe 2: Marker zur Identifizierung von diffuser SSc (dSSc);
Gruppe 3: Marker zur Identifizierung der limitierten SSc (lSSc);
Gruppe 4: Marker zur Identifizierung von SSc Overlap-Syndrom (Überlappungssydromen; SSc-OS) und
Gruppe 5: zusätzliche Marker, die keiner bestimmten Gruppe zugeordnet werden.

| **SEQ ID No.** | **Gene ID** | **Gene Symbol** | **Gene Name** | **Gruppe** | **p<0.05 und fold-change >1.5 in Gruppe** |
|---|---|---|---|---|---|
| **Hauptantigene** / **Hauptmarker** | | | | | |
| **1** | 23135 | KDM6B | lysine (K)-specific demethylase 6B | 1 | SSc; dSSc; lSSc; SSc-OS |
| **2** | 23299 | BICD2 | bicaudal D homolog 2 (Drosophila) | 3 | lSSc |
| **3** | 55695 | NSUN5 | NOL1/NOP2/Sun domain family, member 5 | 1 | SSc; lSSc |
| **4** | 51750 | RTEL1 | regulator of telomere elongation helicase 1 | 1 | SSc; lSSc |
| **5** | 11143 | MYST2 | MYST histone acetyltransferase 2 | 1 | SSc; dSSc |
| **6** | 29968 | PSAT1 | phosphoserine aminotransferase 1 | 2 | dSSc |
| **7** | 51368 | TEX264 | testis expressed 264 | 2 | dSSc |

| **Bevorzugte Antigene / bevorzugte Marker** | | | | | |
|---|---|---|---|---|---|
| **8** | 6737 | TRIM21 | tripartite motif containing 21 | 1 | SSc; dSSc; SSc-OS; lSSc |
| **9** | 11194 | ABCB8 | ATP-binding cassette, sub-family B (MDR/TAP), member 8 | 1 | SSc; lSSc |
| **10** | 57099 | AVEN | apoptosis, caspase activation inhibitor | 1 | SSc; lSSc |
| **11** | 7423 | VEGFB | vascular endothelial growth factor B | 1 | SSc; dSSc; lSSc; SSc-OS |
| **12** | 55049 | C19orf6 0 | chromosome 19 open reading frame 60 | 1 | SSc |
| **13** | 1058 | CENPA | centromere protein A | 1 | SSc; lSSc; |
| **14** | 1060 | CENPC1 | centromere protein C 1 | 1 | SSc; lSSc; dSSc |
| **15** | 80152 | CENPT | centromere protein T | 1 | SSc; lSSc |
| **16** | 1131 | CHRM3 | cholinergic receptor, muscarinic 3 | 1 | SSc; lSSc; SSc-OS |
| **17** | 64689 | GORASP1 | golgi reassembly stacking protein 1, 65kDa | 1 | SSc; SSc-OS |
| **18** | 2997 | GYS1 | glycogen synthase 1 (muscle) | 1 | SSc; lSSc |
| **19** | 10014 | HDAC5 | histone deacetylase 5 | 1 | SSc |
| **20** | 80895 | ILKAP | integrin-linked kinaseassociated serine/threonine phosphatase 2C | 1 | SSc; lSSc |
| **21** | 27257 | LSM1 | LSM1 homolog, U6 small nuclear RNA associated (S. cerevisiae) | 1 | SSc |
| **22** | 153562 | MARVELD 2 | MARVEL domain containing 2 | 1 | SSc; lSSc |
| **23** | 4784 | NFIX | nuclear factor I/X (CCAAT-binding transcription factor) | 1 | SSc; lSSc |
| **24** | 23762 | OSBP2 | oxysterol binding protein 2 | 1 | SSc; lSSc |
| **25** | 415116 | PIM3 | pim-3 oncogene | 1 | SSc |
| **26** | 5364 | PLXNB1 | plexin B1 | 1 | SSc; |
| **27** | 11243 | PMF1 | polyamine-modulated factor 1 | 1 | SSc; dSSc; lSSc |
| **28** | 10450 | PPIE | peptidylprolyl isomerase E (cyclophilin E) | 1 | SSc; dSSc; SSc-OS |
| **29** | 63976 | PRDM16 | PR domain containing 16 | 1 | SSc |
| **30** | 26140 | TTLL3 | tubulin tyrosine ligase-like family, member 3 | 1 | SSc; dSSc; |
| **31** | 84196 | USP48 | ubiquitin specific peptidase 48 | 1 | SSc |
| **32** | 563 | AZGP1 | alpha-2-glycoprotein 1, zinc-binding | 1 | SSc; dSSc; lSSc; SSc-OS |
| **33** | 7791 | ZYX | zyxin | 1 | SSc; dSSc; |
| **34** | 55324 | ABCF3 | ATP-binding cassette, sub-family F (GCN20), member 3 | 2 | dSSc |
| **35** | 39 | ACAT2 | acetyl-Coenzyme A acetyltransferase 2 | 2 | dSSc |
| **36** | 79921 | TCEAL4 | transcription elongation factor A (SII)-like 4 | 2 | dSSc |
| **37** | 81 | ACTN4 | actinin, alpha 4 | 2 | dSSc |
| **38** | 79913 | ACTR5 | ARP5 actin-related protein 5 homolog (yeast) | 2 | dSSc |
| **39** | 216 | ALDH1A1 | aldehyde dehydrogenase 1 family, member A1 | 2 | dSSc |
| **40** | 80216 | ALPK1 | alpha-kinase 1 | 2 | dSSc |
| **41** | 321 | APBA2 | amyloid beta (A4) precursor proteinbinding, family A, member 2 | 2 | dSSc |
| **42** | 27237 | ARHGEF1 6 | Rho guanine exchange factor (GEF) 16 | 2 | dSSc |
| **43** | 8623 | ASMTL | acetylserotonin O-methyltransferase-like | 2 | dSSc |
| **44** | 23400 | ATP13A2 | ATPase type 13A2 | 2 | dSSc |
| **45** | 56946 | C11orf3 0 | chromosome 11 open reading frame 30 | 2 | dSSc |
| **46** | 56912 | C11orf6 0 | chromosome 11 open reading frame 60 | 2 | dSSc |
| **47** | 56985 | C17orf4 8 | chromosome 17 open reading frame 48 | 2 | dSSc |
| **48** | 90580 | C19orf5 2 | chromosome 19 open reading frame 52 | 2 | dSSc |
| **49** | 51507 | C20orf4 3 | chromosome 20 open reading frame 43 | 2 | dSSc |
| **50** | 55755 | CDK5RAP 2 | CDK5 regulatory subunit associated protein 2 | 2 | dSSc |
| **51** | 51727 | CMPK1 | cytidine monophosphate (UMP-CMP) kinase 1, cytosolic | 2 | dSSc |
| **52** | 10391 | CORO2B | coronin, actin binding protein, 2B | 2 | dSSc |

| **SEQ ID No.** | **Gene ID** | **Gene Symbol** | **Gene Name** | **Gruppe** | **p<0.05 und fold-change >1.5 in Gruppe** |
|---|---|---|---|---|---|
| **53** | 9377 | COX5A | cytochrome c oxidase subunit Va | 2 | dSSc |
| **54** | 1488 | CTBP2 | C-terminal binding protein 2 | 2 | dSSc |
| **55** | 8529 | CYP4F2 | cytochrome P450, family 4, subfamily F, polypeptide 2 | 2 | dSSc |
| **56** | 9909 | DENND4B | DENN/MADD domain containing 4B | 2 | dSSc |
| **57** | 10901 | DHRS4 | dehydrogenase/reductase (SDR family) member 4 | 2 | dSSc |
| **58** | 84062 | DTNBP1 | dystrobrevin binding protein 1 | 2 | dSSc |
| **59** | 1936 | EEF1D | eukaryotic translation elongation factor 1 delta (guanine nucleotide exchange protein) | 2 | dSSc |
| **60** | 8891 | EIF2B3 | eukaryotic translation initiation factor 2B, subunit 3 gamma, 58kDa | 2 | dSSc |
| **61** | 64787 | EPS8L2 | EPS8-like 2 | 2 | dSSc |
| **62** | 9638 | FEZ1 | fasciculation and elongation protein zeta 1 (zygin I) | 2 | dSSc |
| **63** | 2300 | FOXL1 | forkhead box L1 | 2 | dSSc |
| **64** | 2519 | FUCA2 | fucosidase, alpha-L- 2, plasma | 2 | dSSc |
| **65** | 79690 | GAL3ST4 | galactose-3-O-sulfotransferase 4 | 2 | dSSc |
| **66** | 54960 | GEMIN8 | gern (nuclear organelle) associated protein 8 | 2 | dSSc |
| **67** | 51031 | GLOD4 | glyoxalase domain containing 4 | 2 | dSSc |
| **68** | 2934 | GSN | gelsolin (amyloidosis, Finnish type) | 2 | dSSc |
| **69** | 3157 | HMGCS1 | 3-hydroxy-3-methyl glut aryl-Coenzyme A synthase 1 (soluble) | 2 | dSSc |
| **70** | 3320 | HSP90AA 1 | heat shock protein 90kDa alpha (cytosolic), class A member 1 | 2 | dSSc |
| **71** | 3633 | INPP5B | inositol polyphosphate-5-phosphatase, 75kDa | 2 | dSSc |
| **72** | 3654 | IRAK1 | interleukin-1 receptorassociated kinase 1 | 2 | dSSc |
| **73** | 23479 | ISCU | iron-sulfur cluster scaffold homolog (E. coli) | 2 | dSSc |
| **74** | 51520 | LARS | leucyl-tRNA synthetase | 2 | dSSc |
| **75** | 4057 | LTF | lactotransferrin | 2 | dSSc |
| **76** | 10724 | MGEA5 | meningioma expressed antigen 5 (hyaluronidase) | 2 | dSSc |
| **77** | 84954 | MPND | MPN domain containing | 2 | dSSc |
| **78** | 4437 | MSH3 | mutS homolog 3 (E. coli) | 2 | dSSc |
| **79** | 23385 | NCSTN | nicastrin | 2 | dSSc |
| **80** | 4758 | NEU1 | sialidase 1 (lysosomal sialidase) | 2 | dSSc |
| **81** | 5034 | P4HB | prolyl 4-hydroxylase, beta polypeptide | 2 | dSSc |
| **82** | 5187 | PER1 | period homolog 1 (Drosophila) | 2 | dSSc |
| **83** | 5195 | PEX14 | peroxisomal biogenesis factor 14 | 2 | dSSc |
| **84** | 10404 | PGCP | plasma glutamate carboxypeptidase | 2 | dSSc |
| **85** | 5493 | PPL | periplakin | 2 | dSSc |
| **86** | 5575 | PRKAR1B | protein kinase, cAMPdependent, regulatory, type I, beta | 2 | dSSc |
| **87** | 84867 | PTPN5 | protein tyrosine phosphatase, nonreceptor type 5 (striatum-enriched) | 2 | dSSc |
| **88** | 9230 | RAB11B | RAB11B, member RAS oncogene family | 2 | dSSc |
| **89** | 84440 | RAB11FI P4 | RAB11 family interacting protein 4 (class II) | 2 | dSSc |
| **90** | 10900 | RUNDC3A | RUN domain containing 3A | 2 | dSSc |
| **91** | 50861 | STMN3 | stathmin-like 3 | 2 | dSSc |
| **92** | 81551 | STMN4 | stathmin-like 4 | 2 | dSSc |
| **93** | 6814 | STXBP3 | syntaxin binding protein 3 | 2 | dSSc |
| **94** | 93426 | SYCE1 | synaptonemal complex central element protein 1 | 2 | dSSc |
| **95** | 6904 | TBCD | tubulin folding cofactor D | 2 | dSSc |
| **96** | 7110 | TMF1 | TATA element modulatory factor 1 | 2 | dSSc |
| **97** | 10102 | TSFM | Ts translation elongation factor, mitochondrial | 2 | dSSc |
| **98** | 7296 | TXNRD1 | thioredoxin reductase 1 | 2 | dSSc |
| **99** | 55585 | UBE2Q1 | ubiquitin-conjugating enzyme E2Q family member 1 | 2 | dSSc |
| **100** | 92912 | UBE2Q2 | ubiquitin-conjugating enzyme E2Q family member 2 | 2 | dSSc |
| **101** | 65264 | UBE2Z | ubiquitin-conjugating enzyme E2Z | 2 | dSSc |
| **102** | 54915 | YTHDF1 | YTH domain family, member 1 | 2 | dSSc |
| **103** | 7764 | ZNF217 | zinc finger protein 217 | 2 | dSSc |
| **104** | 10290 | SPEG | SPEG complex locus | 3 | lSSc |
| **105** | 84936 | ZFYVE19 | zinc finger, FYVE domain containing 19 | 3 | lSSc |
| **106** | 26574 | AATF | apoptosis antagonizing transcription factor | 3 | lSSc |
| **107** | 10152 | ABI2 | abl-interactor 2 | 3 | lSSc |
| **108** | 84320 | ACBD6 | acyl-Coenzyme A binding domain containing 6 | 3 | lSSc |
| **109** | 9049 | AIP | aryl hydrocarbon receptor interacting protein | 3 | lSSc |
| **110** | 286 | ANK1 | ankyrin 1, erythrocytic | 3 | lSSc |
| **111** | 396 | ARHGDIA | Rho GDP dissociation inhibitor (GDI) alpha | 3 | lSSc |
| **112** | 51582 | AZIN1 | antizyme inhibitor 1 | 3 | lSSc |
| **113** | 128061 | C1orf13 1 | chromosome 1 open reading frame 131 | 3 | lSSc |
| **114** | 79095 | C9orf16 | chromosome 9 open reading frame 16 | 3 | lSSc |
| **115** | 11335 | CBX3 | chromobox homolog 3 (HP1 gamma homolog, Drosophila) | 3 | lSSc |
| **116** | 92922 | CCDC102 A | coiled-coil domain containing 102A | 3 | lSSc |
| **117** | 23582 | CCNDBP1 | cyclin D-type bindingprotein 1 | 3 | lSSc |
| **118** | 64946 | CENPH | centromere protein H | 3 | lSSc |
| **119** | 79585 | CORO7 | coronin 7 | 3 | lSSc |
| **120** | 1653 | DDX1 | DEAD (Asp-Glu-Ala-Asp) box polypeptide 1 | 3 | lSSc |
| **121** | 23220 | DTX4 | deltex homolog 4 (Drosophila) | 3 | lSSc |
| **122** | 51143 | DYNC1LI 1 | dynein, cytoplasmic 1, light intermediate chain 1 | 3 | lSSc |
| **123** | 1977 | EIF4E | eukaryotic translation initiation factor 4E | 3 | lSSc |
| **124** | 256364 | EML3 | echinoderm microtubule | 3 | lSSc |
| | | | associated protein like 3 | | |
| **125** | 55740 | ENAH | enabled homolog (Drosophila) | 3 | lSSc |
| **126** | 8320 | EOMES | eomesodermin homolog (Xenopus laevis) | 3 | lSSc |
| **127** | 9130 | FAM50A | family with sequence similarity 50, member A | 3 | lSSc |
| **128** | 89848 | FCHSD1 | FCH and double SH3 domains 1 | 3 | lSSc |
| **129** | 2549 | GAB1 | GRB2-associated binding protein 1 | 3 | lSSc |
| **130** | 2653 | GCSH | glycine cleavage system protein H (aminomethyl carrier) | 3 | lSSc |
| **131** | 10755 | GIPC1 | GIPC PDZ domain containing family, member 1 | 3 | lSSc |
| **132** | 28964 | GIT1 | G protein-coupled receptor kinase interacting ArfGAP 1 | 3 | lSSc |
| **133** | 65056 | GPBP1 | GC-rich promoter binding protein 1 | 3 | lSSc |
| **134** | 2962 | GTF2F1 | general transcription factor IIF, polypeptide 1, 74kDa | 3 | lSSc |
| **135** | 3024 | HIST1H1 A | histone cluster 1, H1a | 3 | lSSc |
| **136** | 3551 | IKBKB | inhibitor of kappa light polypeptide gene enhancer in B-cells, kinase beta | 3 | lSSc |
| **137** | 57461 | ISY1 | ISY1 splicing factor homolog (S. cerevisiae) | 3 | lSSc |
| **138** | 3791 | KDR | kinase insert domain receptor (a type III receptor tyrosine kinase) | 3 | lSSc |
| **139** | 22920 | KIFAP3 | kinesin-associated protein 3 | 3 | lSSc |
| **140** | 4137 | MAPT | microtubule-associated protein tau | 3 | lSSc |
| **141** | 9412 | MED21 | mediator complex subunit 21 | 3 | lSSc |
| **142** | 55034 | MOCOS | molybdenum cofactor sulfurase | 3 | lSSc |
| **143** | 64981 | MRPL34 | mitochondrial ribosomal protein L34 | 3 | lSSc |
| **144** | 55968 | NSFL1C | NSFL1 (p97) cofactor (p47) | 3 | lSSc |
| **145** | 10130 | PDIA6 | protein disulfide isomerase family A, member 6 | 3 | lSSc |
| **146** | 55857 | PLK1S1 | polo-like kinase 1 substrate 1 | 3 | lSSc |
| **147** | 23654 | PLXNB2 | plexin B2 | 3 | lSSc |
| **148** | 6004 | RGS16 | regulator of G-protein signaling 16 | 3 | lSSc |
| **149** | 6047 | RNF4 | ring finger protein 4 | 3 | lSSc |
| **150** | 6125 | RPL5 | ribosomal protein L5 | 3 | lSSc |
| **151** | 6285 | S100B | S100 calcium binding protein B | 3 | lSSc |
| **152** | 6418 | SET | SET nuclear oncogene | 3 | lSSc |
| **153** | 6421 | SFPQ | splicing factor proline/glutamine-rich (polypyrimidine tract binding protein associated) | 3 | lSSc |
| **154** | 6456 | SH3GL2 | SH3-domain GRB2-like 2 | 3 | lSSc |
| **155** | 1059 | CENPB | centromere protein B, 80kDa | 3 | lSSc |
| **172** | 255626 | HIST1H2 BA | histone cluster 1, H2ba | 5 | lSSc; SSc-OS |
| **156** | 84501 | SPIRE2 | spire homolog 2 (Drosophila) | 3 | lSSc |
| **157** | 6709 | SPTAN1 | spectrin, alpha, nonerythrocytic 1 (alphafodrin) | 3 | lSSc |
| **158** | 6741 | SSB | Sjogren syndrome antigen B (autoantigen La) | 3 | lSSc |
| **159** | 25949 | SYF2 | SYF2 homolog, RNA splicing factor (S. cerevisiae) | 3 | lSSc |
| **160** | 6880 | TAF9 | TAF9 RNA polymerase II, TATA box binding protein (TBP)-associated factor, 32kDa | 3 | lSSc |
| **161** | 11022 | TDRKH | tudor and KH domain containing | 3 | lSSc |
| **162** | 7265 | TTC1 | tetratricopeptide repeat domain 1 | 3 | lSSc |
| **163** | 23331 | TTC28 | tetratricopeptide repeat domain 28 | 3 | lSSc |
| **164** | 11344 | TWF2 | twinfilin, actinbinding protein, homolog 2 (Drosophila) | 3 | lSSc |
| **165** | 55833 | UBAP2 | ubiquitin associated protein 2 | 3 | lSSc |
| **166** | 9094 | UNC119 | unc-119 homolog (C. elegans ) | 3 | lSSc |
| **167** | 58525 | WIZ | widely interspaced zinc finger motifs | 3 | lSSc |
| **168** | 7494 | XBP1 | X-box binding protein 1 | 3 | lSSc |
| **169** | 56252 | YLPM1 | YLP motif containing 1 | 3 | lSSc |
| **170** | 51538 | ZCCHC17 | zinc finger, CCHC domain containing 17 | 3 | lSSc |
| **171** | 84240 | ZCCHC9 | zinc finger, CCHC domain containing 9 | 3 | lSSc |
| **173** | 11332 | ACOT7 | acyl-CoA thioesterase 7 | 4 | SSc-OS |
| **174** | 10120 | ACTR1B | ARP1 actin-related protein 1 homolog B, centractin beta (yeast) | 4 | SSc-OS |
| **175** | 118 | ADD1 | adducin 1 (alpha) | 4 | SSc-OS |
| **176** | 9131 | AIFM1 | apoptosis-inducing factor, mitochondrionassociated, 1 | 4 | SSc-OS |
| **177** | 203 | AK1 | adenylate kinase 1 | 4 | SSc-OS |
| **178** | 8165 | AKAP1 | A kinase (PRKA) anchor protein 1 | 4 | SSc-OS |
| **179** | 207 | AKT1 | v-akt murine thymoma viral oncogene homolog 1 | 4 | SSc-OS |
| **180** | 29945 | ANAPC4 | anaphase promoting complex subunit 4 | 4 | SSc-OS |
| **181** | 54522 | ANKRD16 | ankyrin repeat domain 16 | 4 | SSc-OS |
| **182** | 203286 | ANKS6 | ankyrin repeat and sterile alpha motif domain containing 6 | 4 | SSc-OS |
| **183** | 324 | APC | adenomatous polyposis coli | 4 | SSc-OS |
| **184** | 397 | ARHGDIB | Rho GDP dissociation inhibitor (GDI) beta | 4 | SSc-OS |
| **185** | 140459 | ASB6 | ankyrin repeat and SOCS box-containing 6 | 4 | SSc-OS |
| **186** | 513 | ATP5D | ATP synthase, H+ transporting, mitochondrial F1 complex, delta subunit | 4 | SSc-OS |
| **187** | 10476 | ATP5H | ATP synthase, H+ transporting, mitochondrial F0 complex, subunit d | 4 | SSc-OS |
| **188** | 60370 | AVPI1 | arginine vasopressininduced 1 | 4 | SSc-OS |
| **189** | 146712 | B3GNTL1 | UDP-GlcNAc:betaGal beta-1,3-N-acetylglucosaminyltrans ferase-like 1 | 4 | SSc-OS |
| **190** | 593 | BCKDHA | branched chain keto acid dehydrogenase E1, alpha polypeptide | 4 | SSc-OS |
| **191** | 27154 | BRPF3 | bromodomain and PHD finger containing, 3 | 4 | SSc-OS |
| **192** | 64776 | C11orf1 | chromosome 11 open reading frame 1 | 4 | SSc-OS |
| **193** | 144097 | C11orf8 4 | chromosome 11 open reading frame 84 | 4 | SSc-OS |
| **194** | 55195 | C14orf1 05 | chromosome 14 open reading frame 105 | 4 | SSc-OS |
| **195** | 55257 | C20orf2 0 | chromosome 20 open reading frame 20 | 4 | SSc-OS |
| **196** | 51300 | C3orf1 | chromosome 3 open reading frame 1 | 4 | SSc-OS |
| **197** | 763 | CA5A | carbonic anhydrase VA, mitochondrial | 4 | SSc-OS |
| **198** | 794 | CALB2 | calbindin 2 | 4 | SSc-OS |
| **199** | 822 | CAPG | capping protein (actin filament), gelsolinlike | 4 | SSc-OS |
| **200** | 23624 | CBLC | Cas-Br-M (murine) ecotropic retroviral transforming sequence c | 4 | SSc-OS |
| **201** | 54862 | CC2D1A | coiled-coil and C2 domain containing 1A | 4 | SSc-OS |
| **202** | 339230 | CCDC137 | coiled-coil domain containing 137 | 4 | SSc-OS |
| **203** | 55036 | CCDC40 | coiled-coil domain containing 40 | 4 | SSc-OS |
| **204** | 124808 | CCDC43 | coiled-coil domain containing 43 | 4 | SSc-OS |
| **205** | 728642 | CDC2L2 | cell division cycle 2-like 2 (PITSLRE proteins) | 4 | SSc-OS |
| **206** | 79959 | CEP76 | centrosomal protein 76kDa | 4 | SSc-OS |
| **207** | 55748 | CNDP2 | CNDP dipeptidase 2 (metallopeptidase M20 family) | 4 | SSc-OS |
| **208** | 116840 | CNTROB | centrobin, centrosomal BRCA2 interacting protein | 4 | SSc-OS |
| **209** | 8161 | COIL | coilin | 4 | SSc-OS |
| **210** | 1410 | CRYAB | crystallin, alpha B | 4 | SSc-OS |
| **211** | 1674 | DES | desmin | 4 | SSc-OS |
| **212** | 54505 | DHX29 | DEAH (Asp-Glu-Ala-His) box polypeptide 29 | 4 | SSc-OS |
| **213** | 22982 | DIP2C | DIP2 disco-interacting protein 2 homolog C (Drosophila) | 4 | SSc-OS |
| **214** | 1810 | DR1 | down-regulator of transcription 1, TBPbinding (negative cofactor 2) | 4 | SSc-OS |
| **215** | 23741 | EID1 | EP300 interacting inhibitor of differentiation 1 | 4 | SSc-OS |
| **216** | 10613 | ERLIN1 | ER lipid raft associated 1 | 4 | SSc-OS |
| **217** | 90736 | FAM104B | family with sequence similarity 104, member B | 4 | SSc-OS |
| **218** | 58516 | FAM60A | family with sequence similarity 60, member A | 4 | SSc-OS |
| **219** | 2194 | FASN | fatty acid synthase | 4 | SSc-OS |
| **220** | 2209 | FCGR1A | Fc fragment of IgG, high affinity Ia, receptor (CD64) | 4 | SSc-OS |
| **221** | 23307 | FKBP15 | FK506 binding protein 15, 133kDa | 4 | SSc-OS |
| **222** | 23770 | FKBP8 | FK506 binding protein 8, 38kDa | 4 | SSc-OS |
| **223** | 8939 | FUBP3 | far upstream element (FUSE) binding protein 3 | 4 | SSc-OS |
| **224** | 26515 | FXC1 | fracture callus 1 homolog (rat) | 4 | SSc-OS |
| **225** | 2954 | GSTZ1 | glutathione transferase zeta 1 | 4 | SSc-OS |
| **226** | 94239 | H2AFV | H2A histone family, member V | 4 | SSc-OS |
| **227** | 3178 | HNRNPA1 | heterogeneous nuclear ribonucleoprotein A1 | 4 | SSc-OS |
| **228** | 92906 | HNRPLL | heterogeneous nuclear ribonucleoprotein L-like | 4 | SSc-OS |
| **229** | 440498 | HSBP1L1 | heat shock factor binding protein 1-like 1 | 4 | SSc-OS |
| **230** | 3312 | HSPA8 | heat shock 70kDa protein 8 | 4 | SSc-OS |
| **231** | 134728 | IRAK1BP 1 | interleukin-1 receptorassociated kinase 1 binding protein 1 | 4 | SSc-OS |
| **232** | 3735 | KARS | lysyl-tRNA synthetase | 4 | SSc-OS |
| **233** | 8645 | KCNK5 | potassium channel, subfamily K, member 5 | 4 | SSc-OS |
| **234** | 91012 | LASS5 | LAG1 homolog, ceramide synthase 5 | 4 | SSc-OS |
| **235** | 3991 | LIPE | lipase, hormonesensitive | 4 | SSc-OS |
| **236** | 1001291 19 | LOC1001 29119 | hypothetical LOC100129119 | 4 | SSc-OS |
| **237** | 643733 | LOC6437 33 | hypothetical LOC643733 | 4 | SSc-OS |
| **238** | 26065 | LSM14A | LSM14A, SCD6 homolog A (S. cerevisiae) | 4 | SSc-OS |
| **239** | 149986 | LSM14B | LSM14B, SCD6 homolog B (S. cerevisiae) | 4 | SSc-OS |
| **240** | 51599 | LSR | lipolysis stimulated lipoprotein receptor | 4 | SSc-OS |
| **241** | 51631 | LUC7L2 | LUC7-like 2 (S. cerevisiae) | 4 | SSc-OS |
| **242** | 4128 | MAOA | monoamine oxidase A | 4 | SSc-OS |
| **243** | 23542 | MAPK8IP 2 | mitogen-activated protein kinase 8 interacting protein 2 | 4 | SSc-OS |
| **244** | 53615 | MBD3 | methyl-CpG binding domain protein 3 | 4 | SSc-OS |
| **245** | 124995 | MRPL10 | mitochondrial ribosomal protein L10 | 4 | SSc-OS |
| **246** | 65003 | MRPL11 | mitochondrial ribosomal protein L11 | 4 | SSc-OS |
| **247** | 4478 | MSN | moesin | 4 | SSc-OS |
| **248** | 83463 | MXD3 | MAX dimerization protein 3 | 4 | SSc-OS |
| **249** | 4601 | MXI1 | MAX interactor 1 | 4 | SSc-OS |
| **250** | 4780 | NFE2L2 | nuclear factor (erythroid-derived 2)-like 2 | 4 | SSc-OS |
| **251** | 57224 | NHSL1 | NHS-like 1 | 4 | SSc-OS |
| **252** | 4826 | NNAT | neuronatin | 4 | SSc-OS |
| **253** | 29959 | NRBP1 | nuclear receptor binding protein 1 | 4 | SSc-OS |
| **254** | 129401 | NUP35 | nucleoporin 35kDa | 4 | SSc-OS |
| **255** | 23594 | ORC6L | origin recognition complex, subunit 6 like (yeast) | 4 | SSc-OS |
| **256** | 55229 | PANK4 | pantothenate kinase 4 | 4 | SSc-OS |
| **257** | 57326 | PBXIP1 | pre-B-cell leukemia homeobox interacting protein 1 | 4 | SSc-OS |
| **258** | 57060 | PCBP4 | poly(rC) binding protein 4 | 4 | SSc-OS |
| **259** | 94274 | PPP1R14 A | protein phosphatase 1, regulatory (inhibitor) subunit 14A | 4 | SSc-OS |
| **260** | 56978 | PRDM8 | PR domain containing 8 | 4 | SSc-OS |
| **261** | 5764 | PTN | pleiotrophin | 4 | SSc-OS |
| **262** | 6175 | RPLP0 | ribosomal protein, large, P0 | 4 | SSc-OS |
| **263** | 6188 | RPS3 | ribosomal protein S3 | 4 | SSc-OS |
| **264** | 950 | SCARB2 | scavenger receptor class B, member 2 | 4 | SSc-OS |
| **265** | 10806 | SDCCAG8 | serologically defined colon cancer antigen 8 | 4 | SSc-OS |
| **266** | 56948 | SDR39U1 | short chain dehydrogenase/reductase family 39U, member 1 | 4 | SSc-OS |
| **267** | 10993 | SDS | serine dehydratase | 4 | SSc-OS |
| **268** | 22872 | SEC31A | SEC31 homolog A (S. cerevisiae) | 4 | SSc-OS |
| **269** | 866 | SERPINA 6 | serpin peptidase inhibitor, clade A (alpha-1 antiproteinase, antitrypsin), member 6 | 4 | SSc-OS |
| **270** | 30011 | SH3KBP1 | SH3-domain kinase binding protein 1 | 4 | SSc-OS |
| **271** | 4086 | SMAD1 | SMAD family member 1 | 4 | SSc-OS |
| **272** | 79856 | SNX2 2 | sorting nexin 22 | 4 | SSc-OS |
| **273** | 9580 | SOX13 | SRY (sex determining region Y)-box 13 | 4 | SSc-OS |
| **274** | 6730 | SRP68 | signal recognition particle 68kDa | 4 | SSc-OS |
| **275** | 140597 | TCEAL2 | transcription elongation factor A (SII)-like 2 | 4 | SSc-OS |
| **276** | 6924 | TCEB3 | transcription elongation factor B (SIII), polypeptide 3 (110kDa, elongin A) | 4 | SSc-OS |
| **277** | 10915 | TCERG1 | transcription elongation regulator 1 | 4 | SSc-OS |
| **278** | 6949 | TCOF1 | Treacher Collins-Franceschetti syndrome 1 | 4 | SSc-OS |
| **279** | 26517 | TIMM13 | translocase of inner mitochondrial membrane 13 homolog (yeast) | 4 | SSc-OS |
| **280** | 22906 | TRAK1 | trafficking protein, kinesin binding 1 | 4 | SSc-OS |
| **281** | 10107 | TRIM10 | tripartite motif-containing 10 | 4 | SSc-OS |
| **282** | 81844 | TRIM56 | tripartite motifcontaining 56 | 4 | SSc-OS |
| **283** | 92181 | UBTD2 | ubiquitin domain containing 2 | 4 | SSc-OS |
| **284** | 54576 | UGT1A8 | UDP glucuronosyltransferase 1 family, polypeptide A8 | 4 | SSc-OS |
| **285** | 23074 | UHRF1BP 1L | UHRF1 binding protein 1-like | 4 | SSc-OS |
| **286** | 55031 | USP47 | ubiquitin specific peptidase 47 | 4 | SSc-OS |
| **287** | 10493 | VAT1 | vesicle amine transport protein 1 homolog (T. californica) | 4 | SSc-OS |
| **288** | 22911 | WDR47 | WD repeat domain 47 | 4 | SSc-OS |
| **289** | 23613 | ZMYND8 | zinc finger, MYND-type containing 8 | 4 | SSc-OS |
| **290** | 170959 | ZNF431 | zinc finger protein 431 | 4 | SSc-OS |
| **291** | 147837 | ZNF563 | zinc finger protein 563 | 4 | SSc-OS |
| **292** | 4747 | NEFL | neurofilament, light polypeptide | 5 | lSSc; SSc-OS; dSSc; |
| **293** | 3925 | STMN1 | stathmin 1 | 5 | lSSc; SSc-OS; dSSC |
| **294** | 1039 | CDR2 | cerebellar degeneration-related protein 2, 62kDa | 5 | lSSc; SSc-OS |
| **295** | 5504 | PPP1R2 | protein phosphatase 1, regulatory (inhibitor) subunit 2 | 5 | lSSc; SSc-OS |
| **296** | 55131 | RBM28 | RNA binding motif protein 28 | 5 | lSSc; SSc-OS |
| **297** | 6749 | SSRP1 | structure specific recognition protein 1 | 5 | lSSc; SSc-OS |
| **298** | 54969 | C4orf27 | chromosome 4 open reading frame 27 | 5 | dSSc; lSSc |
| **299** | 784 | CACNB3 | calcium channel, voltage-dependent, beta 3 subunit | 5 | dSSc; lSSc |
| **300** | 842 | CASP9 | caspase 9, apoptosisrelated cysteine peptidase | 5 | dSSc; lSSc |
| **301** | 1105 | CHD1 | chromodomain helicase DNA binding protein 1 | 5 | dSSc; lSSc |
| **302** | 1687 | DFNA5 | deafness, autosomal dominant 5 | 5 | dSSc; lSSc |
| **303** | 2237 | FEN1 | flap structure-specific endonuclease 1 | 5 | dSSc; lSSc |
| 304 | 2961 | GTF2E2 | general transcription factor IIE, polypeptide 2, beta 34kDa | 5 | dSSc; lSSc |
| **305** | 4313 | MMP2 | matrix metallopeptidase 2 (gelatinase A, 72kDa gelatinase, 72kDa type IV collagenase) | 5 | dSSc; lSSc |
| **306** | 64976 | MRPL40 | mitochondrial ribosomal protein L40 | 5 | dSSc; lSSc |
| **307** | 8775 | NAPA | N-ethylmaleimidesensitive factor attachment protein, alpha | 5 | dSSc; lSSc |
| **308** | 1001370 49 | PLA2G4B | phospholipase A2, group IVB (cytosolic) | 5 | dSSc; lSSc |
| **309** | 5515 | PPP2CA | protein phosphatase 2 (formerly 2A), catalytic subunit, alpha isoform | 5 | dSSc; lSSc |
| **310** | 5819 | PVRL2 | poliovirus receptorrelated 2 (herpesvirus entry mediator B) | 5 | dSSc; lSSc |
| **311** | 9400 | RECQL5 | RecQ protein-like 5 | 5 | dSSc; lSSc |
| **312** | 11124 | FAF1 | Fas (TNFRSF6) associated factor 1 | 5 | SSc-OS; dSSc |
| **313** | 54521 | WDR44 | WD repeat domain 44 | 5 | dSSc; SSc-OS |
| **314** | 7150 | TOP1 | TOP1 | 1 | SSc |
| **315** | 23135 | KDM6B | KDM6B | 1 | SSc;dSSc;SS c-OS; |
| **316** | 55695 | NSUN5 | NSUN5 | 1 | SSc;lSSc |
| **317** | 7644 | ZNF91 | ZNF91 | 2 | dSSc |
| **318** | / | / | PGSScAg318 | 1 | SSc |
| **319** | 7 | / | PGSScAg319 | 1 | SSc |

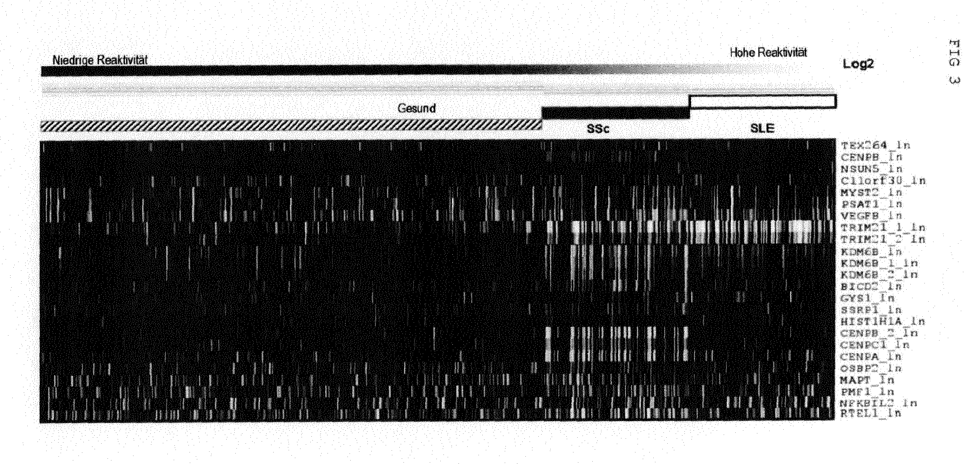

Figur 3 zeigt die Autoantikörperreaktivität von SSc Patienten im Vergleich zu Gesunden Spendern und SLE Patienten. Dargestellt ist eine sogenannte Heatmap der logarithmierten MFI Werte, wobei Signalhöhe in einer Schwarz/Weiß Skala wiedergegeben wird.

Nach univariater statistischer Auswertung wurde ein Schwellenwert von p<0.05 und eine 1,5 fach veränderte Reaktivität gegenüber der Kontrollgruppe angelegt.

Gruppe 1 umfasst Antigene, die in der Gruppe aller SSc Patienten im Vergleicht zu Gesundkontrollen und/oder anderen rheumatischen Erkrankungen einen Schwellenwert von p<0.05 unterschreiten und eine 1,5 fach veränderte Reaktivität gegenüber der Kontrollgruppe erreichen: KDM6B, NSUN5, RTEL1, MYST2, TRIM21, ABCB8, AVEN, VEGFB, C19orf60, CENPA, CENPC1, CENPT, CHRM3, GORASP1, GYS1, HDAC5, ILKAP, LSM1, MARVELD2, NFIX, OSBP2, PIM3, PLXNB1, PMF1, PPIE, PRDM16, TTLL3, USP48, AZGP1, AZGP1, ZYX.

Tabelle 3 fasst die Ergebnisse der statistischen Tests für 36 Antigene aus Tabelle 2 zusammen, die einen p-Wert von <0.05 gegenüber Gesundproben aufweisen.

Angegeben sind der p-Wert (p-value), die Erhöhung der Reaktivität gegenüber der Kontrollgruppe (Fold-Change), die Area under the Curve (AUC) und der Confidence Interval (CI), sowie Sensitivität (Sens.) und Spezifität (Spec.).

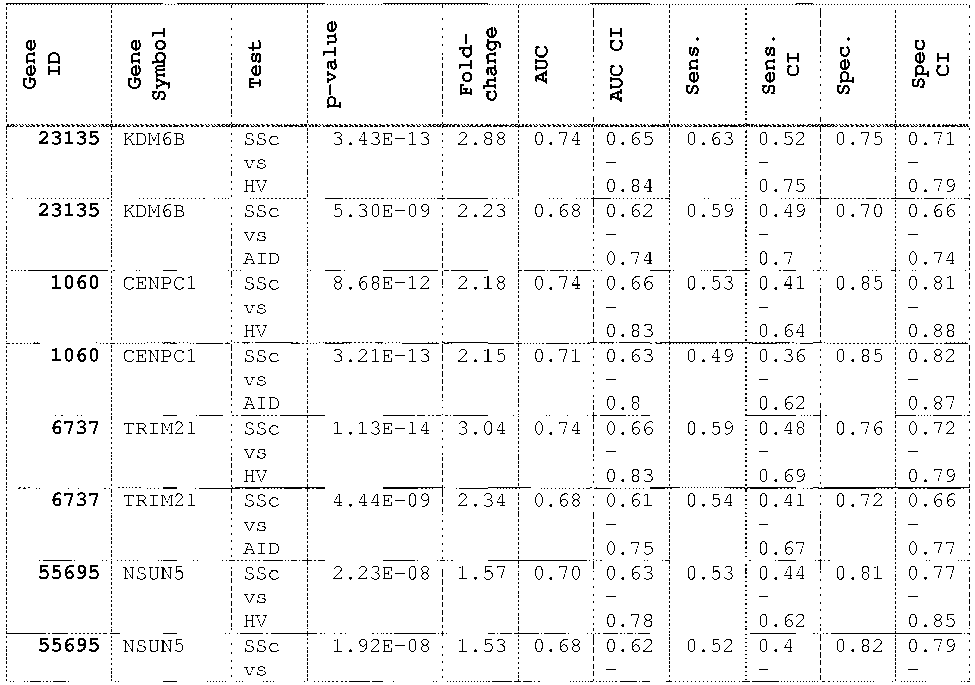

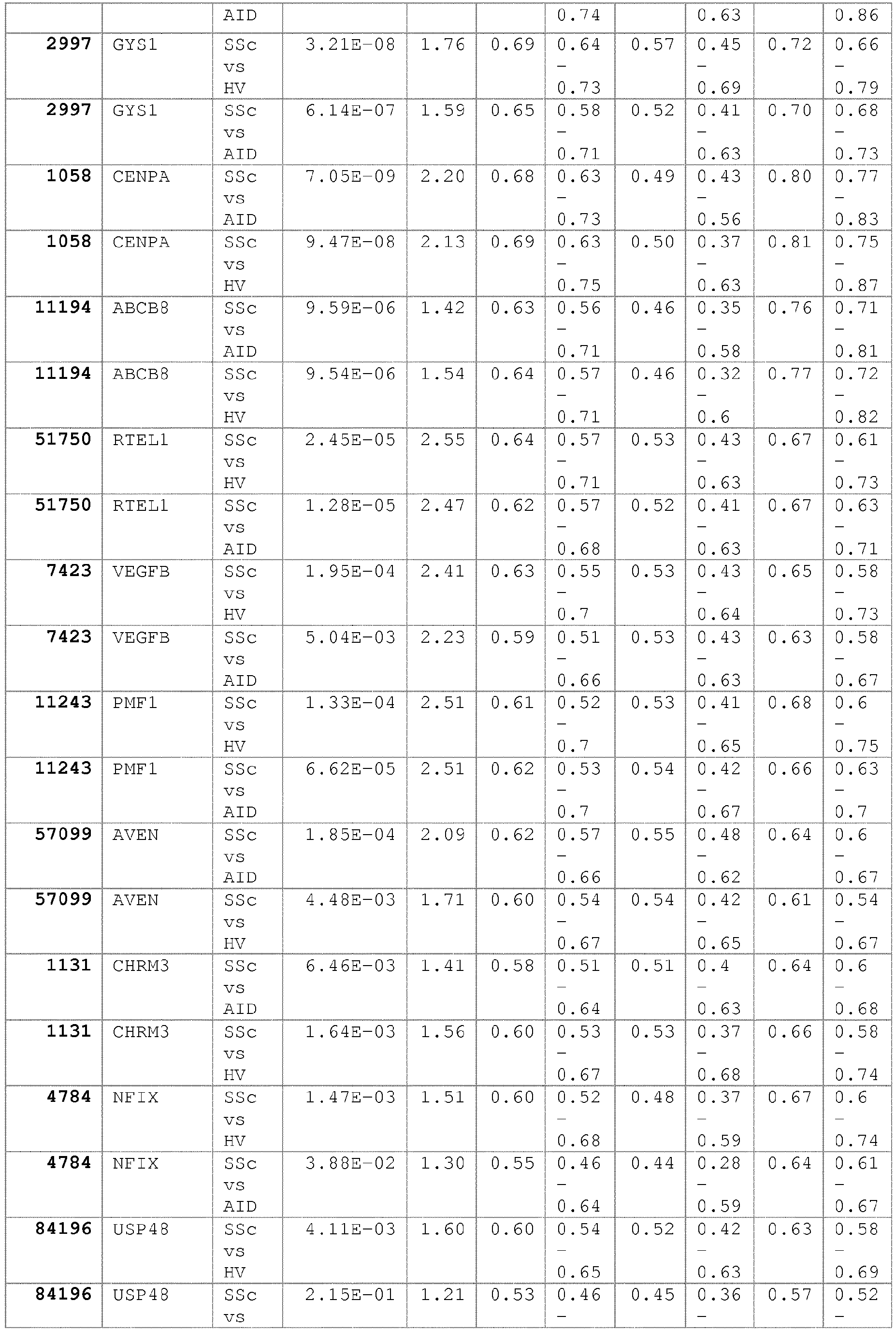

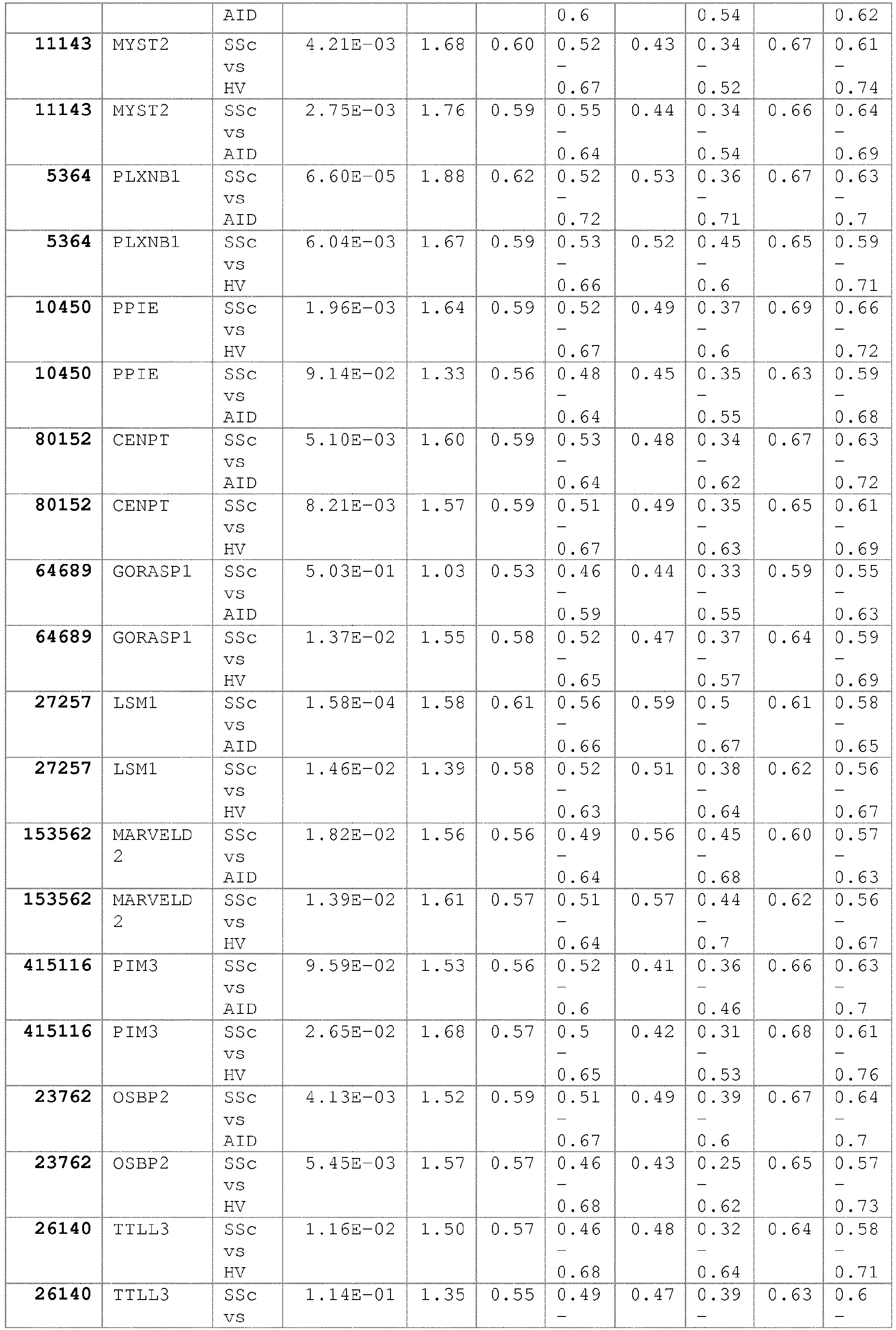

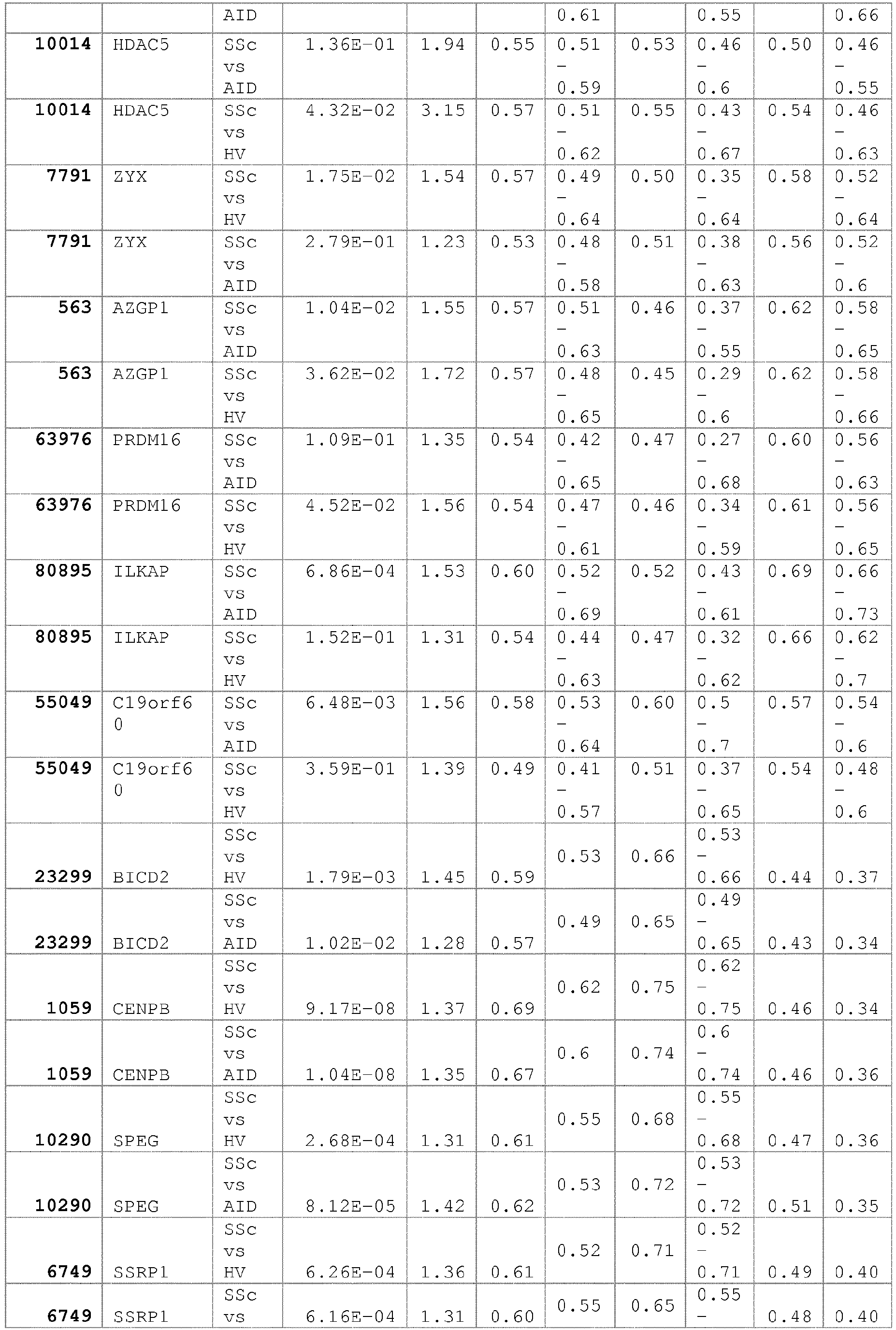

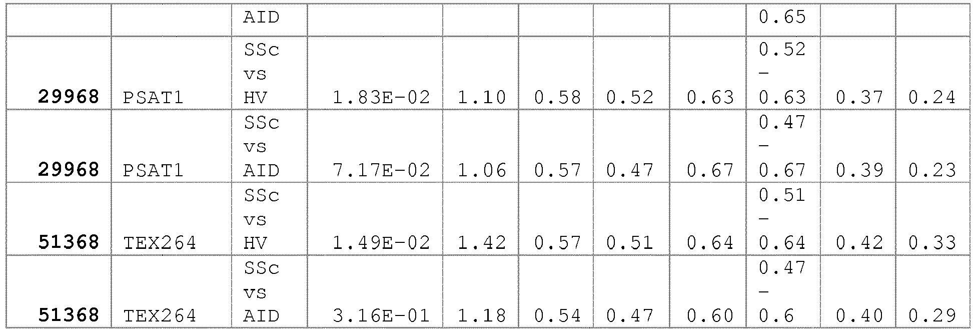

Um die Frequenz der neuidentifizierten Antigene aus Tabelle 3 im Vergleich zu bekannten Antigenen zu analysieren, wurde ein Schwellenwert von 3 Standardabweichungen (SD) über dem Mittelwert der Gesundproben definiert.

Erstaunlicherweise wurden mindestens 6 zusätzliche Antigene identifiziert, deren Frequenz bei Betrachtung aller SSc Patienten größer gleich 10% ist. Dazu gehören KDM6B, (21%), BICD2 (19%), RTEL1 (14%), NSUN5 (13%), SSRP1 (10%) und SPEG (10%).Zusätzlich wurden zwei weitere Antigene mit einer Frequenz größer oder gleich 5% in SSc gefunden: VEGFB(9%) und PSAT1 (7%).

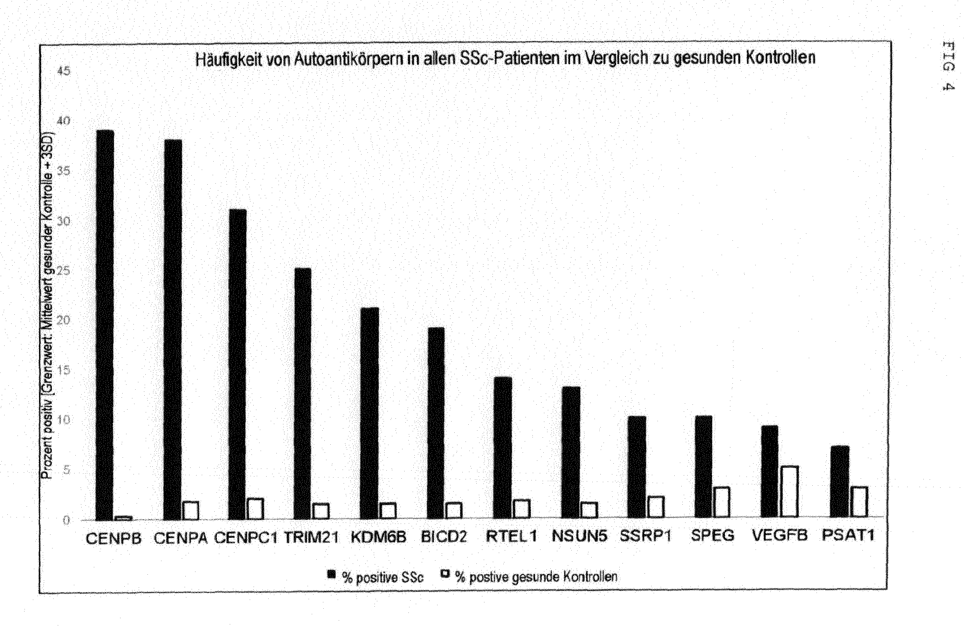

Figur 4 zeigt die Frequenz von 8 neuen Antigenen in SSc Patienten im Vergleich zu anti-Centromere Antikörpern.

Beispiel 7: Identifizierung von Autoantikörper Reaktivitäten in Patienten mit diffuser Subform.

Nur etwa 62.5% der analysierten Patienten mit limitierter Form wiesen Anti-Scl70 Antikörper auf. Weitere 12.5% der Patienten zeigten anti-Centromere Antikörper. Um weitere Autoantikörper in Patienten mit limitierter SSc zu identifizieren, wurden die Serumproben der Patienten mit diffuser SSc mit verschiedenen Kontrollgruppen verglichen. Diese bestanden aus Patienten mit limitierter SSc und Patienten mit Overlap Syndrome. Autoantikörper, die in der Gruppe der SSc Patienten mit diffuser Form einen p-Wert von kleiner als 0.05 und einen Fold-Change größer als 1.5 aufwiesen wurden ausgewählt. Das Ergebnis der statistischen Tests ist in Tabelle 2 zusammengefasst.

Gruppe 2 umfasst 72 zusätzliche Antigene, die für die Identifizierung von Patienten mit diffuser SSc geeignet sind.

Gruppe 2 Antigene Gene Symbol: PSAT1, TEX264, ABCF3, ACAT2, TCEAL4, ACTN4, ACTR5, ALDH1A1, ALPK1, APBA2, ARHGEF16, ASMTL, ATP13A2, C11orf30, C11orf60, C17orf48, C19orf52, C20orf43, CDK5RAP2, CMPK1, CORO2B, COX5A, CTBP2, CYP4F2, DENND4B, DHRS4, DTNBP1, EEF1D, EIF2B3, EPS8L2, FEZ1, FOXL1, FUCA2, GAL3ST4, GEMIN8, GLOD4, GSN, HMGCS1, HSP90AA1, INPP5B, IRAK1, ISCU, LARS, LTF, MGEA5, MPND, MSH3, NCSTN, NEU1, P4HB, PER1, PEX14, PGCP, PPL, PRKAR1B, PTPN5, RAB11B, RAB11FIP4, RUNDC3A, STMN3, STMN4, STXBP3, SYCE1, TBCD, TMF1, TSFM, TXNRD1, UBE2Q1, UBE2Q2, UBE2Z, YTHDF1, ZNF217.

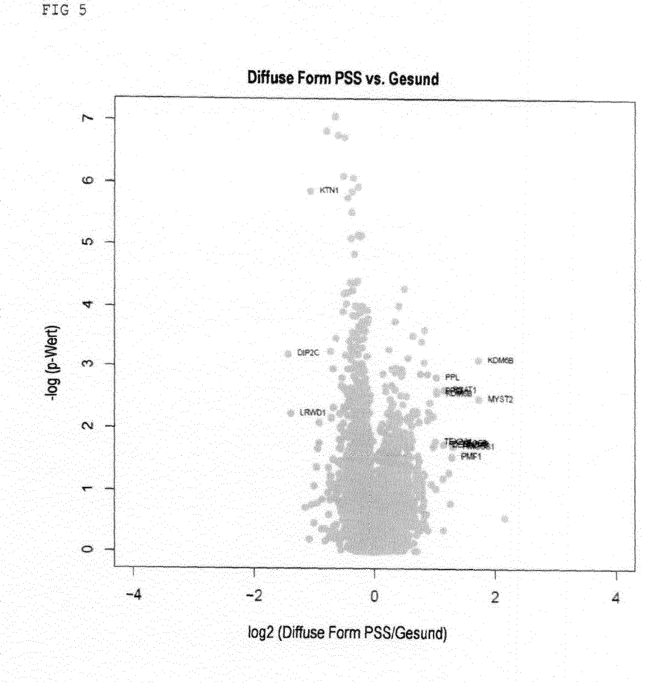

Figur 5 zeigt den Vulcano Plot der Autoantikörperreaktivitäten von SSc Patienten mit diffuser Subform gegen Gesundkontrollen.

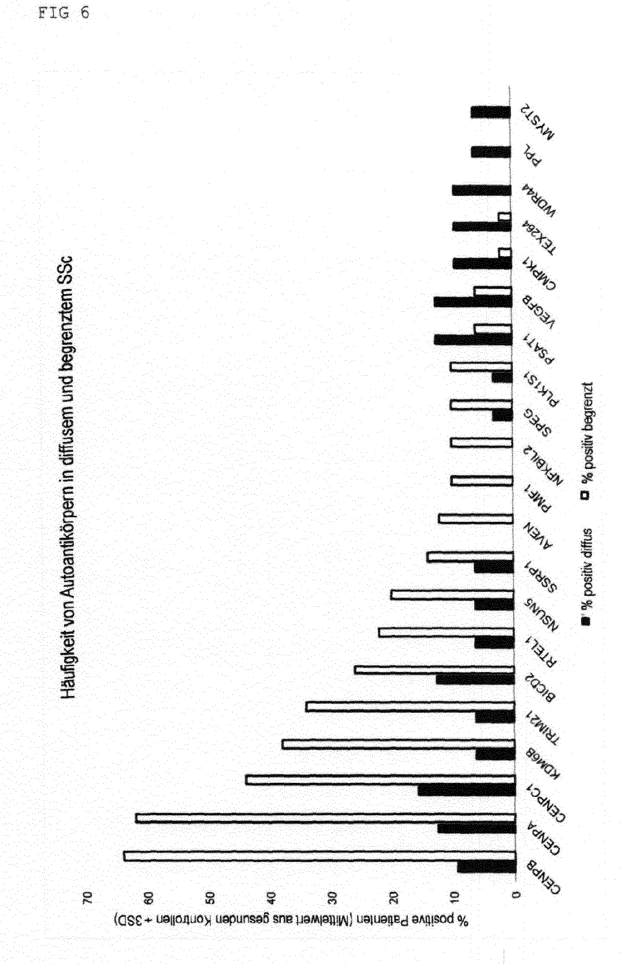

Figur 6 zeigt die Frequenz der Autantikörper in der diffusen Form im Vergleich zur limitierten Form.

Patienten mit diffuser SSc weisen häufiger Antikörper für die Antigene PSAT1 (12.5%), VEGFB (12.5%), CMPK1 (9.4%), TEX264 (9.4%), WDR44 (9.4%), PPL (6.3%) und MYST2 (6.3%) auf.

Tabelle 4 fasst die Ergebnisse der statischen Tests für ausgewählte Antigene der Gruppe 1 und 2 zusammen. Diese Antigene sind zur Identifizierung und Unterscheidung der SSc-Subgruppen limitiert und diffus geeignet.

**Tabelle 4: Zusammenfassung der p-values, AUC und Fold-Change Reaktivität der Antigene in den SSc-Subgruppen limitiert und diffuse zusammen.**

| **GeneID** | **Gene Symbol** | **Test** | **p-value** | **Fold-change** | **AUC** | **Sensitivi ty** | **Specifici ty** |
|---|---|---|---|---|---|---|---|
| **57099** | AVEN | Diffuse vs HV | 6.04E-01 | -1.46 | 0.4 4 | 0.48 | 0.56 |
| **57099** | AVEN | Limited vs HV | 1.74E-06 | 4.00 | 0.7 0 | 0.63 | 0.67 |
| **23299** | BICD2 | Diffuse vs HV | 7.98E-01 | 1.05 | 0.4 5 | 0.30 | 0.56 |
| **23299** | BICD2 | Limited vs HV | 2.53E-05 | 2.10 | 0.7 1 | 0.59 | 0.71 |
| **1058** | CENPA | Diffuse vs HV | 3.21E-01 | 1.26 | 0.5 5 | 0.35 | 0.70 |
| **1058** | CENPA | Limited vs HV | 9.49E-11 | 31.39 | 0.7 9 | 0.70 | 0.85 |
| **1059** | CENPB | Diffuse vs HV | 8.87E-01 | -1.05 | 0.5 0 | 0.36 | 0.63 |
| **1059** | CENPB | Limited vs HV | 7.69E-12 | 28.22 | 0.8 2 | 0.72 | 0.91 |
| **1060** | CENPC1 | Diffuse vs HV | 2.96E-02 | 1.51 | 0.6 3 | 0.45 | 0.77 |
| **1060** | CENPC1 | Limited vs HV | 7.42E-16 | 12.12 | 0.8 4 | 0.63 | 0.90 |
| **51727** | CMPK1 | Diffuse vs HV | 1.14E-01 | 1.73 | 0.6 0 | 0.55 | 0.60 |
| **51727** | CMPK1 | Limited vs HV | 5.98E-02 | -1.51 | 0.6 0 | 0.64 | 0.48 |
| **23135** | KDM6B | Diffuse vs HV | 2.82E-03 | 2.06 | 0.6 5 | 0.63 | 0.67 |
| **23135** | KDM6B | Limited vs HV | 1.82E-14 | 7.15 | 0.8 4 | 0.70 | 0.82 |
| **11143** | MYST2 | Diffuse vs HV | 3.44E-03 | 3.32 | 0.6 5 | 0.55 | 0.71 |
| **11143** | MYST2 | Limited vs HV | 1.95E-01 | 1.33 | 0.5 6 | 0.45 | 0.63 |
| **4796** | NFKBIL2 | Diffuse vs HV | 1.21E-01 | 1.47 | 0.5 7 | 0.39 | 0.69 |
| **4796** | NFKBIL2 | Limited vs HV | 9.90E-03 | 2.42 | 0.6 4 | 0.56 | 0.73 |
| **55695** | NSUN5 | Diffuse vs HV | 2.02E-01 | 1.05 | 0.5 8 | 0.42 | 0.75 |
| **55695** | NSUN5 | Limited vs HV | 4.07E-12 | 2.69 | 0.8 1 | 0.71 | 0.87 |
| **55857** | PLK1S1 | Diffuse vs HV | 2.01E-01 | -1.39 | 0.5 5 | 0.61 | 0.55 |
| **55857** | PLK1S1 | Limited vs HV | 9.02E-03 | 1.65 | 0.6 0 | 0.49 | 0.61 |
| **11243** | PMF1 | Diffuse vs HV | 2.89E-02 | 2.45 | 0.6 4 | 0.60 | 0.67 |
| **11243** | PMF1 | Limited vs HV | 5.75E-04 | 3.29 | 0.6 4 | 0.55 | 0.69 |
| **5493** | PPL | Diffuse vs HV | 1.53E-03 | 2.05 | 0.6 5 | 0.53 | 0.68 |
| **5493** | PPL | Limited vs HV | 8.45E-02 | 1.30 | 0.5 8 | 0.50 | 0.64 |
| **29968** | PSAT1 | Diffuse vs HV | 2.46E-03 | 2.24 | 0.6 6 | 0.52 | 0.80 |
| **29968** | PSAT1 | Limited vs HV | 6.76E-01 | 1.01 | 0.5 2 | 0.33 | 0.75 |
| **51750** | RTEL1 | Diffuse vs HV | 3.30E-01 | -1.35 | 0.4 5 | 0.49 | 0.48 |
| **51750** | RTEL1 | Limited vs HV | 5.56E-10 | 5.97 | 0.7 8 | 0.74 | 0.75 |
| **10290** | SPEG | Diffuse vs HV | 2.46E-01 | 1.02 | 0.5 4 | 0.33 | 0.55 |
| **10290** | SPEG | Limited vs HV | 7.89E-04 | 2.34 | 0.6 9 | 0.61 | 0.62 |
| **6749** | SSRP1 | Diffuse vs HV | 2.45E-01 | -1.15 | 0.4 7 | 0.40 | 0.54 |
| **6749** | SSRP1 | Limited vs HV | 1.37E-05 | 2.11 | 0.7 1 | 0.62 | 0.73 |
| **51368** | TEX264 | Diffuse vs HV | 1.66E-02 | 2.02 | 0.6 3 | 0.52 | 0.70 |
| **51368** | TEX264 | Limited vs HV | 1.12E-01 | 1.31 | 0.5 7 | 0.36 | 0.64 |
| **7423** | VEGFB | Diffuse vs HV | 1.77E-02 | 2.46 | 0.6 2 | 0.54 | 0.66 |
| **7423** | VEGFB | Limited vs HV | 8.40E-03 | 2.15 | 0.6 1 | 0.56 | 0.64 |
| **54521** | WDR44 | Diffuse vs HV | 5.07E-02 | 2.37 | 0.6 4 | 0.57 | 0.61 |
| **54521** | WDR44 | Limited vs HV | 3.00E-01 | -1.21 | 0.5 6 | 0.58 | 0.44 |

Beispiel 8: Identifizierung von Autoantikörper Reaktivitäten in Patienten mit limitierter Subform.

Nur etwa 60% der analysierten Patienten mit limitierter Form wiesen Anti-Centromere Antikörper auf. Weitere 18% der Patienten zeigten anti-Scl70 Antikörper. Um weitere Autoantikörper in Patienten mit limitierter SSc zu identifizieren, wurden die Serumproben der Patienten mit limitierter SSc mit verschiedenen Kontrollgruppen verglichen. Diese bestanden aus Patienten mit diffuser SSc und Patienten mit Overlap Syndrome. Autoantikörper, die in der Gruppe der SSc Patienten mit limitierter Form einen p-Wert von kleiner als 0.05 und einen Fold-Change größer als 1.5 aufwiesen wurden ausgewählt. Das Ergebnis der statistischen Tests ist in Tabelle 2 zusammengefasst.

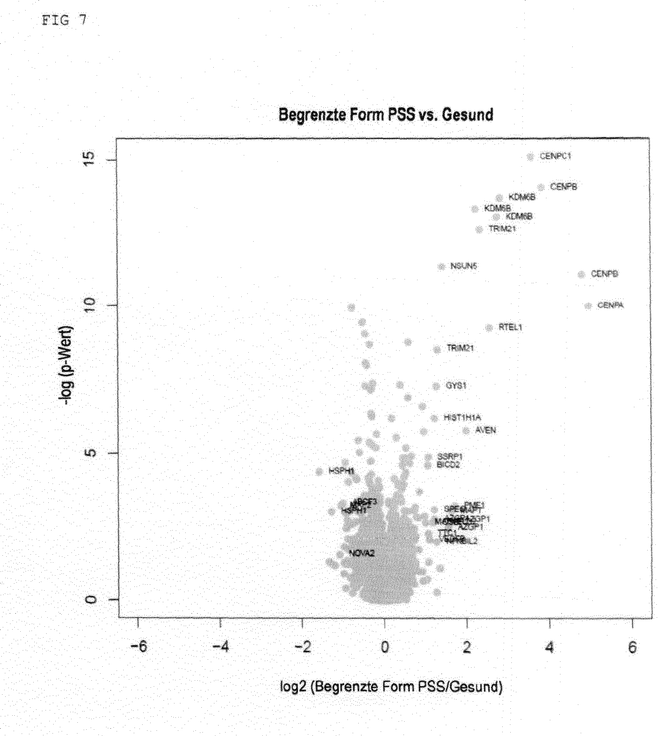

Figur 7 zeigt den Vulcano Plot der Autoantikörperreaktivitäten von Patienten mit limitierter SSc im Vergleich zu Gesundproben.

Tabelle 4 fasst die Ergebnisse der statistischen Tests der SSc-Subgruppen limitiert und diffus zusammen.

Gruppe 3 umfasst 69 zusätzliche Antigene, die für die Identifizierung von Patienten mit limitierter SSc geeignet sind.

Gruppe 3 Antigene Gene Symbol:
BICD2, SPEG, ZFYVE19, AATF, ABI2, ACBD6, AIP, ANK1, ARHGDIA, AZIN1, C1orf131, C9orf16, CBX3, CCDC102A, CCNDBP1, CENPH, CORO7, DDX1, DTX4, DYNC1LI1, EIF4E, EML3, ENAH, EOMES, FAM50A, FCHSD1, GAB1, GCSH, GIPC1, GIT1, GPBP1, GTF2F1, HIST1H1A, IKBKB, ISY1, KDR, KIFAP3, MAPT, MED21, MOCOS, MRPL34, NSFL1C, PDIA6, PLK1S1, PLXNB2, RGS16, RNF4, RPL5, S100B, SET, SFPQ, SH3GL2, CENPB, SPIRE2, SPTAN1, SSB, SYF2, TAF9, TDRKH, TTC1, TTC28, TWF2, UBAP2, UNC119, WIZ, XBP1, YLPM1, ZCCHC17, ZCCHC9

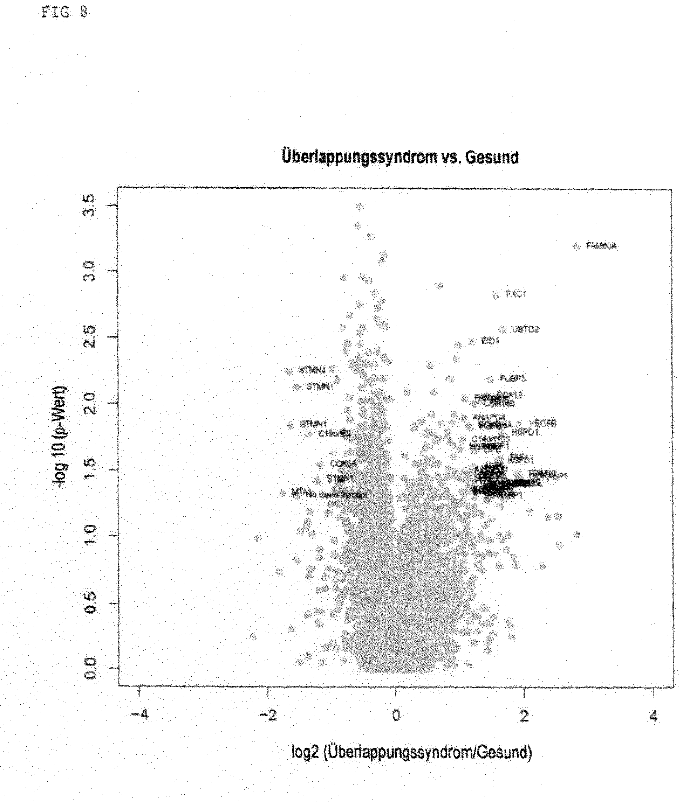

Figur 8 zeigt die Frequenz der Autoantikörper in der diffusen Form im Vergleich zur limitierten Form.

Neben anti-Centromere und anti-Ro52 (TRIM21) Antikörpern wurden in Patienten mit limitierter SSc mit einer Frequenz von größer gleich 10% Antikörper gegen die Antigene KDM6B (38%), BICD2 (26%), RTEL1 (22%), NSUN5 (20%), SSRP1 (14%), AVEN (12%), PMF1 (10%), NFKBIL2 (10%),SPEG (10%) und PLK1S1 (10%) gefunden.

Beispiel 9: Identifizierung von Autoantikörper Reaktivitäten in Patienten mit Overlap Syndrome

Eine häufige Verlaufsform der SSc ist das sogenannte Überlappungssyndrom oder Overlap Syndrome. In der klinischen Praxis ist die Abgrenzung zu anderen Kollagenosen oftmals schwierig.

Gruppe 4 umfasst 119 Antigene, die zur Identifizierung von Patienten mit Overlap Syndrome geeignet sind:
Gruppe 4 Antigene Gene Symbol:ACOT7, ACTR1B, ADD1, AIFM1, AK1, AKAP1, AKT1, ANAPC4, ANKRD16, ANKS6, APC, ARHGDIB, ASB6, ATP5D, ATP5H, AVPI1, B3GNTL1, BCKDHA, BRPF3, C11orf1, C11orf84, C14orf105, C20orf20, C3orf1, CA5A, CALB2, CAPG, CBLC, CC2D1A, CCDC137, CCDC40, CCDC43, CDC2L2, CEP76, CNDP2, CNTROB, COIL, CRYAB, DES, DHX29, DIP2C, DR1, EID1, ERLIN1, FAM104B, FAM60A, FASN, FCGR1A, FKBP15, FKBP8, FUBP3, FXC1, GSTZ1, H2AFV, HNRNPA1, HNRPLL, HSBP1L1, HSPA8, IRAK1BP1, KARS, KCNK5, LASS5, LIPE, LOC100129119, LOC643733, LSM14A, LSM14B, LSR, LUC7L2, MAOA, MAPK8IP2, MBD3, MRPL10, MRPL11, MSN, MXD3, MXI1, NFE2L2, NHSL1, NNAT, NRBP1, NUP35, ORC6L, PANK4, PBXIP1, PCBP4, PPP1R14A, PRDM8, PTN, RPLP0, RPS3, SCARB2, SDCCAG8, SDR39U1, SDS, SEC31A, SERPINA6, SH3KBP1, SMAD1, SNX22, SOX13, SRP68, TCEAL2, TCEB3, TCERG1, TCOF1, TIMM13, TRAK1, TRIM10, TRIM56, UBTD2, UGT1A8, UHRF1BP1L, USP47, VAT1, WDR47, ZMYND8, ZNF431, ZNF563.

Gruppe 5 in Tabelle 2 enthält weitere statistisch signifikante Antigene, die für die Diagnose und Differentialdiagnose von SSc gegenüber Gesund und anderen Autoimmunerkrankungen herangezogen werden können.

Gruppe 5 Antigene Gene Symbol: HIST1H2BA, NEFL, STMN1, STMN1, CDR2, PPP1R2, RBM28, SSRP1, C4orf27, CACNB3, CASP9, CHD1, DFNA5, FEN1, GTF2E2, MMP2, MRPL40, NAPA, PLA2G4B, PPP2CA, PVRL2, RECQL5, FAF1, WDR44.

Beispiel 10: Anwendung von Antigen Panels und zur verbesserten Diagnose von SSc

Aufgrund der klinischen und serologischen Heterogenität der SSc Erkrankung ist es nicht möglich mit nur einem Biomarker diese zu diagnostizieren. Laut Mierau et al. (2011) weisen nur ca. 35.9% der SSc Patienten Autoantikörper gegen Centromere-Proteine und nur 30.1% Autoantikörper gegen anti-topoisomerase I (anti-Scl70) auf. Daher besteht weiterhin ein hoher Bedarf an spezifischen diagnostischen und prognostischen Markern.

Um ein verbessertes diagnostisches Antigenpanel für SSc zu entwickeln, wurden aus den Tabellen 3 und 4 Antigene ausgewählt.

In die engere Auswahl gelangten zunächst fünf der in SSc am häufigsten vertretenen Antigene KDM6B, BICD2, RTEL1 und NSUN5 (Figur 4).

Zusätzlich berücksichtigt wurde auch die Häufigkeit von Autoantikörpern in der besonders schwer verlaufenden diffusen SSc. Hier wurden zwei der am häufigsten reaktiven Antigene BICD2 (12.5) und PSAT1 (12.5) ausgewählt.

Zusätzlich wurden Antigene ausgewählt, gegen die anti-Scl70 negative und anti-Centromere negative Patienten reaktiv sind.

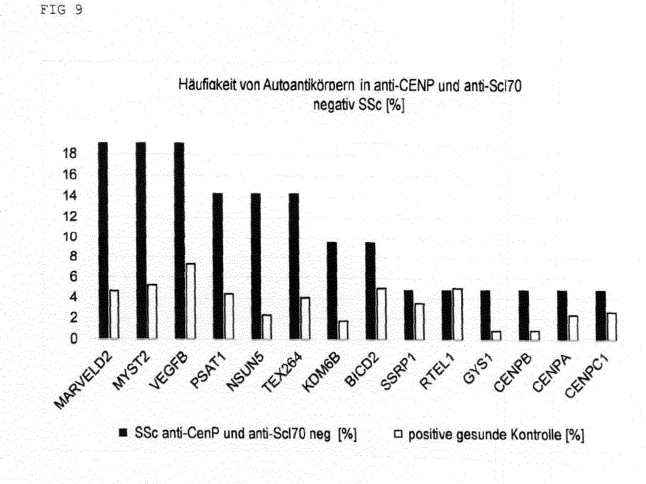

Figur 9 zeigt die Häufigkeit von Autoantikörpern gegen eine Auswahl von Antigenen aus Tabelle 3 und 4 in anti-Centromere und anti-Scl70 negativen Patienten.

Insbesondere die Antigene MARVELD2 (19%), MYST2 (19%), VEGFB (19%) .PSAT1 (14%) . NSUN5 (14%) und TEX264 (14%) weisen eine Frequenz von mehr als 10% in anti-Centromere und anti-Scl70 negativen SSc Patienten auf. Diese Antigene sind daher besonders zur Verbesserung der Diagnose geeignet.

Für die Entwicklung eines verbesserten diagnostischen Tests wurden daher vier der am häufigsten in doppelt negativen SSc Patienten reaktiven Antigene MYST2, PSAT1, NSUN5 und TEX264 ausgewählt.

Die finale Zusammensetzung der Panels ist in Tabelle 5 dargestellt.

**Tabelle 5: Zusammensetzung des verbesserten diagnostischen Panels.**

| Gene ID | Gene Symbol | Gene Name | Panel I | Panel II |
|---|---|---|---|---|
| 1059 | CENPB | centromere protein B, 80kDa | x | x |
| 7250 | TOP1 | topoisomerase (DNA) I | x | x |
| 23135 | KDM6B | lysine (K)-specific demethylase 6B | | x |
| 23299 | BICD2 | bicaudal D homolog 2 (Drosophila) | | x |
| 55695 | NSUN5 | NOL1/NOP2/Sun domain family, member 5 | | x |
| 51750 | RTEL1 | regulator of telomere elongation helicase 1 | | x |
| 11143 | MYST2 | MYST histone acetyltransferase 2 | | x |
| 29968 | PSAT1 | phosphoserine aminotransferase 1 | | x |

Die ersten 7 Antigene in Tabelle 2 umfassen die wichtigsten Antigene, die für die Berechnung von Biomarker-Panels zur Diagnose von SSc verwendet werden: KDM6B, NSUN5, BICD2, RTEL1, MYST2, PSAT1 und TEX264

Zur Validierung der in Tabelle 5 genannten Antigene aus Panel I und Panel II wurden 100 SSc Patienten und 100 Gesundproben mit Antigen-gekoppelten Luminex Beads vermessen.

Die MFI Werte der Antigene TOP1, CENPB, KDM6B, NSUN5, BICD2, RTEL1, MYST2, PSAT1 und TEX264 wurden zur Berechnung der Area under the Curve (AUC), Sensitivität und Spezifität herangezogen.

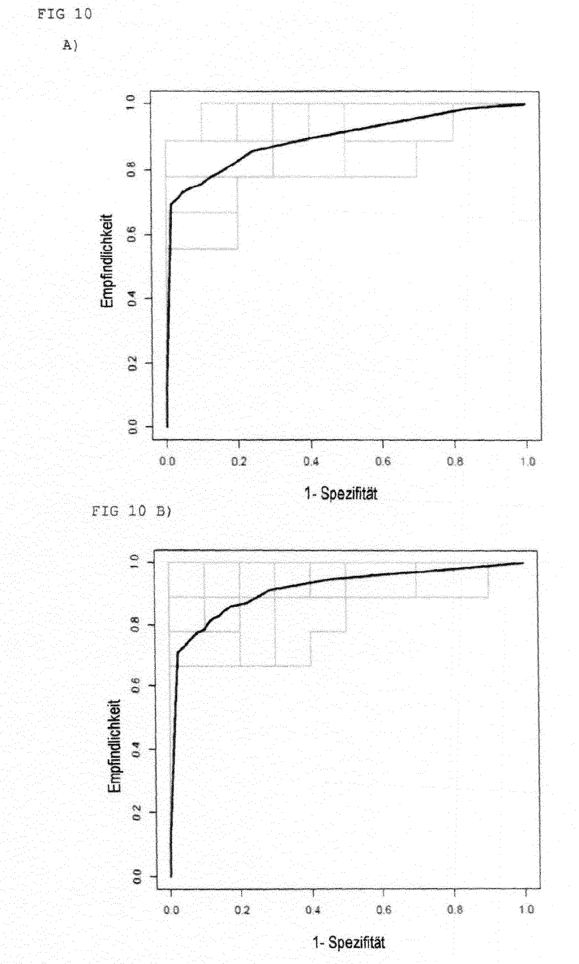

Figur 10a zeigt die Receiver Operator Kurven (ROC) für ein logistisches Regressionsmodel basierend auf dem Panel I bestehend aus anti-CENPB und anti-Scl70.

Figur 10b die ROC Kurve für das verbesserte Panel bestehend aus anti-CENPB und anti-Scl70 und den fünf neuen Antigenen KDM6B, NSUN5, BICD2, RTEL1, MYST2, PSAT1 und TEX264.

Durch den Einschluss der 5 Antigene KDM6B, NSUN5, BICD2, RTEL1, MYST2, PSAT1 und TEX264 konnte die Sensitivität um 7% von 72% auf 79% gesteigert werden.

Anhand des Beispiels wird deutlich, wie sich unter Einbeziehung der zusätzlichen Marker die Vorhersagequalität eines Biomarkermodells steigern lässt um eine bessere Klassifikation von Erkrankten zu erreichen.

Die Einbeziehung zusätzlicher Marker zu den bekannten Markern verbessert die Vorhersagequalität im angewendeten Verfahren mit etwa 7% ROC deutlich.

**Tabelle 6: AUC, Sensitivität und Spezifität der SSc Panels**

| **SSc vs Healthy Control** | **AUC** | **CI (AUC)** | **Sens.** | **CI (Sens.)** | **Spec.** | **CI (Spec)** |
|---|---|---|---|---|---|---|
| panel I | 0.91 | [0.862, 0.965] | 0.72 | [0.621, 0.823] | 0.97 | [0.935, 1] |
| panel II | 0.94 | [0.891, 0.98] | 0.79 | [0.71, 0.868] | 0.94 | [0.88, 1] |

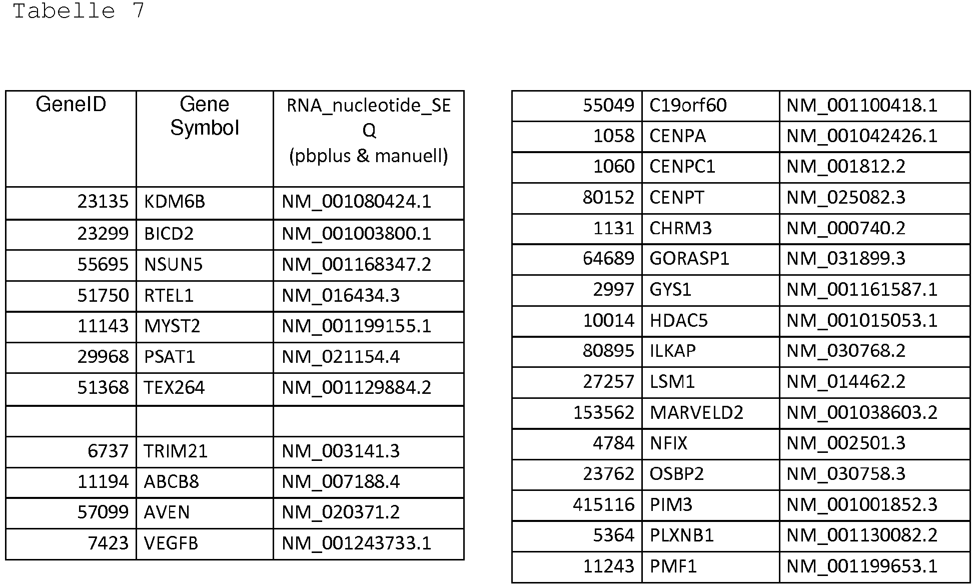

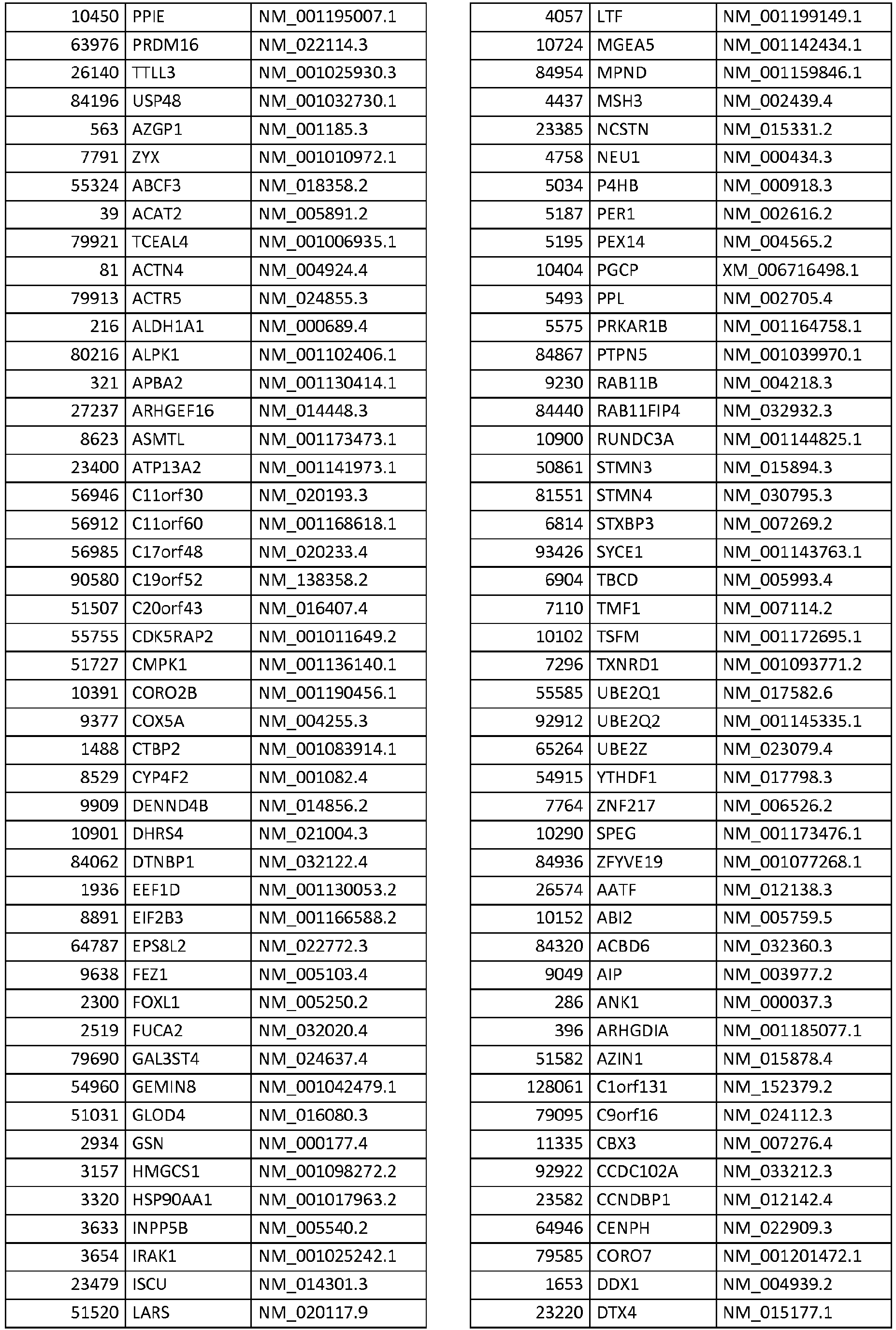

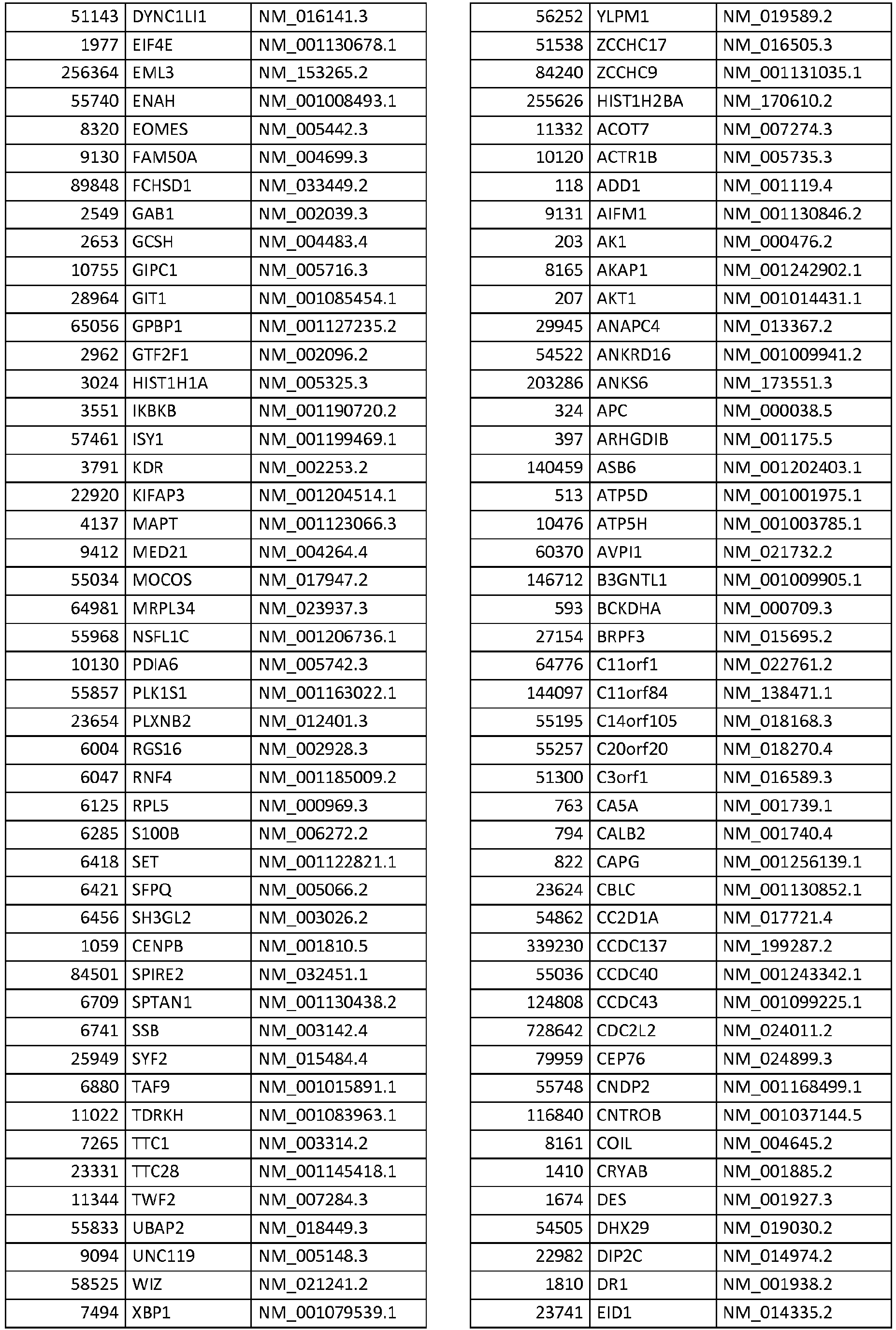

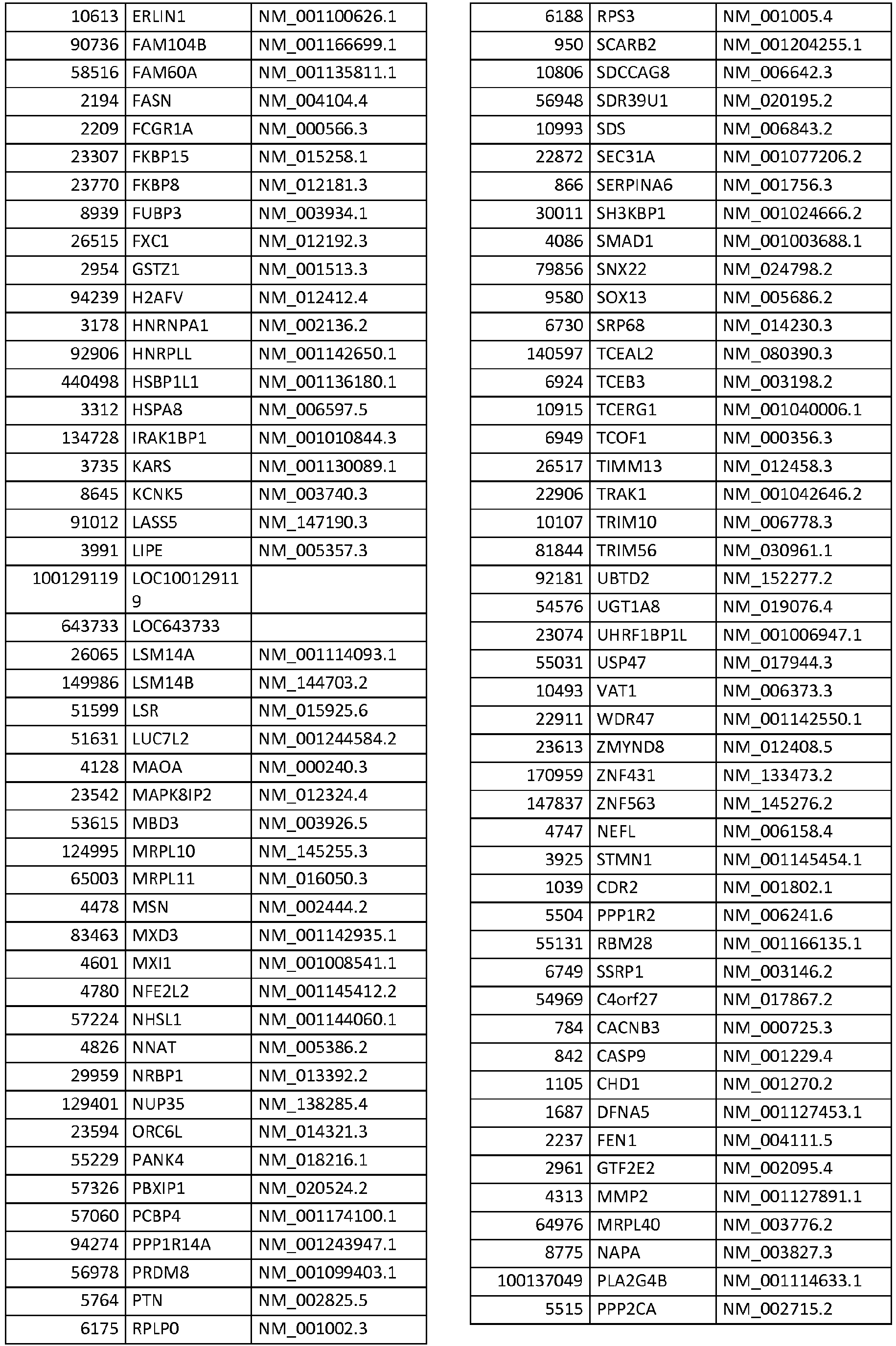

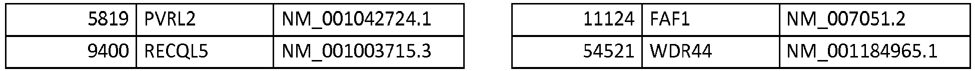

### Beispiel 11: Validierung von SSc-assozierten Autoantikörpern in einem unabhängigen Probenkollektiv

Die in Tabelle 2 genannten Marker wurden in einem zweiten unabhängigen Probenkollektiv getestet (SSc Kohorte II). Insgesamt wurden 180 Serumproben von SSc Patienten analysiert, deren demogrophischen und klinischen Daten der EUSTAR Datenbank entnommen worden sind. EUSTAR ist eine multizentrische, prospektive Kohorte der European League Against Rheumatism (EULAR) Scleroderma Trials and Research (EUSTAR) group. Als Kontrollproben wurden n=99 Serumproben von Gesundkontrollen und n=110 Serumproben von Patienten mit rheumatischen Autoimmunerkrankungen (AD) eingesetzt. Diese setzten sich zusammen aus Serumproben von Patienten mit der Diagnose SLE, Myositis, Arthritis und Sjögrens's Syndrom.

**Tabelle 6: Demographische, klinische und serologische Daten der SSc Kohorte II.**

| Anzahl (%); Mittelwert (%) | Gesamt SSc | Hautbeteiligung | |
|---|---|---|---|
| | SSc (n=180) | dSSc(n=57) | lSSc (n=83) |
| Weiblich n (%) | 142 (79.9) | 42 (737) | 67 (80.7) |
| Männlich n (%) | 38 (21.1) | 15 (26.3) | 16 (19.3) |
| Alter Mittelwert (SD) | 56.5 (13.8) | 52.9 (12.2) | 58.2 (15.1) |
| Mittlere Erkrankungsdauer in Jahren (SD) | 8.2 (9.7) | 6.98 (6.8) | 9.9 (11.9) |
| ANA positive n (%) | 176 (97.8) | 55 (96.5) | 82 (98.8) |
| anti-CENPB positive n (%) | 66 (36.7) | 5 (8.8) | 34 (41) |
| anti-Scl70 positive n (%) | 50 (27.8) | 21 (36.8) | 21 (25.3) |

Hierzu wurden die in Tabelle 2 genannten humanen Proteine an Luminex Beads gekoppelt und die Protein-gekoppelten Beads in einem Multiplex Assay mit den Patientenproben vermessen. Die Bindung von Autoantikörpern wurde mittels eines PEkonjugierten Autoantikörpers in einem Luminex Instrument gemessen.

Es wurde eine univariate statistische Auswertugen durchgeführt mit dem Wilcoxon-Rangsummentest. Das vorgegebene Signifikanzniveau wurde bei 0.05 angelegt. Tabelle 7 enthält 41 Antigene, die sowohl in der SSc Kohorte I, die zur Entdeckung der Marker verwendet wurde (Bespiel 6), als auch in der SSc Kohorte II einen p-Wert kleiner als das festgelegte Signifikanzviveau 0.05 erreichten. Für den Vergleich aller SSc Proben gegen Gesundkontrollen (HC) erreichten 31 Marker einen p-Wert kleiner als 0.05. Für den Vergleich aller SSc Proben gegen eine zusammengefasste Gruppe aus Serumproben verschiedener rheumatoider Erkrankungen (AD) erreichten 24 Marker einen p-Wert kleiner als 0.05.

**Tabelle 7: Zusammenfassung der p-Werte (Wilcoxon-Rangsummentest)für 41 Marker für die Diagnose von SSc in der SSc Kohorte II**

| | | **SSc vs HC** | | | | **SSc vs AD** |
|---|---|---|---|---|---|---|
| **Mar ker Nr** | **Gene Symbol** | **SSc** | **Scl70 & CENPB neg** | **dSSc** | **lSSc** | **SSc** |
| **1** | KDM6B | 3.09E-10 | 1.33E-03 | 6.13E-05 | 2.02E-08 | 2.25E-02 |
| **2** | BICD2 | 4.19E-03 | 6.48E-01 | 7.77E-01 | 5.11E-04 | 7.37E-01 |
| **3** | NSUN5 | 1.62E-05 | 4.06E-01 | 9.41E-01 | 4.30E-06 | 5.04E-03 |
| **4** | RTEL1 | 1.28E-03 | 3.69E-01 | 4.16E-01 | 4.32E-04 | 7.74E-02 |
| **8** | TRIM21 | 5.30E-09 | 1.51E-05 | 1.65E-06 | 5.90E-07 | 3.88E-01 |
| **9** | ABCB8 | 1.81E-03 | 8.10E-01 | 3.37E-01 | 1.13E-02 | 1.02E-02 |
| **10** | AVEN | 2.06E-03 | 7.33E-01 | 4.37E-01 | 1.83E-03 | 7.88E-02 |
| **13** | CENPA | 2.31E-04 | 3.02E-01 | 3.03E-01 | 8.03E-05 | 1.92E-03 |
| **14** | CENPC | 4.17E-11 | 5.64E-03 | 2.25E-02 | 5.14E-10 | 5.01E-07 |
| **15** | CENPT | 7.50E-02 | 4.61E-01 | 8.25E-01 | 1.05E-02 | 1.98E-02 |
| **18** | GYS1 | 1.99E-02 | 2.91E-02 | 3.40E-01 | 2.07E-02 | 8.24E-01 |
| **22** | MARVELD2 | 3.77E-02 | 1.84E-01 | 1.26E-01 | 1.71E-01 | 1.78E-02 |
| **27** | PMF1 | 4.96E-02 | 9.77E-01 | 3.39E-01 | 3.28E-01 | 3.60E-02 |
| **30** | TTLL3 | 2.01E-02 | 1.30E-01 | 9.82E-01 | 2.04E-02 | 9.67E-01 |
| **40** | ALPK1 | 1.07E-02 | 1.90E-03 | 5.15E-02 | 5.65E-02 | 7.13E-01 |
| **44** | ATP13A2 | 1.83E-02 | 7.52E-02 | 6.30E-01 | 1.61E-02 | 3.62E-01 |
| **48** | C19orf52 | 8.67E-03 | 1.70E-01 | 7.77E-01 | 3.51E-02 | 5.48E-03 |
| **75** | LTF | 7.97E-03 | 4.52E-01 | 7.16E-01 | 3.10E-03 | 9.21E-01 |
| **92** | STMN4 | 5.50E-05 | 6.47E-04 | 3.64E-03 | 7.59E-04 | 3.06E-02 |
| **108** | ACBD6 | 4.17E-03 | 3.71E-01 | 2.99E-01 | 6.41E-03 | 4.02E-02 |
| **115** | CBX3 | 2.66E-03 | 5.41E-01 | 2.33E-01 | 3.48E-02 | 4.45E-03 |
| **118** | CENPH | 2.27E-01 | 1.78E-01 | 5.44E-01 | 4.22E-02 | 2.19E-02 |
| **123** | EIF4E | 2.78E-02 | 9.74E-01 | 6.72E-01 | 1.52E-02 | 2.54E-04 |
| **134** | GTF2F1 | 1.07E-04 | 4.08E-02 | 3.28E-02 | 6.88E-05 | 2.30E-01 |
| **137** | ISY1 | 2.19E-02 | 2.06E-02 | 4.30E-02 | 1.35E-01 | 3.65E-01 |
| **138** | KDR | 3.68E-02 | 1.77E-01 | 4.65E-02 | 4.22E-01 | 3.39E-04 |
| **140** | MAPT | 2.50E-04 | 5.34E-01 | 2.76E-01 | 2.63E-05 | 7.82E-01 |
| **155** | CENPB | 4.27E-12 | 7.99E-01 | 8.41E-03 | 6.90E-11 | 2.91E-09 |
| **158** | SSB | 9.04E-02 | 1.56E-01 | 6.06E-01 | 3.07E-02 | 2.61E-01 |
| **213** | DIP2C | 2.44E-02 | 5.27E-02 | 5.08E-01 | 8.26E-03 | 1.87E-01 |
| **224** | TIMM10B | 4.05E-02 | 5.16E-03 | 3.86E-02 | 2.42E-01 | 3.51E-01 |
| **227** | HNRNPA1 | 1.48E-02 | 2.54E-02 | 1.67E-03 | 1.28E-01 | 7.77E-01 |
| **229** | HSBP1L1 | 2.45E-02 | 1.03E-01 | 1.91E-02 | 2.88E-01 | 1.05E-01 |
| **232** | KARS | 1.25E-02 | 5.71E-03 | 2.85E-02 | 1.12E-01 | 8.70E-03 |
| **257** | PBXIP1 | 6.43E-06 | 2.08E-01 | 2.49E-01 | 8.61E-05 | 3.83E-01 |
| **290** | ZNF431 | 3.94E-02 | 3.68E-02 | 1.59E-01 | 9.45E-02 | 4.67E-02 |
| **292** | NEFL | 6.97E-03 | 6.01E-02 | 9.19E-02 | 3.95E-02 | 8.50E-01 |
| **293** | STMN1 | 6.11E-05 | 6.13E-04 | 3.69E-03 | 8.75E-04 | 4.19E-03 |
| **295** | PPP1R2 | 1.19E-02 | 7.79E-01 | 3.69E-01 | 6.39E-02 | 1.98E-01 |
| **297** | SSRP1 | 4.79E-02 | 7.17E-01 | 7.91E-01 | 2.02E-02 | 4.19E-03 |
| **314** | TOP1 | 6.19E-07 | 4.23E-01 | 6.94E-05 | 4.10E-05 | 4.96E-07 |

### Beispiel 12: Anwendung von Autoantikörper-Panels zur Diagnose von SSc

Aus den in Tabelle 2 und Tabelle 7 enthaltenen Markern wurden verschiedene Markerkombinationen mittels logistischer Regressionsmodelle getestet. Dabei wurde als Vorgabe die Spezifität gegenüber der Kontrollgruppe auf mindestens 95% gesetzt. Eine Übersicht über die in den Panels 1,3-8 verwendeten Antigene ist in Tabelle 8 enthalten. Zusätzlich wurde ein diagnostisches Panel 9 unter Verwendung aller Marker aus Tabelle 2 gerechnet. Die Panels 1,3,4,5 und 9 eignen sich bevorzugt zur Diagnose aller SSc Patienten, unabhängig von der jeweiligen Subform. Die AUC (Area under the Curve) der Panels 1,3,4,6 und 9 ist in Tabelle 8 für 2 unabhängige SSc Probenkollektive (Kohorte I und Kohorte II) angegeben.

Panel 1 besteht aus den üblicherweise diagnostisch verwendeten Antigenen Scl70(Top1) und CENPB und erreicht eine AUC von 0.81 in Kohorte I und 0.85 in Kohorte II für den Vergleich SSc versus Gesund (HC).

Panel 3 besteht aus aus zwei diagnostischen Antigenen, CENPB und Scl70, und zwei neuen SSc Antigenen, KDM6B und BICD2. Gegenüber dem üblicherweise verwendeten Markern des Panels 1 zeigt ein logistisches Model für das Panel 3, eine deutlich höhere AUC für den Vergleich SSc versus Gesund (HC): für Kohorte I 0.88 gegenüber 0.81 und für Kohorte II 0.87 gegenüber 0.85.

Panel 4 basiert auf Panel 3 und enthält zwei weitere neue Marker, NSUN5 und PPP1R2. Ein logistisches Regressionsmodel für das Panel 4 erreicht für die Diagnose von SSc im Vergleich zu Gesundproben für die Kohorte I eine AUC von 0.9 und für die Kohorte II eine AUC von 0.85.

Panel 5 basiert auf Panel 4 und enthält neben den diagnostischen Markern CENPB und Scl70, die Marker KDM6B, BICD2, NSUN5, PPP1R2, RTEL1, TRIM21, ABCB8, AVEN, CENPA,CENPC1, GYS1, MARVELD2, PMF1, TTLL3, ATP13A2, LTF, STMN4, ACBD6, GTF2F1, KDR, DIP2C, FXC1, HNRNPA1, HSBP1L1, KARS, PBXIP1, ZNF431, STMN1 und SSRP1.

**Tabelle 8: Zusammensetzung der Marker-Panels**

| | | **Diagnose SSc** | | | | **CENPB & Scl70 neg.** | **dSSc** | **lSSc** |
|---|---|---|---|---|---|---|---|---|
| **#** | **Gene Symbol** | **P1** | **P3** | **P4** | **P5** | **P6** | **P7** | **P8** |
| 155 | CENPB | x | x | x | x | | | x |
| 314 | TOP1 | x | x | x | x | | x | x |
| 1 | KDM6B | | x | x | x | x | x | x |
| 2 | BICD2 | | x | x | x | | | x |
| 3 | NSUN5 | | | x | x | | | x |
| 4 | RTEL1 | | | | x | | | x |
| 8 | TRIM21 | | | | x | x | x | x |
| 9 | ABCB8 | | | | x | | | x |
| 10 | AVEN | | | | x | | | x |
| 13 | CENPA | | | | x | | | x |
| 14 | CENPC1 | | | | x | | x | x |
| 15 | CENPT | | | | | | | x |
| 18 | GYS1 | | | | x | x | | x |
| 22 | MARVELD2 | | | | x | | | |
| 27 | PMF1 | | | | x | | | |
| 30 | TTLL3 | | | | x | | | |
| 40 | ALPK1 | | | | | x | | |
| 44 | ATP13A2 | | | | x | | | |
| 48 | C19orf52 | | | | | | | x |
| 75 | LTF | | | | x | | | |
| 92 | STMN4 | | | | x | x | | x |
| 108 | ACBD6 | | | | x | | | x |
| 115 | CBX3 | | | | | | | x |
| 118 | CENPH | | | | | | | x |
| 123 | EIF4E | | | | | | | x |
| 134 | GTF2F1 | | | | x | | | x |
| 137 | ISY1 | | | | | | x | |
| 138 | KDR | | | | x | | | |
| 140 | MAPT | | | | | | | x |
| 158 | SSB | | | | | | | x |
| 213 | DIP2C | | | | x | | | x |
| 224 | FXC1 | | | | x | | | |
| 227 | HNRNPA1 | | | | x | x | x | |
| 229 | HSBP1L1 | | | | x | | | |
| 232 | KARS | | | | x | | | |
| 257 | PBXIP1 | | | | x | | | x |
| 290 | ZNF431 | | | | x | | | |
| 292 | NEFL | | | | | | | x |
| 293 | STMN1 | | | | x | x | | x |
| 295 | PPP1R2 | | | x | x | | | |
| 297 | SSRP1 | | | | x | | | x |

Beispiel 13: Anwendung von Autoantikörper-Panels zur Identifizierung von Patienten mit fehlendem Nachweis von anti-Zentromer und anti-Scl70 (Top1)- Autoantikörpern.

Wie in Tabelle 6 dargelegt, testeten nur etwa 41% der SSc Patienten aus der SSc Kohorte II positiv für anti-CENPB Antikörper und 25.3% positiv für anti-Scl70 Antikörper. Da in der Literatur sehr wenige Patienten mit doppelt-positiver Reaktivität beschrieben worden sind, wird der Anteil an anti-CENPB und anti-Scl70 negativen Patienten mit etwa 30% angegeben. Um diese diagnostische Lücke zu schließen, wurde ein logistisches Regressionsmodel für Panels 6, das aus 7 Markern besteht erstellt.

Panel 6 enthält die Marker KDM6B, TRIM21, GYS1, ALPK1, STMN4, HNRNPA1, STMN1.

Wie in Tabelle 6 zusammengefasst wurde mit den Markern des Panels 6 in der Kohorte I eine AUC von 0.91 und für Kohorte II eine AUC von 0.84 erreicht.

Beispiel 14: Anwendung von Autoantikörper-Panels zur Identifizierung von SSc Patienten mit diffuser Form Wie in Tabelle 6 dargelegt wurden 36.8% der SSc Patienten mit diffuser SSc (dSSc) und 25.3% der Patienten mit limitierter Form positiv für Autoantikörper gegen Scl70 (Top1) getestet. Dies zeigt, dass anti-Scl70 Antikörper zwar mit der diffusen Form assoziiert sind, aber nicht spezifisch sind. Zur Diagnose der diffusen Form werden daher zusätzliche Marker benötigt. Panel 7 umfasst 6 Marker, die besonders zur Diagnose der diffusen SSc geeignet sind.

Panel 7 enthält die Marker KDM6B, TOP1, TRIM21, CENPC1, ISY1, HNRNPA1.

Für Panel 7 wurde mittels einer logistischen Regression eine AUC von 0.81 für Kohorte I und 0.87 für Kohorte II berechnet.

Beispiel 15: Anwendung von Autoantikörper-Panels zur Identifizierung von SSc Patienten mit limitierter Form Wie in Tabelle 6 dargelegt wurden 41% der SSc Patienten mit limitierter SSc(lSSc) und 8.8% der Patienten mit diffuser Form positiv für Autoantikörper gegen CENPB getestet. Dies zeigt, dass anti-CENPB Antikörper zwar mit der limitierten Form assoziiert sind, aber nicht spezifisch sind. Zur Diagnose der limitierten Form werden daher zusätzliche Marker benötigt. Panel 8 umfasst 27 Marker, die besonders zur Diagnose der limitierten SSc geeignet sind.

Panel 8 umfasst die Marker KDM6B, BICD2, NSUN5, CENPB, TOP1, RTEL1, TRIM21, ABCB8, AVEN, CENPA, CENPC1, CENPT, GYS1, C19orf52, STMN4, ACBD6, CBX3, CENPH, EIF4E, GTF2F1, MAPT, SPTAN1, DIP2C, PBXIP1, NEFL, STMN1, SSRP1.

Für Panel 8 wurde mittels einer logistischen Regression eine AUC von 0.97 für Kohorte I und 0.99 für Kohorte II berechnet.

**Tabelle 9: AUC der SSc Panels in der SSc Kohorte I und Kohorte II**

| **Panel** | **SSc Kohorte** | **Vergleich** | **AUC** |
|---|---|---|---|
| **Panel 1** | Kohorte I | SSc vs HC | 0.81 |
| **Panel 1** | Kohorte I | SSc vs SLE | 0.84 |
| **Panel 3** | Kohorte I | SSc vs HC | 0.88 |
| **Panel 3** | Kohorte I | SSc vs SLE | 0.84 |
| **Panel 4** | Kohorte I | SSc vs HC | 0.90 |
| **Panel 4** | Kohorte I | SSc vs SLE | 0.84 |
| **Panel 5** | Kohorte I | SSc vs HC | 0.93 |
| **Panel 6** | Kohorte I | SSc CENPB & Scl70 neg vs HC | 0.91 |
| **Panel 7** | Kohorte I | dSSc vs HC | 0.81 |
| **Panel 8** | Kohorte I | lSSc vs HC | 0.97 |
| **Panel 9** | Kohorte I | SSc vs HC | 0.99 |
| **Panel 1** | Kohorte II | SSc vs HC | 0.85 |
| **Panel 1** | Kohorte II | SSc vs AI | 0.84 |
| **Panel 3** | Kohorte II | SSc vs HC | 0.87 |
| **Panel 3** | Kohorte II | SSc vs AI | 0.85 |
| **Panel 4** | Kohorte II | SSc vs HC | 0.88 |
| **Panel 4** | Kohorte II | SSc vs AI | 0.85 |
| **Panel 5** | Kohorte II | SSc vs HC | 0.96 |
| **Panel 6** | Kohorte II | SSc CENPB & Scl70 neg vs HC | 0.84 |
| **Panel 7** | Kohorte II | dSSc vs HC | 0.87 |
| **Panel 8** | Kohorte II | lSSc vs HC | 0.99 |
| **Panel 9** | Kohorte II | SSc vs HC | 0.99 |

### Beispiel 16: ELISA für die Bestimmung von anti-KDM6B Antikörpern in der Systemsklerose

Entsprechend der Erfindung wurde rekombinant hergestelltes und verbessertes KDM6B Protein (Seq ID 315), welches die Aminosäuren 42-421 de Uniprot Datenbank Eintrags 015054 umfasst. KDM6B wird über mehrere Stufen mittels Nickelchelat-Affinitäts Chromatographie, Größenausschlusschromatographie und Ionentauscher Chromatographie aufgereinigt. Anschließend wurde das aufgereinigte KDM6B in einer Konzentration von 1 µg pro Milliliter in einem Na-Karbonatpuffer pH 9.0 auf eine Flachboden ELISA Platte (beispielsweise NUNC) mit 100µl pro Kavität aufgebracht und 4h bei Raumtemperatur (RT) inkubiert.

Nach der Inkubation wurde der überschüssige Puffer entfernt und die freien Bindungsstellen der ELISA Platte wurden mit einem Inert-Protein (Kalt-Wasserfisch-Gelantine) in PBS für 1h bei Raumtemperatur blockiert.

Serum oder Plasmaproben wurden 1:101 in HBS-T Puffer verdünnt in die Kavitäten aufgebracht und 30 min bei Raumtemperatur inkubiert. Während dieser Zeit binden die KD6MB spezifischen IgG Autoantikörper aus der Serum oder Plasma Probe an das gebundene Protein.

Unspezifisch-gebundene Antikörper werden nach der Inkubation durch zweimaliges Waschen mit einem PBS Tween Puffer entfernt. Es folgt eine halbstündige Inkubation mit einem HRP (Horse-Radish-Peroxidase-konjugierten) anti-human IgG Detektionsantikörper. Wiederum werden nach der Inkubation unspezifische Bindungspartner durch zweimaliges waschen mit PBS-Tween-Puffer entfernt.

Die kolorimetrische Nachweis Reaktion erfolgt durch Zugabe eines TMB (Tetra Methylbenzidin) Substrats. Diese Reaktion wird nach 15 Minuten durch Zugabe eine 1M Schwefelsäure gestoppt, und in einem ELISA Messgerät (TECAN) bei einer Wellenlänge von 450nm vs 620nm ausgewertet. Die farbliche Intensität(optische Dichte, OD) der Nachweis Reaktion ist direkt proportional zur Konzentration der KDM6B-spezifischen IgG Autoantikörper in der Serum oder Plasma Probe.

Die Auswertung des Tests erfolgt semi-quantitativ über eine ein-Punkt Kalibration.

Die OD-Werte des anti-KDM6B ELISAs wurden zur Berechnung der p-Werte mittels Wilcoxon-Rangsummentest und der Berechnung eines logistischen Regressionsmodells mit ROC-Analyse für den Vergleich SSc gegen HC herangezogen. Da die Gruppe der SSc-Patienten größer als die der Kontrollgruppe HC ist, wird für die logistische Regression ein Random Under-Sampling durchgeführt, sodass die SSc Kohorte II gleich große Gruppen hat. Die ROC-Analyse wurde berechnet, indem aus diesem verkleinerten Datensatz der SSc Kohorte II 75% zum Trainieren und 25% zum Testen des Modells verwendet werden. Dieser Ansatz wird 100 Mal wiederholt.

Tabelle 10 zeigt die p-Werte für den Vergleich SSc versus Gesundkontrollen und SSc versus Autoimmunkontrollen, welcher aus Proben der SSc Kohorte II berechnet wurde.

Tabelle 11 zeigt die empirisch kalkulierte Fläche unter der Kurve (AUC), Sensitivität und Spezfität des anti-KDM6B ELISAs für die Diagnose SSc (SSc Kohorte II) im Vergleich zu Gesundproben.

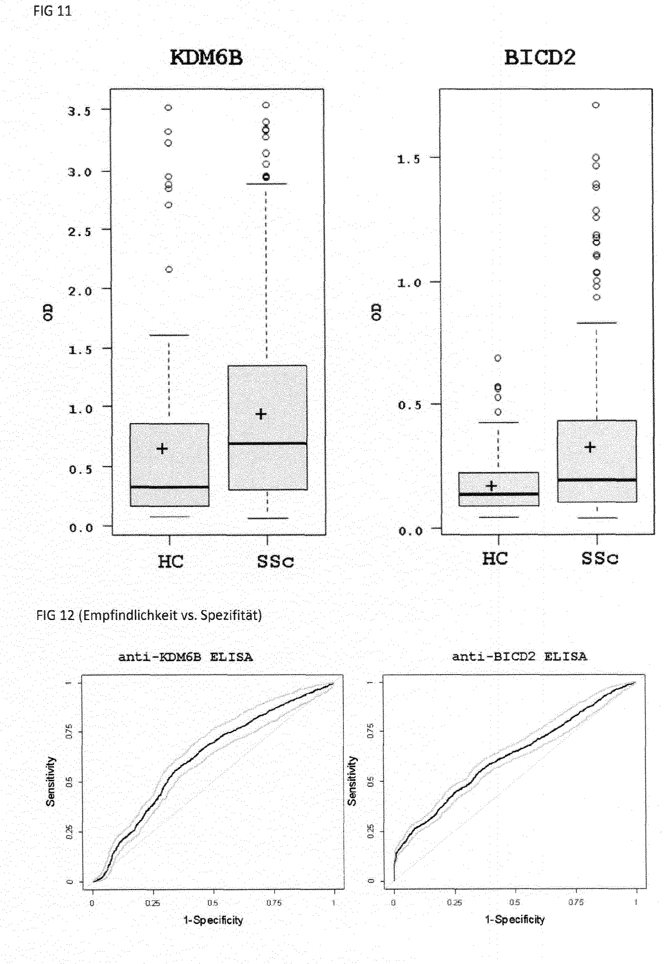

Figur 12 zeigt ROC Kurve für ein logistisches Regressionsmodel basierend auf den ELISA OD Werten von KDM6B für die Diagnose von SSc im Vergleich zu Gesundkontrollen.

### Beispiel 17: ELISA für die Bestimmung von anti-BICD2 Antikörpern in der Systemsklerose

Entsprechend der Erfindung wurde rekombinant hergestelltes BICD2 Protein über mehrere Stufen mittels Nickelchelat-Affinitäts Chromatographie, Größenausschlusschromatographie und Ionentauscher Chromatographie aufgereinigt. Anschließend wurde das aufgereinigte BICD2 in einer Konzentration von 1,7 µg pro Milliliter in einem Na-Karbonatpuffer pH 9.0 auf eine Flachboden ELISA Platte (beispielsweise NUNC) mit 100µl pro Kavität aufgebracht und 4h bei RT inkubiert.

Nach der Inkubation wurde der überschüssige Puffer entfernt und die freien Bindungsstellen der ELISA Platte werden mit einem Inert-Protein (Kalt-Wasserfisch-Gelantine) in PBS für 1h bei Raumtemperatur blockiert.

Serum oder Plasmaproben wurden 1:101 in HBS-T Puffer verdünnt in die Kavitäten aufgebracht und 30 min bei Raumtemperatur inkubiert. Während dieser Zeit binden die BICD2-spezifischen IgG Autoantikörper aus der Serum oder Plasma Probe an das gebundene Protein.

Unspezifisch-gebundene Antikörper werden nach der Inkubation durch zweimaliges Waschen mit einem PBS Tween Puffer entfernt. Es folgt eine halbstündige Inkubation mit einem HRP (Horse-Radish-Peroxidase-konjugierten anti-human IgG Detektionsantikörper. Wiederum werden nach der Inkubation unspezifische Bindungspartner durch zweimaliges waschen mit PBS-Tween-Puffer entfernt.

Die kolorimetrische Nachweis Reaktion erfolgt durch Zugabe eines TMB (Tetra Methylbenzidin) Substrats. Diese Reaktion wird nach 15 Minuten durch Zugabe eine 1M Schwefelsäure gestoppt, und in einem ELISA Messgerät (TECAN) bei einer Wellenlänge von 450nm vs 620nm ausgewertet. Die farbliche Intensität der Nachweis Reaktion ist direkt proportional zur Konzentration der BICD2-spezifischen IgG Autoantikörper in der Serum oder Plasma Probe.

Die OD-Werte des Antigens BICD2 wurde zur Berechnung der p-Werte mittels Wilcoxon-Rangsummentest und der Berechnung von die Receiver Operator Kurven (ROC) herangezogen.

Die OD-Werte des anti-BICD2 ELISAs wurden zur Berechnung der p-Werte mittels Wilcoxon-Rangsummentest und der Berechnung eines logistischen Regressionsmodells mit ROC-Analyse für den Vergleich SSc gegen HC herangezogen. Da die Gruppe der SSc-Patienten größer als die der Kontrollgruppe HC ist, wird für die logistische Regression ein Random Under-Sampling durchgeführt, sodass die SSc Kohorte II gleich große Gruppen hat. Die ROC-Analyse wurde berechnet, indem aus diesem verkleinerten Datensatz der SSc Kohorte II 75% zum Trainieren und 25% zum Testen des Modells verwendet werden. Dieser Ansatz wird 100 Mal wiederholt.

Tabelle 10 zeigt die p-Werte für den Vergleich SSc versus Gesundkontrollen (HC) und SSc versus Autoimmunkontrollen (AD), welcher aus Proben der SSc Kohorte II berechnet wurde.

**Tabelle 10: Wilcoxon-Rangsummentest (p-Wert) für den anti-KDM6B und anti-BICD2 ELISA für die Diagnose von SSc im Vergleich zu Gesundkontrollen.**

| **Vergleich** | **KDM6B** | **BICD2** |
|---|---|---|
| **SSc vs HC** | 1.05E-04 | 1.13E-04 |

Figur 11 zeigt den Boxplot des BICD2 ELISAs für SSc Proben im Vergleich zu Gesundproben.

Tabelle 11 zeigt die empirisch kalkulierte Fläche unter der Kurve (AUC), Sensitivität und Spezfität für den anti-BICD2 ELISA.Die Berechnung wurde für Proben der SSc Kohorte II und Gesundproben durchgeführt.

**Tabelle 11: Empirisch kalkulierte, Sensitivität und Spezifität des anti-KDM6B und anti-BICD2 ELISAs für die SSc Kohorte II für die Diagnose SSc im Vergleich zu Gesundkontrollen**

| **ELISA** | **Test** | **Wert** |
|---|---|---|
| **anti-KDM6B** | AUC | 0.63 |
| | Sensitivität | 0.19 |
| | Spezifität | 0.90 |
| **anti-BICD2** | AUC | 0.64 |
| | Sensitivität | 0.19 |
| | Spezifität | 0.95 |

Figur 12 zeigt ROC Kurve mit Konfidenzbändern für ein logistisches Regressionsmodel basierend auf den ELISA OD Werten von BICD2 für die Diagnose von SSc im Vergleich zu Gesundkontrollen.

Literatur:
Mehra S, Walker J, Patterson K, Fritzler MJ (2013). Autoantibodies in systemic sclerosis. Autoimmun Rev. 12 (3) : 340-54.
Mierau R, Moinzadeh P, Riemekasten G, Melchers I, Meurer M, Reichenberger F,Buslau M, Worm M, Blank N, Hein R, Müller-Ladner U, Kuhn A, Sunderkötter C, Juche A, Pfeiffer C, Fiehn C, Sticherling M, Lehmann P, Stadler R, Schulze-Lohoff E,Seitz C, Foeldvari I, Krieg T, Genth E, Hunzelmann N (2011). Frequency of disease-associated and other nuclear autoantibodies in patients of the German Network for Systemic Scleroderma: correlation with characteristic clinical features. Arthritis Res Ther. 13(5):R172
LeRoy EC, Black C, Fleischmajer R, Jablonska S, Krieg T, Medsger TA Jr, Rowell N, Wollheim F (1988). Scleroderma (systemic sclerosis): classification, subsets and pathogenesis. J Rheumatol. 15(2):202-5.
Watts R., (2006). Autoantibodies in the autoimmune rheumatic diseases, Medicine, 34 (11): 441-444

## Patentansprüche

1. Verwendung von den Markern CENPB (SEQ ID No. 793 oder kodiert von SEQ ID NO: 155 oder 474), TOP1 (SEQ ID No. 952 oder kodiert von SEQ ID NO: 314 oder 633), KDM6B (SEQ ID No. 639 oder kodiert von SEQ ID NO: 1 oder 320,) und BICD2 (SEQ ID No. 640 oder kodiert von SEQ ID NO: 2 oder 321) bei der in vitro/ex vivo diagnose von Systemische Sklerose (SSc), wobei CENPB, TOP1, KDM6B und BICD2 verwendet werden um eine Wechselwirkung mit Antikörpern aus einer Körperflüssigkeit oder eines Gewebeauszug eines Patienten mit SSc aufzuweisen.

2. Verwendung nach Anspruch 1 wobei zusätzlich NSUN5 (SEQ ID No. 641 oder kodiert von SEQ ID NO: 3 oder 322) und / oder PPP1R2 (SEQ ID No. 933 oder kodiert von SEQ ID NO: 295 oder 614) verwended werden um eine Wechselwirkung mit Antikörpern aus einer Körperflüssigkeit oder eines Gewebeauszug eines Patienten mit SSc aufzuweisen.

3. Verwendung nach Anspruch 1 wobei zusätzlich NSUN5 (SEQ ID No. 641 oder kodiert von SEQ ID NO: 3 oder 322) und PPP1R2 (SEQ ID No. 933 oder kodiert von SEQ ID NO: 295 oder 614) verwended werden um eine Wechselwirkung mit Antikörpern aus einer Körperflüssigkeit oder eines Gewebeauszug eines Patienten mit SSc aufzuweisen.

4. Verwendung nach einem der Ansprüche 1 bis 3 wobei zusätzlich mindestens ein Marker ausgewählt aus der Gruppe RTEL1 (SEQ ID No. 642 oder kodiert von SEQ ID NO: 4 oder 323), MYST2 (SEQ ID No. 643 oder kodiert von SEQ ID NO: 5 oder 324), PSAT1 (SEQ ID No. 644 oder kodiert von SEQ ID NO: 6 oder 325), TEX264 (SEQ ID No. 645 oder kodiert von SEQ ID NO: 7 oder 326), TRIM21 (SEQ ID No. 646 oder kodiert von SEQ ID NO: 8 oder 327), ABCB8 (SEQ ID No. 647 oder kodiert von SEQ ID NO: 9 oder 328), AVEN (SEQ ID No. 648 oder kodiert von SEQ ID NO: 10 oder 329), CENPA (SEQ ID No. 651 oder kodiert von SEQ ID NO: 13 oder 332), CENPC1 (SEQ ID No. 652 oder kodiert von SEQ ID NO: 14 oder 333), GYS1 (SEQ ID No. 656 oder kodiert von SEQ ID NO: 18 oder 337), MARVELD2 (SEQ ID No. 660 oder kodiert von SEQ ID NO: 22 oder 341), PMF1 (SEQ ID No. 665 oder kodiert von SEQ ID NO: 27 oder 346), TTLL3 (SEQ ID No. 668 oder kodiert von SEQ ID NO: 30 oder 349), ATP13A2 (SEQ ID No. 682 oder kodiert von SEQ ID NO: 44 oder 363), LTF (SEQ ID No. 713 oder kodiert von SEQ ID NO: 75 oder 394), STMN4 (SEQ ID No. 730 oder kodiert von SEQ ID NO: 92 oder 411), ACBD6 (SEQ ID No. 746 oder kodiert von SEQ ID NO: 108 oder 427), GTF2F1 (SEQ ID No. 772 oder kodiert von SEQ ID NO: 134 oder 453), KDR (SEQ ID No. 776 oder kodiert von SEQ ID NO: 138 oder 457), DIP2C (SEQ ID No. 851 oder kodiert von SEQ ID NO: 213 oder 532), FXC1 (SEQ ID No. 862 oder kodiert von SEQ ID NO: 224 oder 543), HNRNPA1 (SEQ ID No. 865 oder kodiert von SEQ ID NO: 227 oder 546), HSBP1L1 (SEQ ID No. 867 oder kodiert von SEQ ID NO: 229 oder 548), KARS (SEQ ID No. 870 oder kodiert von SEQ ID NO: 232 oder 551), PBXIP1 (SEQ ID No. 895 oder kodiert von SEQ ID NO: 257 oder 576), ZNF431 (SEQ ID No. 928 oder kodiert von SEQ ID NO: 290 oder 609), STMN1 (SEQ ID No. 931 oder kodiert von SEQ ID NO: 293 oder 612), SSRP1 (SEQ ID No. 935 oder kodiert von SEQ ID NO: 297 oder 616) verwended wird um eine Wechselwirkung mit Antikörpern aus einer Körperflüssigkeit oder eines Gewebeauszug eines Patienten mit SSc aufzuweisen.

5. Verwendung nach Anspruch 1, wobei zusätzlich die Marker NSUN5 (SEQ ID No. 641 oder kodiert von SEQ ID NO: 3 oder 322) PPP1R2 (SEQ ID No. 933 oder kodiert von SEQ ID NO: 295 oder 614) RTEL1 (SEQ ID No. 642 oder kodiert von SEQ ID NO: 4 oder 323), TRIM21 (SEQ ID No. 646 oder kodiert von SEQ ID NO: 8 oder 327), ABCB8 (SEQ ID No. 647 oder kodiert von SEQ ID NO: 9 oder 328), AVEN (SEQ ID No. 648 oder kodiert von SEQ ID NO: 10 oder 329), CENPA (SEQ ID No. 651 oder kodiert von SEQ ID NO: 13 oder 332), CENPC1 (SEQ ID No. 652 oder kodiert von SEQ ID NO: 14 oder 333), GYS1 (SEQ ID No. 656 oder kodiert von SEQ ID NO: 18 oder 337), MARVELD2 (SEQ ID No. 660 oder kodiert von SEQ ID NO: 22 oder 341), PMF1 (SEQ ID No. 665 oder kodiert von SEQ ID NO: 27 oder 346), TTLL3 (SEQ ID No. 668 oder kodiert von SEQ ID NO: 30 oder 349), ATP13A2 (SEQ ID No. 682 oder kodiert von SEQ ID NO: 44 oder 363), LTF (SEQ ID No. 713 oder kodiert von SEQ ID NO: 75 oder 394), STMN4 (SEQ ID No. 730 oder kodiert von SEQ ID NO: 92 oder 411), ACBD6 (SEQ ID No. 746 oder kodiert von SEQ ID NO: 108 oder 427), GTF2F1 (SEQ ID No. 772 oder kodiert von SEQ ID NO: 134 oder 453), KDR (SEQ ID No. 776 oder kodiert von SEQ ID NO: 138 oder 457), DIP2C (SEQ ID No. 851 oder kodiert von SEQ ID NO: 213 oder 532), FXC1 (SEQ ID No. 862 oder kodiert von SEQ ID NO: 224 oder 543), HNRNPA1 (SEQ ID No. 865 oder kodiert von SEQ ID NO: 227 oder 546), HSBP1L1 (SEQ ID No. 867 oder kodiert von SEQ ID NO: 229 oder 548), KARS (SEQ ID No. 870 oder kodiert von SEQ ID NO: 232 oder 551), PBXIP1 (SEQ ID No. 895 oder kodiert von SEQ ID NO: 257 oder 576), ZNF431 (SEQ ID No. 928 oder kodiert von SEQ ID NO: 290 oder 609), STMN1 (SEQ ID No. 931 oder kodiert von SEQ ID NO: 293 oder 612), und SSRP1 (SEQ ID No. 935 oder kodiert von SEQ ID NO: 297 oder 616) verwended werden um eine Wechselwirkung mit Antikörpern aus einer Körperflüssigkeit oder eines Gewebeauszug eines Patienten mit SSc aufzuweisen.

6. Verwendung nach Anspruch 1, wobei zusätzlich die Marker NSUN5 (SEQ ID No. 641 oder kodiert von SEQ ID NO: 3 oder 322), RTEL1 (SEQ ID No. 642 oder kodiert von SEQ ID NO: 4 oder 323), TRIM21 (SEQ ID No. 646 oder kodiert von SEQ ID NO: 8 oder 327), ABCB8 (SEQ ID No. 647 oder kodiert von SEQ ID NO: 9 oder 328), AVEN (SEQ ID No. 648 oder kodiert von SEQ ID NO: 10 oder 329), CENPA (SEQ ID No. 651 oder kodiert von SEQ ID NO: 13 oder 332), CENPC1 (SEQ ID No. 652 oder kodiert von SEQ ID NO: 14 oder 333), CENPT (SEQ ID No. 653 oder kodiert von SEQ ID NO: 15 oder 334), GYS1 (SEQ ID No. 656 oder kodiert von SEQ ID NO: 18 oder 337), C19orf52 (SEQ ID No. 686 oder kodiert von SEQ ID NO: 48 oder 367), STMN4 (SEQ ID No. 730 oder kodiert von SEQ ID NO: 92 oder 411), ACBD6 (SEQ ID No. 746 oder kodiert von SEQ ID NO: 108 oder 427), CBX3 (SEQ ID No. 753 oder kodiert von SEQ ID NO: 115 oder 434), CENPH (SEQ ID No. 756 oder kodiert von SEQ ID NO: 118 oder 437), EIF4E (SEQ ID No. 761 oder kodiert von SEQ ID NO: 123 oder 442), GTF2F1 (SEQ ID No. 772 oder kodiert von SEQ ID NO: 134 oder 453), MAPT (SEQ ID No. 778 oder kodiert von SEQ ID NO: 140 oder 459), DIP2C (SEQ ID No. 851 oder kodiert von SEQ ID NO: 213 oder 532), PBXIP1 (SEQ ID No. 895 oder kodiert von SEQ ID NO: 257 oder 576), NEFL (SEQ ID No. 930 oder kodiert von SEQ ID NO: 292 oder 611), STMN1 (SEQ ID No. 931 oder kodiert von SEQ ID NO: 293 oder 612), und SSRP1 (SEQ ID No. 935 oder kodiert von SEQ ID NO: 297 oder 616) verwended werden um eine Wechselwirkung mit Antikörpern aus einer Körperflüssigkeit oder eines Gewebeauszug eines Patienten mit SSc aufzuweisen.

7. Ein fester Träger, auf dem die Marker CENPB (SEQ ID No. 793 oder kodiert von SEQ ID NO: 155 oder 474), TOP1 (SEQ ID No. 952 oder kodiert von SEQ ID NO: 314 oder 633), KDM6B (SEQ ID No. 639 oder kodiert von SEQ ID NO: 1 oder 320) und BICD2 (SEQ ID No. 640 oder kodiert von SEQ ID NO: 2 oder 321) fixiert sind.

## Claims

1. Use of the markers CENPB (SEQ ID No. 793 or encoded by SEQ ID NO: 155 or 474), TOP1 (SEQ ID No. 952 or encoded by SEQ ID NO: 314 or 633), KDM6B (SEQ ID No. 639 or encoded by SEQ ID NO: 1 or 320), and BICD2 (SEQ ID No. 640 or encoded by SEQ ID NO: 2 or 321) in the in vitro/ex vivo diagnosis of systemic sclerosis (SSc), wherein CENPB, TOP1, KDM6B and BICD2 are used to demonstrate an interaction with antibodies from a bodily fluid or a tissue sample from a patient with SSc.

2. Use according to claim 1, wherein, in addition, NSUN5 (SEQ ID No. 641 or encoded by SEQ ID NO: 3 or 322) and/or PPP1R2 (SEQ ID No. 933 or encoded by SEQ ID NO: 295 or 614) are used to demonstrate an interaction with antibodies from a bodily fluid or a tissue sample from a patient with SSc.

3. Use according to claim 1, wherein, in addition, NSUN5 (SEQ ID No. 641 or encoded by SEQ ID NO: 3 or 322) and PPP1R2 (SEQ ID No. 933 or encoded by SEQ ID NO: 295 or 614) are used to demonstrate an interaction with antibodies from a bodily fluid or a tissue sample from a patient with SSc.

4. Use according to one of claims 1 to 3, wherein, in addition, at least one marker selected from the group of RTEL1 (SEQ ID No. 642 or encoded by SEQ ID NO: 4 or 323), MYST2 (SEQ ID No. 643 or encoded by SEQ ID NO: 5 or 324), PSAT1 (SEQ ID No. 644 or encoded by SEQ ID NO: 6 or 325), TEX264 (SEQ ID No. 645 or encoded by SEQ ID NO: 7 or 326), TRIM21 (SEQ ID No. 646 or encoded by SEQ ID NO: 8 or 327), ABCB8 (SEQ ID No. 647 or encoded by SEQ ID NO: 9 or 328), AVEN (SEQ ID No. 648 or encoded by SEQ ID NO: 10 or 329), CENPA (SEQ ID No. 651 or encoded by SEQ ID NO: 13 or 332), CENPC1 (SEQ ID No. 652 or encoded by SEQ ID NO: 14 or 333), GYS1 (SEQ ID No. 656 or encoded by SEQ ID NO: 18 or 337), MARVELD2 (SEQ ID No. 660 or encoded by SEQ ID NO: 22 or 341), PMF1 (SEQ ID No. 665 or encoded by SEQ ID NO: 27 or 346), TTLL3 (SEQ ID No. 668 or encoded by SEQ ID NO: 30 or 349), ATP13A2 (SEQ ID No. 682 or encoded by SEQ ID NO: 44 or 363), LTF (SEQ ID No. 713 or encoded by SEQ ID NO: 75 or 394), STMN4 (SEQ ID No. 730 or encoded by SEQ ID NO: 92 or 411), ACBD6 (SEQ ID No. 746 or encoded by SEQ ID NO: 108 or 427), GTF2F1 (SEQ ID No. 772 or encoded by SEQ ID NO: 134 or 453), KDR (SEQ ID No. 776 or encoded by SEQ ID NO: 138 or 457), DIP2C (SEQ ID No. 851 or encoded by SEQ ID NO: 213 or 532), FXC1 (SEQ ID No. 862 or encoded by SEQ ID NO: 224 or 543), HNRNPA1 (SEQ ID No. 865 or encoded by SEQ ID NO: 227 or 546), HSBP1L1 (SEQ ID No. 867 or encoded by SEQ ID NO: 229 or 548), KARS (SEQ ID No. 870 or encoded by SEQ ID NO: 232 or 551), PBXIP1 (SEQ ID No. 895 or encoded by SEQ ID NO: 257 or 576), ZNF431 (SEQ ID No. 928 or encoded by SEQ ID NO: 290 or 609), STMN1 (SEQ ID No. 931 or encoded by SEQ ID NO: 293 or 612), SSRP1 (SEQ ID No. 935 or encoded by SEQ ID NO: 297 or 616) is used to demonstrate an interaction with antibodies from a bodily fluid or a tissue sample from a patient with SSc.

5. Use according to claim 1, wherein, in addition, the markers NSUN5 (SEQ ID No. 641 or encoded by SEQ ID NO: 3 or 322), PPP1R2 (SEQ ID No. 933 or encoded by SEQ ID NO: 295 or 614), RTEL1 (SEQ ID No. 642 or encoded by SEQ ID NO: 4 or 323), TRIM21 (SEQ ID No. 646 or encoded by SEQ ID NO: 8 or 327), ABCB8 (SEQ ID No. 647 or encoded by SEQ ID NO: 9 or 328), AVEN (SEQ ID No. 648 or encoded by SEQ ID NO: 10 or 329), CENPA (SEQ ID No. 651 or encoded by SEQ ID NO: 13 or 332), CENPC1 (SEQ ID No. 652 or encoded by SEQ ID NO: 14 or 333), GYS1 (SEQ ID No. 656 or encoded by SEQ ID NO: 18 or 337), MARVELD2 (SEQ ID No. 660 or encoded by SEQ ID NO: 22 or 341), PMF1 (SEQ ID No. 665 or encoded by SEQ ID NO: 27 or 346), TTLL3 (SEQ ID No. 668 or encoded by SEQ ID NO: 30 or 349), ATP13A2 (SEQ ID No. 682 or encoded by SEQ ID NO: 44 or 363), LTF (SEQ ID No. 713 or encoded by SEQ ID NO: 75 or 394), STMN4 (SEQ ID No. 730 or encoded by SEQ ID NO: 92 or 411), ACBD6 (SEQ ID No. 746 or encoded by SEQ ID NO: 108 or 427), GTF2F1 (SEQ ID No. 772 or encoded by SEQ ID NO: 134 or 453), KDR (SEQ ID No. 776 or encoded by SEQ ID NO: 138 or 457), DIP2C (SEQ ID No. 851 or encoded by SEQ ID NO: 213 or 532), FXC1 (SEQ ID No. 862 or encoded by SEQ ID NO: 224 or 543), HNRNPA1 (SEQ ID No. 865 or encoded by SEQ ID NO: 227 or 546), HSBP1L1 (SEQ ID No. 867 or encoded by SEQ ID NO: 229 or 548), KARS (SEQ ID No. 870 or encoded by SEQ ID NO: 232 or 551), PBXIP1 (SEQ ID No. 895 or encoded by SEQ ID NO: 257 or 576), ZNF431 (SEQ ID No. 928 or encoded by SEQ ID NO: 290 or 609), STMN1 (SEQ ID No. 931 or encoded by SEQ ID NO: 293 or 612), and SSRP1 (SEQ ID No. 935 or encoded by SEQ ID NO: 297 or 616) are used to demonstrate an interaction with antibodies from a bodily fluid or a tissue sample from a patient with SSc.

6. Use according to claim 1, wherein, in addition, the markers NSUN5 (SEQ ID No. 641 or encoded by SEQ ID NO: 3 or 322), RTEL1 (SEQ ID No. 642 or encoded by SEQ ID NO: 4 or 323), TRIM21 (SEQ ID No. 646 or encoded by SEQ ID NO: 8 or 327), ABCB8 (SEQ ID No. 647 or encoded by SEQ ID NO: 9 or 328), AVEN (SEQ ID No. 648 or encoded by SEQ ID NO: 10 or 329), CENPA (SEQ ID No. 651 or encoded by SEQ ID NO: 13 or 332), CENPC1 (SEQ ID No.652 or encoded by SEQ ID NO: 14 or 333), CENPT (SEQ ID No. 653 or encoded by SEQ ID NO: 15 or 334), GYS1 (SEQ ID No. 656 or encoded by SEQ ID NO: 18 or 337), C19orf52 (SEQ ID No. 686 or encoded by SEQ ID NO: 48 or 367), STMN4 (SEQ ID No. 730 or encoded by SEQ ID NO: 92 or 411), ACBD6 (SEQ ID No. 746 or encoded by SEQ ID NO: 108 or 427), CBX3 (SEQ ID No. 753 or encoded by SEQ ID NO: 115 or 434), CENPH (SEQ ID No. 756 or encoded by SEQ ID NO: 118 or 437), EIF4E (SEQ ID No. 761 or encoded by SEQ ID NO: 123 or 442), GTF2F1 (SEQ ID No. 772 or encoded by SEQ ID NO: 134 or 453), MAPT (SEQ ID No. 778 or encoded by SEQ ID NO: 140 or 459), DIP2C (SEQ ID No. 851 or encoded by SEQ ID NO: 213 or 532), PBXIP1 (SEQ ID No. 895 or encoded by SEQ ID NO: 257 or 576), NEFL (SEQ ID No. 930 or encoded by SEQ ID NO: 292 or 611), STMN1 (SEQ ID No. 931 or encoded by SEQ ID NO: 293 or 612), and SSRP1 (SEQ ID No. 935 or encoded by SEQ ID NO: 297 or 616) are used to demonstrate an interaction with antibodies from a bodily fluid or a tissue sample from a patient with SSc.

7. Solid support, on which the markers CENPB (SEQ ID No.793 or encoded by SEQ ID NO: 155 or 474), TOP1 (SEQ ID No. 952 or encoded by SEQ ID NO: 314 or 633), KDM6B (SEQ ID No. 639 or encoded by SEQ ID NO: 1 or 320) and BICD2 (SEQ ID No. 640 or encoded by SEQ ID NO: 2 or 321) are attached.

## Revendications

1. Utilisation des marqueurs CENPB (SEQ ID N° 793 ou codé par SEQ ID N° 155 ou 474), TOP1 (SEQ ID N° 952 ou codé par SEQ ID N° 314 ou 633), KDM6B (SEQ ID N° 639 ou codé par SEQ ID N° 1 ou 320) et BICD2 (SEQ ID N° 640 ou codé par SEQ ID N° 2 ou 321) pour le diagnostic in vitro/ex vivo de la sclérodermie systémique (ScS), dans laquelle CENPB, TOP1, KDM6B et BICD sont utilisés pour indiquer une interaction avec des anticorps issus d'un liquide corporel ou d'un prélèvement de tissu d'un patient atteint de ScS.

2. Utilisation selon la revendication 1, dans laquelle en supplément NSUN5 (SEQ ID N° 641 ou codé par SEQ ID N° 3 ou 322) et/ou PPP1R2 (SEQ ID N° 933 ou codé par SEQ ID N° 295 ou 614) sont utilisés pour indiquer une interaction avec des anticorps issus d'un fluide corporel ou d'un prélèvement de tissu d'un patient atteint de ScS.

3. Utilisation selon la revendication 1, dans laquelle en supplément NSUN5 (SEQ ID N° 641 ou codé par SEQ ID N° 3 ou 322) et PPP1R2 (SEQ ID N° 933 ou codé par SEQ ID N° 295 ou 614) sont utilisés pour indiquer une interaction avec des anticorps issus d'un liquide corporel ou d'un prélèvement de tissu d'un patient atteint de ScS.

4. Utilisation selon l'une quelconque des revendications 1 à 3, dans laquelle en supplément au moins un marqueur choisi parmi le groupe RTEL1 (SEQ ID N° 642 ou codé par SEQ ID N° 4 ou 323), MYST2 (SEQ ID N° 643 ou codé par SEQ ID N° 5 ou 324), PSAT1 (SEQ ID N° 644 ou codé par SEQ ID N° 6 ou 325), TEX264 (SEQ ID N° 645 ou codé par SEQ ID N° 7 ou 326), TRIM21 (SEQ ID N° 646 ou codé par SEQ ID N° 8 ou 327), ABCB8 (SEQ ID N° 647 ou codé par SEQ ID N° 9 ou 328), AVEN (SEQ ID N° 648 ou codé par SEQ ID N° 10 ou 329), CENPA (SEQ ID N° 651 ou codé par SEQ ID N° 13 ou 332), CENPC1 (SEQ ID N° 652 ou codé par SEQ ID N° 14 ou 333), GYS1 (SEQ ID N° 656 ou codé par SEQ ID N° 18 ou 337), MARVELD2 (SEQ ID N° 660 ou codé par SEQ ID N° 22 ou 341), PMF1 (SEQ ID N° 665 ou codé par SEQ ID N° 27 ou 346), TTLL3 (SEQ ID N° 668 ou codé par SEQ ID N° 30 ou 349), ATP13A2 (SEQ ID N° 682 ou codé par SEQ ID N° 44 ou 363), LTF (SEQ ID N° 713 ou codé par SEQ ID N° 75 ou 394), STMN4 (SEQ ID N° 730 ou codé par SEQ ID N° 92 ou 411), ACBD6 (SEQ ID N° 746 ou codé par SEQ ID N° 108 ou 427), GTF2F1 (SEQ ID N°772 ou codé par SEQ ID N° 134 ou 453), KDR (SEQ ID N° 776 ou codé par SEQ ID N° 138 ou 457), DIP2C (SEQ ID N° 851 ou codé par SEQ ID N° 213 ou 532), FXC1 (SEQ ID N° 862 ou codé par SEQ ID N° 224 ou 543), HNRNPA1 (SEQ ID N° 865 ou codé par SEQ ID N° 227 ou 546), HSBP1L1 (SEQ ID N° 867 ou codé par SEQ ID N° 229 ou 548), KARS (SEQ ID N° 870 ou codé par SEQ ID N° 232 ou 551), PBXIP1 (SEQ ID N° 895 ou codé par SEQ ID N° 257 ou 576), ZNF431 (SEQ ID N° 928 ou codé par SEQ ID N° 290 ou 609), STMN1 (SEQ ID N° 931 ou codé par SEQ ID N° 293 ou 612), SSRP1 (SEQ ID N° 935 ou codé par SEQ ID N° 297 ou 616) est utilisé pour indiquer une interaction avec des anticorps issus d'un fluide corporel ou d'un prélèvement de tissu d'un patient atteint de ScS.

5. Utilisation selon la revendication 1, dans laquelle en supplément les marqueurs NSUN5 (SEQ ID N° 641 ou codé par SEQ ID N° 3 ou 322), PPP1R2 (SEQ ID N° 933 ou codé par SEQ ID N° 295 ou 614), RTEL1 (SEQ ID N° 642 ou codé par SEQ ID N° 4 ou 323), TRIM21 (SEQ ID N° 646 ou codé par SEQ ID N° 8 ou 327), ABCB8 (SEQ ID N° 647 ou codé par SEQ ID N° 9 ou 328), AVEN (SEQ ID N° 648 ou codé par SEQ ID N° 10 ou 329), CENPA (SEQ ID N° 651 ou codé par SEQ ID N° 13 ou 332), CENPC1 (SEQ ID N° 652 ou codé par SEQ ID N° 14 ou 333), GYS1 (SEQ ID N° 656 ou codé par SEQ ID N° 18 ou 337), MARVELD2 (SEQ ID N° 660 ou codé par SEQ ID N° 22 ou 341), PMF1 (SEQ ID N° 665 ou codé par SEQ ID N° 27 ou 346), TTLL3 (SEQ ID N° 668 ou codé par SEQ ID N° 30 ou 349), ATP13A2 (SEQ ID N° 682 ou codé par SEQ ID N° 44 ou 363), LTF (SEQ ID N° 713 ou codé par SEQ ID N° 75 ou 394), STMN4 (SEQ ID N° 730 ou codé par SEQ ID N° 92 ou 411), ACBD6 (SEQ ID N° 746 ou codé par SEQ ID N° 108 ou 427), GTF2F1 (SEQ ID N° 772 ou codé par SEQ ID N° 134 ou 453), KDR (SEQ ID N° 776 ou codé par SEQ ID N° 138 ou 457), DIP2C (SEQ ID N° 851 ou codé par SEQ ID N° 213 ou 532), FXC1 (SEQ ID N° 862 ou codé par SEQ ID N° 224 ou 543), HNRNPA1 (SEQ ID N° 865 ou codé par SEQ ID N° 227 ou 546), HSBP1L1 (SEQ ID N° 867 ou codé par SEQ ID N° 229 ou 548), KARS (SEQ ID N° 870 ou codé par SEQ ID N° 232 ou 551), PBXIP1 (SEQ ID N° 895 ou codé par SEQ ID N° 257 ou 576), ZNF431 (SEQ ID N° 928 ou codé par SEQ ID N° 290 ou 609), STMN1 (SEQ ID N° 931 ou codé par SEQ ID N° 293 ou 612), et SSRP1 (SEQ ID N° 935 ou codé par SEQ ID N° 297 ou 616) sont utilisés pour indiquer une interaction avec des anticorps issus d'un liquide corporel ou d'un prélèvement de tissu d'un patient atteint de ScS.

6. Utilisation selon la revendication 1, dans laquelle en supplément les marqueurs NSUN5 (SEQ ID N° 641 ou codé par SEQ ID N° 3 ou 322), RTEL1 (SEQ ID N° 642 ou codé par SEQ ID N° 4 ou 323), TRIM21 (SEQ ID N° 646 ou codé par SEQ ID N° 8 ou 327), ABCB8 (SEQ ID N° 647 ou codé par SEQ ID N° 9 ou 328), AVEN (SEQ ID N° 648 ou codé par SEQ ID N° 10 ou 329), CENPA (SEQ ID N° 651 ou codé par SEQ ID N° 13 ou 332), CENPC1 (SEQ ID N° 652 ou codé par SEQ ID N° 14 ou 333), CENPT (SEQ ID N° 653 ou codé par SEQ ID N° 15 ou 334), GYS1 (SEQ ID N° 656 ou codé par SEQ ID N° 18 ou 337), C19orf52 (SEQ ID N° 686 ou codé par SEQ ID N° 48 ou 367), STMN4 (SEQ ID N° 730 ou codé par SEQ ID N° 92 ou 411), ACBD6 (SEQ ID N° 746 ou codé par SEQ ID N° 108 ou 427), CBX3 (SEQ ID N° 753 ou codé par SEQ ID N° 115 ou 434), CENPH (SEQ ID N° 756 ou codé par SEQ ID N° 118 ou 437), EIF4E (SEQ ID N° 761 ou codé par SEQ ID N° 123 ou 442), GTF2F1 (SEQ ID N° 772 ou codé par SEQ ID N° 134 ou 453), MAPT (SEQ ID N° 778 ou codé par SEQ ID N° 140 ou 459), DIP2C (SEQ ID N° 851 ou codé par SEQ ID N° 213 ou 532), PBXIP1 (SEQ ID N° 895 ou codé par SEQ ID N° 257 ou 576), NEFL (SEQ ID N° 930 ou codé par SEQ ID N° 292 ou 611), STMN1 (SEQ ID N° 931 ou codé par SEQ ID N° 293 ou 612), et SSRP1 (SEQ ID N° 935 ou codé par SEQ ID N° 297 ou 616) sont utilisés pour indiquer une interaction avec des anticorps issus d'un fluide corporel ou d'un prélèvement de tissu d'un patient atteint de ScS.

7. Support solide, sur lequel les marqueurs CENPB (SEQ ID N° 793 ou codé par SEQ ID N° 155 ou 474), TOP1 (SEQ ID N° 952 ou codé par SEQ ID N° 314 ou 633), KDM6B (SEQ ID N° 639 ou codé par SEQ ID N° 1 ou 320) et BICD2 (SEQ ID N° 640 ou codé par SEQ ID N° 2 ou 321) sont fixés.
